(19) Europäisches Patentamt / European Patent Office / Office européen des brevets

(11) EP 4 768 499 A1

(12) **EUROPEAN PATENT APPLICATION**
published in accordance with Art. 153(4) EPC

(43) Date of publication:
**01.07.2026 Bulletin 2026/27**

(21) Application number: **24888700.2**

(22) Date of filing: **05.11.2024**

(51) International Patent Classification (IPC):
***C07K 7/54*** *(2006.01)* ***C40B 40/10*** *(2006.01)*

(52) Cooperative Patent Classification (CPC):
**C07K 7/54; C40B 40/10**

(86) International application number:
**PCT/JP2024/039298**

(87) International publication number:
**WO 2025/100405 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.11.2023 JP 2023189465**

(71) Applicant: **Chugai Seiyaku Kabushiki Kaisha**
**Kita-ku**
**Tokyo 115-8543 (JP)**

(72) Inventors:
- **MORITA, Yuya**
  **Yokohama City, Kanagawa 2448602 (JP)**
- **TANAKA, Shota**
  **Yokohama City, Kanagawa 2448602 (JP)**

(74) Representative: **Vossius & Partner**
**Patentanwälte Rechtsanwälte mbB**
**Siebertstraße 3**
**81675 München (DE)**

# (54) MACROCYCLIC PEPTIDE COMPOUND HAVING CELL MEMBRANE PERMEABILITY AND METABOLIC STABILITY, AND LIBRARY CONTAINING SAME

(57) The present invention provides a cyclic peptide compound optionally linked to a nucleic acid, wherein the cyclic peptide compound contains a cyclic portion, (1) the cyclic portion is composed of 13 or 14 amino acid residues, (2) among the amino acid residues composing the cyclic portion, the number of N-substituted amino acid residues is 7 or more, the number of amino acid residues having the same side chain is 3 or less, and the number of natural amino acid residues is 4 or less, and (3) ClogP/total amide bond (ClogP/total AB) is 1.0 to 1.8.



Processed by Luminess, 75001 PARIS (FR)

## Description

[Technical Field]

The present invention provides a macrocyclic peptide compound having cell membrane permeability and metabolic stability, and a library containing the same.

[Background Art]

Middle-molecule compounds (molecular weight: 500 to 2000) have attracted attention as a modality capable of achieving development of a drug against a tough target which is typified by protein-protein interaction inhibition or the like (Non Patent Literature 1). It has been considered difficult to develop a peptide itself as a medicament because peptides are generally poor in drug-likeness (e.g., metabolic stability and cell membrane permeability (hereinafter also referred to as "membrane permeability"). In recent years, it has been found that by cyclization of a peptide or use of a non-natural amino acid such as a N-methylamino acid in a peptide, metabolic stability or membrane permeability are improved (Non Patent Literatures 2 and 3). It has come to be known that among cyclic peptides containing a non-natural amino acid, particularly, cyclic peptides containing a N-substituted amino acid may have drug-likeness (Patent Literature 1). It has been also suggested that library compounds of cyclic peptides containing a non-natural amino acid are useful for the development of a protein-protein interaction inhibitor (Non Patent Literature 4). Furthermore, in recent years, a cyclic peptide compound having high membrane permeability, a library containing the compound, a production method thereof, and a screening method have been reported (Patent Literature 2), and an amino acid capable of improving the membrane permeability of peptide compounds, a peptide compound containing the amino acid, and a production method thereof have also been reported (Patent Literature 3).

Patent Literature 1 describes that it is preferable that the number of amino acids and amino acid analogs composing the cyclic portion is 5 to 12, particularly preferably 9 to 11, when considering achieving both membrane permeability and metabolic stability.

Patent Literature 2 discloses a pre-incubation method using Caco-2 cells as a method for more accurately measuring the membrane permeability of highly lipophilic drugs and the like compared to conventional methods. The method is used for evaluating the membrane permeability of cyclic peptides mainly composed of 11 amino acid residues (Patent Literatures 2 and 3).

However, for cyclic peptides in which the number of amino acid residues comprising the cyclic portion is 13 or more residues, it has not been sufficiently investigated which types of cyclic peptides have drug-likeness.

[Citation List]

[Patent Literature]

[Patent Literature 1]
International Publication No. WO 2013/100132
[Patent Literature 2]
International Publication No. WO 2018/225864
[Patent Literature 3]
International Publication No. WO 2020/122182


[Non Patent Literature]

[Non Patent Literature 1]
Future Med. Chem., 2009, 1, 1289-1310.
[Non Patent Literature 2]
Acc. Chem. Res. 2008, 41, 1331-1342.
[Non Patent Literature 3]
Angew. Chem. Int. Ed., 2013, 52, 254-269.
[Non Patent Literature 4]
Chem. Rev., 2019, 119, 10360-10391.

[Summary of Invention]

[Technical Problem]

The present invention has been made in view of such circumstances, and an object of the present invention is to provide a library for screening for a cyclic peptide having excellent metabolic stability and membrane permeability. In another aspect, an object of the present invention is to provide a cyclic peptide compound having high membrane permeability, a production method thereof, or a screening method.

[Solution to Problem]

It is believed that as the size of cyclic peptide increases, that is, the number of amino acid residues composing the cyclic portion increases, the surface area available for contact with target molecules increases. Thus, it is considered advantageous for peptides to have a large number of amino acid residues composing the cyclic portion, from the viewpoint of acquiring interactions with target molecules. Accordingly, the present inventors have conducted intensive research to determine whether there exists a region in cyclic peptides in which the number of amino acid residues composing the cyclic portion is 13 or more, deviating from the conventionally considered favorable range, where both membrane permeability and metabolic stability can be achieved. Specifically, various cyclic peptides having different side chains and composed of 13 or 14 amino acid residues have been synthesized, and the drug-likeness of cyclic peptide compounds in which the number of amino acid residues composing the cyclic portion is 13 or 14 have been evaluated by metabolic stability testing using liver microsomes and membrane permeability testing using Caco-2 cells. As a result, the inventors have found that a peptide compound composed of 13 or 14 amino acid residues, in which physical and chemical properties such as the number of N-substituted amino acids and lipophilicity are controlled, can exhibit excellent membrane permeability and metabolic stability, and have developed a library to obtain cyclic peptide compounds more efficiently.

The present invention provides the following [A1] to [A52], [B1] to [B73], and [C1] to [C6].

[A1]
A cyclic peptide compound optionally linked to a nucleic acid, the compound comprising a cyclic portion, wherein

(1) the cyclic portion is composed of 13 or 14 amino acid residues,
(2) among the amino acid residues composing the cyclic portion, the number of N-substituted amino acid residues is 7 or more, the number of amino acid residues having the same side chain is 3 or less, and the number of natural amino acid residues is 4 or less, and
(3) ClogP/total amide bond (ClogP/total AB) is 1.0 to 1.8.

[A2]
The cyclic peptide compound optionally linked to a nucleic acid according to [A1], wherein the number of amino acid residues composing the cyclic peptide compound is 13 or 14.
[A2-1]
The cyclic peptide compound optionally linked to a nucleic acid according to [A1] or [A2], wherein the number of amino acid residues composing the cyclic peptide compound is 13.
[A2-2]
The cyclic peptide compound optionally linked to a nucleic acid according to [A1] or [A2], wherein the number of amino acid residues composing the cyclic peptide compound is 14.
[A3]
The cyclic peptide compound optionally linked to a nucleic acid according to [A1] or [A2], wherein the number of N-substituted amino acids composing the cyclic portion is 7 to 13.
[A4]
The cyclic peptide compound optionally linked to a nucleic acid according to [A3], wherein the number of N-substituted amino acids composing the cyclic portion is 7 to 10.
[A5]
The cyclic peptide compound optionally linked to a nucleic acid according to [A1] or [A2], wherein the number of N-substituted amino acids composing the cyclic portion is 8 or more.
[A6]
The cyclic peptide compound optionally linked to a nucleic acid according to [A5], wherein the number of N-substituted amino acids composing the cyclic portion is 8 to 13.
[A7]
The cyclic peptide compound optionally linked to a nucleic acid according to [A6], wherein the number of N-substituted

amino acids composing the cyclic portion is 8 to 10.

[A8]

The cyclic peptide compound optionally linked to a nucleic acid according to [A1] or [A2], wherein a proportion of the number of N-substituted amino acid residues to the total number of amino acid residues composing the cyclic portion is 60% or more.

[A8-1]

The cyclic peptide compound optionally linked to a nucleic acid according to [A8], wherein a proportion of the number of N-substituted amino acid residues to the total number of amino acid residues composing the cyclic portion is 65% or more.

[A8-2]

The cyclic peptide compound optionally linked to a nucleic acid according to [A8], wherein a proportion of the number of N-substituted amino acid residues to the total number of amino acid residues composing the cyclic portion is 70% or more.

[A9]

The cyclic peptide compound optionally linked to a nucleic acid according to any of [A1] to [A8], wherein the N-substituted amino acids are N-alkyl amino acids.

[A10]

The cyclic peptide compound optionally linked to a nucleic acid according to [A9], wherein the N-alkyl amino acids are N-$C_1$-$C_6$ alkyl amino acids.

[A11]

The cyclic peptide compound optionally linked to a nucleic acid according to [A10], wherein the N-alkyl amino acids are N-methylamino acids or N-ethyl amino acids.

[A12]

The cyclic peptide compound optionally linked to a nucleic acid according to [A11], wherein the N-alkyl amino acids are N-methyl amino acids.

[A13]

The cyclic peptide compound optionally linked to a nucleic acid according to any of [A1] to [A12], wherein ClogP/total AB of the cyclic peptide compound is 1.0 to 1.5.

[A14]

The cyclic peptide compound optionally linked to a nucleic acid according to [A13], wherein ClogP/total AB of the cyclic peptide compound is 1.1 to 1.4.

[A15]

The cyclic peptide compound optionally linked to a nucleic acid according to any of [A1] to [A14], wherein ClogP of the cyclic peptide compound is 13 to 20.

[A16]

The cyclic peptide compound optionally linked to a nucleic acid according to any of [A1] to [A15], wherein the cyclic peptide compound has 3 or less hydrogen bond donors.

[A17]

The cyclic peptide compound optionally linked to a nucleic acid according to any of [A1] to [A16], wherein a side chain of the amino acid composing the cyclic portion is composed of at least one selected from the group consisting of an alkyl group, a cycloalkyl group, an aralkyl group, and an amide group, wherein an arbitrary atom of the alkyl group and the nitrogen atom of the amino group of the amino acid may be taken together to form a ring.

[A18]

The cyclic peptide compound optionally linked to a nucleic acid according to [A17], wherein the alkyl group is a $C_1$-$C_6$ alkyl group optionally substituted with at least one group selected from the group consisting of a halogen atom, a hydroxy group, a $C_3$-$C_8$ cycloalkyl group, and a $C_1$-$C_6$ alkoxy group.

[A19]

The cyclic peptide compound optionally linked to a nucleic acid according to [A17] or [A18], wherein the cycloalkyl group is a $C_3$-$C_{10}$ cycloalkyl group optionally substituted with at least one group selected from the group consisting of a halogen atom, a hydroxy group, a $C_1$-$C_6$ alkyl group, a $C_1$-$C_6$ haloalkyl group, a $C_1$-$C_6$ alkoxy group, and a $C_1$-$C_6$ haloalkoxy group.

[A20]

The cyclic peptide compound optionally linked to a nucleic acid according to any of [A17] to [A19], wherein the aralkyl group is a $C_7$-$C_{14}$ aralkyl group optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

[A21]

The cyclic peptide compound optionally linked to a nucleic acid according to any of [A17] to [A20], wherein the aralkyl group is an optionally substituted $C_5$-$C_{10}$ aryl $C_1$-$C_6$ alkyl group, in which the $C_5$-$C_{10}$ aryl group is optionally substituted

with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

[A22]

The cyclic peptide compound optionally linked to a nucleic acid according to any of [A17] to [A21], wherein the amide group is represented by formula: $-CONR^1R^2$, wherein $R^1$ and $R^2$ are each independently a hydrogen atom or a $C_1$-$C_6$ alkyl group, or $R^1$ and $R^2$, together with a nitrogen atom to which they are attached, form a 4- to 8-membered saturated heterocyclic ring, and the 4- to 8-membered saturated heterocyclic ring is optionally substituted with at least one group independently selected from the group consisting of a halogen atom, an oxo group, a $C_1$-$C_6$ alkyl group, and a 4- to 7-membered heterocyclyl group.

[A23]

The cyclic peptide compound optionally linked to a nucleic acid according to any of [A1] to [A22], wherein the cyclic peptide compound contains at least one amide bond formed between a carboxy group contained in a side chain of an amino acid and a nitrogen atom of an amino group of another amino acid.

[A24]

The cyclic peptide compound optionally linked to a nucleic acid according to any of [A1] to [A23], wherein the cyclic peptide compound contains an amino acid selected from the group consisting of Asp-pip and MeAsp-pip.

[A25]

The cyclic peptide compound optionally linked to a nucleic acid according to any of [A1] to [A24], wherein the amino acids composing the cyclic portion include an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.

[A26]

The cyclic peptide compound optionally linked to a nucleic acid according to any of [A1] to [A25], wherein the amino acids composing the cyclic portion are each independently an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.

[A27]

The cyclic peptide compound optionally linked to a nucleic acid according to any of [A1] to [A26], wherein the amino acids composing the cyclic portion include an $\alpha$-amino acid, and the number of the $\alpha$-amino acid is 10 to 14.

[A28]

The cyclic peptide compound optionally linked to a nucleic acid according to any of [A1] to [A27], wherein the amino acids composing the cyclic portion include an $\alpha$-amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu).

[A29]

The cyclic peptide compound optionally linked to a nucleic acid according to [A27] or [A28], wherein among the amino acids composing the cyclic portion, $\alpha$-amino acids are each independently selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu).

[A30]

The cyclic peptide compound optionally linked to a nucleic acid according to any of [A1] to [A31], wherein the amino acids composing the cyclic portion include an amino acid having a $\beta$-amino acid skeleton, and the number of the amino acid having a $\beta$-amino acid skeleton is 0 to 2.

[A31]

The cyclic peptide compound optionally linked to a nucleic acid according to any of [A1] to [A30], wherein the amino acids composing the cyclic portion include an amino acid having a $\beta$-amino acid skeleton selected from the group consisting of Asp-pip, bAla, bMeAla, D-3-Abu, D-3-MeAbu, and MeAsp-pip.

[A32]

The cyclic peptide compound optionally linked to a nucleic acid according to [A30] or [A31], wherein among the amino acids composing the cyclic portion, the amino acids having a $\beta$-amino acid skeleton are each independently selected from the group consisting of Asp-pip, bAla, bMeAla, D-3-Abu, D-3-MeAbu, and MeAsp-pip.

[A33]

The cyclic peptide compound optionally linked to a nucleic acid according to any of [A1] to [A32], wherein the amino acids composing the cyclic portion include an amino acid having a $\gamma$-amino acid skeleton, and the number of the amino acid having a $\gamma$-amino acid skeleton is 0 to 2.

[A34]

The cyclic peptide compound optionally linked to a nucleic acid according to any of [A1] to [A33], wherein the amino acids composing the cyclic portion include gMeAbu.

[A35]
The cyclic peptide compound optionally linked to a nucleic acid according to [A33] or [A34], wherein among the amino acids composing the cyclic portion, γ-amino acids are each independently gMeAbu.
[A36]
The cyclic peptide compound optionally linked to a nucleic acid according to any of [A1] to [A35], wherein among the amino acids composing the cyclic portion, the number of natural amino acids is 1 to 4.
[A37]
The cyclic peptide compound optionally linked to a nucleic acid according to any of [A1] to [A36], wherein the amino acids composing the cyclic portion include a natural amino acid selected from the group consisting of Gly, Ala, Val, Leu, Ile, Thr, Phe, and Pro.
[A38]
The cyclic peptide compound optionally linked to a nucleic acid according to [A37], wherein among the amino acids composing the cyclic portion, natural amino acids are each independently selected from the group consisting of Gly, Ala, Val, Leu, Ile, Thr, Phe, and Pro.
[A39]
The cyclic peptide compound optionally linked to a nucleic acid according to any of [A1] to [A38], wherein among amino acids composing the cyclic portion, the number of non-natural amino acids is 9 or more.
[A40]
The cyclic peptide compound optionally linked to a nucleic acid according to any of [A1] to [A39], wherein the amino acids composing the cyclic portion include a non-natural amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).
[A41]
The cyclic peptide compound optionally linked to a nucleic acid according to any of [A36] to [A40], wherein among the amino acids composing the cyclic portion, non-natural amino acids are each independently selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).
[A42]
The cyclic peptide compound optionally linked to a nucleic acid according to any of [A1] to [A41], wherein a value of Caco-2 $P_{app}$ (cm/sec) of the cyclic peptide compound is $0.4 \times 10^{-6}$ or more.
[A43]
The cyclic peptide compound optionally linked to a nucleic acid according to [A44], wherein the value of Caco-2 $P_{app}$ (cm/sec) is calculated by membrane permeability evaluation under the following conditions:

(a) Caco-2 cells are cultured on 96-well Transwell for 3 weeks, then a compound to be evaluated, a DMEM medium, and FaSSIF (containing 1% DMSO) are added to the Apical side, a DMEM medium is added to the Basal side, and the cells are preincubated under a 5% $CO_2$ atmosphere at 37°C with shaking at 80 rpm for 24 hours;
(b) after the preincubation, the preincubation solutions on the Apical side and the Basal side are aspirated and washed, and a FaSSIF/HBSS buffer solution (containing 1% DMSO, pH 6.0) containing the compound is added to the Apical side, and a HBSS buffer solution (containing 4% BSA, pH 7.4) is added to the Basal side;
(c) each well is shaken under a 5% $CO_2$ atmosphere at 37°C and 80 rpm, and at 180 minutes after the start of shaking, a sample on the Basal side is collected, and a permeation amount of the compound is measured by liquid chromatography-mass spectrometry (LC/MS); and
(d) from the measured permeation amount, a permeability coefficient (Caco-2 $P_{app}$ (cm/sec)) is calculated.

[A44]
A cyclic peptide compound selected from compounds pd001 to pd168 listed in Table 15.
[A45]
The cyclic peptide compound linked to a nucleic acid (cyclic peptide-nucleic acid complex) according to any of [A1] to [A44].
[A46]
The cyclic peptide-nucleic acid complex according to [A45], wherein the cyclic peptide compound has a cyclic portion and a linear portion, and the nucleic acid is bound to the linear portion.
[A47]

The cyclic peptide-nucleic acid complex according to [A46], wherein the cyclic peptide compound further comprises a linker, and
the nucleic acid is bound to the linear portion via the linker.

[A48]
The cyclic peptide-nucleic acid complex according to [A47], wherein the linker contains puromycin.
[A49]
The cyclic peptide-nucleic acid complex according to any of [A45] to [A48], wherein the nucleic acid is DNA or RNA.
[A50]
The cyclic peptide-nucleic acid complex according to any of [A45] to [A49], wherein the nucleic acid contains a nucleic acid encoding the cyclic peptide compound.
[A51]
The cyclic peptide-nucleic acid complex according to any of [A45] to [A49], wherein the nucleic acid is a nucleic acid encoding the cyclic peptide compound.
[A52]
The cyclic peptide-nucleic acid complex according to any of [A45] to [A49], wherein the nucleic acid contains a nucleotide sequence encoding a cyclic peptide moiety that forms the cyclic peptide-nucleic acid complex.
[B1]
A library comprising the cyclic peptide compound optionally linked to a nucleic acid according to any of [A1] to [A52].
[B2]

A library comprising a cyclic peptide compound optionally linked to a nucleic acid,
wherein the cyclic peptide compound contains a cyclic portion, and
wherein a proportion of the number of cyclic peptide compounds in which the number of amino acid residues composing the cyclic portion is 13 or 14, relative to the total number of cyclic peptide compounds contained in the library is 10% or more.

[B2-1]
The library according to [B1] or [B2], wherein a proportion of the number of cyclic peptide compounds in which the number of amino acid residues composing the cyclic portion is 13 or 14 to the total number of cyclic peptide compounds contained in the library is 20% or more.
[B2-2]
The library according to [B1] or [B2], wherein a proportion of the number of cyclic peptide compounds in which the number of amino acid residues composing the cyclic portion is 13 or 14 to the total number of cyclic peptide compounds contained in the library is 40% or more.
[B2-3]
The library according to [B1] or [B2], wherein a proportion of the number of cyclic peptide compounds in which the number of amino acid residues composing the cyclic portion is 13 or 14 to the total number of cyclic peptide compounds contained in the library is 60% or more.
[B2-4]
The library according to [B1] or [B2], wherein a proportion of the number of cyclic peptide compounds in which the number of amino acid residues composing the cyclic portion is 13 or 14 to the total number of cyclic peptide compounds contained in the library is 80% or more.
[B3]
The library according to [B1] or [B2], which consists substantially of cyclic peptide compounds in which the number of amino acid residues composing the cyclic portion is 13 or 14.
[B4]
The library according to any of [B1] to [B3], wherein a proportion of the number of cyclic peptide compounds in which the number of amino acid residues composing the cyclic peptide compound is 13 or 14 to the total number of cyclic peptide compounds contained in the library is 10% or more.
[B4-1]
The library according to any of [B1] to [B4], wherein a proportion of the number of cyclic peptide compounds in which the number of amino acid residues composing the cyclic peptide compound is 13 or 14 to the total number of cyclic peptide compounds contained in the library is 20% or more.
[B4-2]
The library according to any of [B1] to [B4], wherein a proportion of the number of cyclic peptide compounds in which the number of amino acid residues composing the cyclic peptide compound is 13 or 14 to the total number of cyclic peptide compounds contained in the library is 40% or more.
[B4-3]
The library according to any of [B1] to [B4], wherein a proportion of the number of cyclic peptide compounds in which the number of amino acid residues composing the cyclic peptide compound is 13 or 14 to the total number of cyclic peptide compounds contained in the library is 60% or more.

[B4-4]
The library according to any of [B1] to [B4], wherein a proportion of the number of cyclic peptide compounds in which the number of amino acid residues composing the cyclic peptide compound is 13 or 14 to the total number of cyclic peptide compounds contained in the library is 80% or more.
[B5]
The library according to any of [B1] to [B4], which consists substantially of cyclic peptide compounds in which the number of amino acid residues composing the cyclic peptide compound is 13 or 14.
[B6]
The library according to any of [B1] to [B5], wherein, in the cyclic peptide compounds contained in the library, a proportion of the number of the N-substituted amino acids to the total number of amino acids composing the cyclic portion is 50% or more on average.
[B7]
The library according to any of [B1] to [B6], wherein the number of the N-substituted amino acids is 7 to 13 on average.
[B8]
The library according to [B7], wherein in the cyclic peptide compounds contained in the library, the number of the N-substituted amino acids composing the cyclic portion is 7 to 10 on average.
[B9]
The library according to [B7], wherein in the cyclic peptide compounds contained in the library, the number of the N-substituted amino acids composing the cyclic portion is 8 to 13 on average.
[B10]
The library according to [B7], wherein in the cyclic peptide compounds contained in the library, the number of the N-substituted amino acids composing the cyclic portion is 8 to 10 on average.
[B11]
The library according to any of [B1] to [B6], wherein in the cyclic peptide compounds contained in the library, a proportion of the number of cyclic peptide compounds in which the number of N-substituted amino acids composing the cyclic portion is 7 to 10 is 10% or more.
[B11-1]
The library according to any of [B1] to [B6], wherein in the cyclic peptide compounds contained in the library, a proportion of the number of cyclic peptide compounds in which the number of N-substituted amino acids composing the cyclic portion is 7 to 10 is 20% or more.
[B11-2]
The library according to any of [B1] to [B6], wherein, in the cyclic peptide compounds contained in the library, a proportion of the number of cyclic peptide compounds in which the number of N-substituted amino acids composing the cyclic portion is 7 to 10 is 40% or more.
[B11-3]
The library according to any of [B1] to [B6], wherein in the cyclic peptide compounds contained in the library, a proportion of the number of cyclic peptide compounds in which the number of N-substituted amino acids composing the cyclic portion is 7 to 10 is 60% or more.
[B11-4]
The library according to any of [B1] to [B6], wherein in the cyclic peptide compounds contained in the library, a proportion of the number of cyclic peptide compounds in which the number of N-substituted amino acids composing the cyclic portion is 7 to 10 is 80% or more.
[B12]
The library according to any of [B1] to [B6], wherein in the cyclic peptide compounds contained in the library, a proportion of the number of cyclic peptide compounds in which the number of N-substituted amino acids composing the cyclic portion is 8 to 13 is 10% or more.
[B12-1]
The library according to any of [B1] to [B6], wherein in the cyclic peptide compounds contained in the library, a proportion of the number of cyclic peptide compounds in which the number of N-substituted amino acids composing the cyclic portion is 8 to 13 is 20% or more.
[B12-2]
The library according to any of [B1] to [B6], wherein in the cyclic peptide compounds contained in the library, a proportion of the number of cyclic peptide compounds in which the number of N-substituted amino acids composing the cyclic portion is 8 to 13 is 40% or more.
[B12-3]
The library according to any of [B1] to [B6], wherein in the cyclic peptide compounds contained in the library, a proportion of the number of cyclic peptide compounds in which the number of N-substituted amino acids composing the cyclic portion is 8 to 13 is 60% or more.

[B12-4]
The library according to any of [B1] to [B6], wherein in the cyclic peptide compounds contained in the library, a proportion of the number of cyclic peptide compounds in which the number of N-substituted amino acids composing the cyclic portion is 8 to 13 is 80% or more.
[B13]
The library according to any of [B1] to [B6], wherein in the cyclic peptide compounds contained in the library, a proportion of the number of cyclic peptide compounds in which the number of N-substituted amino acids composing the cyclic portion is 8 to 10 is 10% or more.
[B13-1]
The library according to any of [B1] to [B6], wherein in the cyclic peptide compounds contained in the library, a proportion of the number of cyclic peptide compounds in which the number of N-substituted amino acids composing the cyclic portion is 8 to 10 is 20% or more.
[B13-2]
The library according to any of [B1] to [B6], wherein in the cyclic peptide compounds contained in the library, a proportion of the number of cyclic peptide compounds in which the number of N-substituted amino acids composing the cyclic portion is 8 to 10 is 40% or more.
[B13-3]
The library according to any of [B1] to [B6], wherein in the cyclic peptide compounds contained in the library, a proportion of the number of cyclic peptide compounds in which the number of N-substituted amino acids composing the cyclic portion is 8 to 10 is 60% or more.
[B13-4]
The library according to any of [B1] to [B6], wherein in the cyclic peptide compounds contained in the library, a proportion of the number of cyclic peptide compounds in which the number of N-substituted amino acids composing the cyclic portion is 8 to 10 is 80% or more.
[B14]
The library according to any of [B1] to [B10], wherein the N-substituted amino acids are N-alkyl amino acids.
[B15]
The library according to [B14], wherein the N-alkyl amino acids are N-$C_1$-$C_6$ alkyl amino acids.
[B16]
The library according to [B14], wherein the N-alkyl amino acids are N-methylamino acids or N-ethyl amino acids.
[B17]
The library according to [B14], wherein the N-alkyl amino acids are N-methyl amino acids.
[B18]
The library according to any of [B1] to [B17], wherein an average of ClogP/total AB of each cyclic peptide compound contained in the library is 1.0 to 1.8.
[B18-1]
The library according to any of [B1] to [B17], wherein an average of ClogP/total AB of each cyclic peptide compound contained in the library is 1.0 to 1.5.
[B18-2]
The library according to any of [B1] to [B17], wherein an average of ClogP/total AB of each cyclic peptide compound contained in the library is 1.1 to 1.4.
[B19]
The library according to any of [B1] to [B18-2], wherein an average of ClogP/total AB of a peptide moiety other than a nucleic acid linking portion in each cyclic peptide compound contained in the library is 1.0 to 1.8.
[B19-1]
The library according to any of [B1] to [B18-2], wherein an average of ClogP/total AB of a peptide moiety other than a nucleic acid linking portion in each cyclic peptide compound contained in the library is 1.0 to 1.5.
[B19-2]
The library according to any of [B1] to [B18-2], wherein an average of ClogP/total AB of a peptide moiety other than a nucleic acid linking portion in each cyclic peptide compound contained in the library is 1.1 to 1.4.
[B20]
The library according to any of [B1] to [B19-2], wherein an average of ClogP of each cyclic peptide compound contained in the library is 13 to 20.
[B21]
The library according to any of [B1] to [B20], wherein an average of ClogP of a peptide moiety other than a nucleic acid linking portion of each cyclic peptide compound contained in the library is 13 to 20.
[B22]
The library according to any of [B1] to [B21], wherein

the cyclic peptide compound has a hydrogen bond donor, and
an average of the number of the hydrogen bond donor in each cyclic peptide compound contained in the library is 8 or less.
[B22-1]
The library according to any of [B1] to [B21], wherein
the cyclic peptide compound comprises a hydrogen bond donor, and
an average of the number of the hydrogen bond donor in a cyclic portion of each cyclic peptide compound contained in the library is 8 or less.
[B23]
The library according to [B22], wherein an average of the number of the hydrogen bond donor in each cyclic peptide compound contained in the library is 3 to 8.
[B23-1]
The library according to [B22], wherein an average of the number of the hydrogen bond donor in a cyclic portion of each cyclic peptide compound contained in the library is 3 to 8.
[B24]
The library according to any of [B1] to [B23], wherein the cyclic peptide compound has a hydrogen bond donor in a cyclic portion thereof.
[B25]
The library according to any of [B1] to [B24], wherein in each cyclic peptide compound contained in the library, an average of the number of amino acids having the same side chain among amino acids composing the cyclic portion is 3 or less.
[B26]
The library according to any of [B1] to [B24], wherein in each cyclic peptide compound contained in the library, the number of amino acids having the same side chain among amino acids composing the cyclic portion is 3 or less.
[B27]
The library according to any of [B1] to [B26], wherein in each cyclic peptide compound, a side chain of each amino acid composing the cyclic portion is composed of at least one selected from the group consisting of an alkyl group, a cycloalkyl group, an aralkyl group, and an amide group, wherein an arbitrary atom of the alkyl group and the nitrogen atom of the amino group of the amino acid may be taken together to form a ring.
[B28]
The library according to [B27], wherein the alkyl group is a $C_1$-$C_6$ alkyl group optionally substituted with at least one group selected from the group consisting of a halogen atom, a hydroxy group, a $C_3$-$C_8$ cycloalkyl group, and a $C_1$-$C_6$ alkoxy group.
[B29]
The library according to [B27] or [B28], wherein the cycloalkyl group is a $C_3$-$C_{10}$ cycloalkyl group optionally substituted with at least one group selected from the group consisting of a halogen atom, a hydroxy group, a $C_1$-$C_6$ alkyl group, a $C_1$-$C_6$ haloalkyl group, a $C_1$-$C_6$ alkoxy group, and a $C_1$-$C_6$ haloalkoxy group.
[B30]
The library according to any of [B27] to [B29], wherein the aralkyl group is $C_7$-$C_{14}$ aralkyl group optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.
[B31]
The library according to any of [B27] to [B30], wherein the aralkyl group is an optionally substituted $C_5$-$C_{10}$ aryl $C_1$-$C_6$ alkyl group, in which the $C_5$-$C_{10}$ aryl group is optionally substituted with at least one group selected from the group consisting of one or more halogen atoms, $C_1$-$C_6$ alkyl groups, $C_1$-$C_6$ haloalkyl groups, $C_1$-$C_6$ alkoxy groups, and $C_1$-$C_6$ haloalkoxy groups.
[B32]
The library according to any of [B27] to [B31], wherein in each cyclic peptide compound, the amide group is represented by formula: $-CONR^1R^2$, wherein $R^1$ and $R^2$ are each independently a hydrogen atom or a $C_1$-$C_6$ alkyl group, or $R^1$ and $R^2$, together with a nitrogen atom to which they are attached, form a 4- to 8-membered saturated heterocyclic ring, and the 4-to 8-membered saturated heterocyclic ring is optionally substituted with at least one group independently selected from the group consisting of a halogen atom, an oxo group, a $C_1$-$C_6$ alkyl group, and a 4- to 7-membered heterocyclyl group.
[B33]
The library according to any of [B1] to [B32], wherein in each cyclic peptide compound, amino acids composing the cyclic portion include an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.

[B34]
The library according to any of [B1] to [B33], wherein in each cyclic peptide compound, the amino acids composing the cyclic portion are each independently selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.
[B35]
The library according to any of [B1] to [B34], wherein amino acids composing the cyclic portion include an α-amino acid, and the number of the α-amino acid is 10 to 14 on average.
[B36]
The library according to any of [B1] to [B35], wherein in each cyclic peptide compound, the amino acid composing the cyclic portion include an α-amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu).
[B37]
The library according to any of [B35] or [B36], wherein in each cyclic peptide compound, α-amino acids among the amino acids composing the cyclic portion are each independently selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu).
[B38]
The library according to any of [B1] to [B37], wherein in each cyclic peptide compound, amino acids composing the cyclic portion include an amino acid having a β-amino acid skeleton, and the number of the amino acid having a β-amino acid skeleton is 0 to 2 on average.
[B39]
The library according to any of [B1] to [B38], wherein in each cyclic peptide compound, amino acids composing the cyclic portion include an amino acid having a β-amino acid skeleton selected from the group consisting of Asp-pip, bAla, bMeAla, D-3-Abu, D-3-MeAbu, and MeAsp-pip.
[B40]
The library according to [B38] or [B39], wherein in each cyclic peptide compound, amino acids having a β-amino acid skeleton among the amino acids composing the cyclic portion are each independently selected from the group consisting of Asp-pip, bAla, bMeAla, D-3-Abu, D-3-MeAbu, and MeAsp-pip.
[B41]
The library according to any of [B1] to [B40], wherein in each cyclic peptide compound, amino acids composing the cyclic portion include an amino acid having a γ-amino acid skeleton, and the number of the amino acid having a γ-amino acid skeleton is 0 to 2 on average.
[B42]
The library according to any of [B1] to [B41], wherein in each cyclic peptide compound, amino acids composing the cyclic portion include gMeAbu.
[B43]
The library according to [B40] or [B41], wherein in each cyclic peptide compound, the amino acid having a γ-amino acid skeleton among amino acids composing the cyclic portion is gMeAbu.
[B44]
The library according to any of [B1] to [B43], wherein among amino acids composing the cyclic portion in each cyclic peptide compound, the number of natural amino acids is 1 or more on average.
[B45]
The library according to any of [B1] to [B44], wherein among amino acids composing the cyclic portion in each cyclic peptide compound, the number of natural amino acids is 5 or less on average.
[B46]
The library according to any of [B1] to [B45], wherein among amino acids composing the cyclic portion in each cyclic peptide compound, the number of natural amino acids is 1 to 5 on average.
[B47]
The library according to [B46], wherein among amino acids composing the cyclic portion in each cyclic peptide compound, the number of natural amino acids is 1 to 4 on average.
[B48]
The library according to any of [B1] to [B47], wherein among amino acids composing the cyclic portion in each cyclic peptide compound, a proportion of the number of natural amino acids to the number of amino acids is 10% or more on average.
[B49]
The library according to any of [B1] to [B48], wherein among amino acids composing the cyclic portion in each cyclic

peptide compound, a proportion of the number of natural amino acids to the number of amino acids is 40% or less on average.
[B50]
The library according to any of [B1] to [B49], wherein among amino acids composing the cyclic portion in each cyclic peptide compound, a proportion of the number of natural amino acids to the number of amino acids is 10% to 40% on average.
[B51]
The library according to any of [B1] to [B50], wherein amino acids composing the cyclic portion in each cyclic peptide compound include a natural amino acid selected from the group consisting of Gly, Ala, Val, Leu, Ile, Thr, Phe, and Pro.
[B52]
The library according to any of [B1] to [B51], wherein amino acids composing the cyclic portion in each cyclic peptide compound each independently include a natural amino acid selected from the group consisting of Gly, Ala, Val, Leu, Ile, Thr, Phe, and Pro.
[B53]
The library according to any of [B1] to [B52], wherein in each cyclic peptide compound, among amino acids composing the cyclic portion, the number of non-natural amino acids is 7 or more on average.
[B54]
The library according to any of [B1] to [B53], wherein among amino acids composing the cyclic portion in each cyclic peptide compound, the number of non-natural amino acids is 8 or more on average.
[B55]
The library according to any of [B1] to [B54], wherein in each cyclic peptide compound, a proportion of the number of non-natural amino acids to the total number of amino acids composing the cyclic portion is 50% or more on average.
[B55-1]
The library according to [B55], wherein in each cyclic peptide compound, a proportion of the number of non-natural amino acids to the total number of amino acids composing the cyclic portion is 60% or more on average.
[B55-2]
The library according to [B55], wherein in each cyclic peptide compound, a proportion of the number of non-natural amino acids to the total number of amino acids composing the cyclic portion is 70% or more on average.
[B55-3]
The library according to [B55], wherein in each cyclic peptide compound, a proportion of the number of non-natural amino acids to the total number of amino acids composing the cyclic portion is 80% or more on average.
[B56]
The library according to any of [B1] to [B55-3], wherein the cyclic peptide compound in each cyclic peptide compound include a non-natural amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).
[B57]
The library according to any of [B1] to [B56], wherein, in each cyclic peptide compound, amino acids composing the cyclic portion each independently include a non-natural amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).
[B58]
The library according to any of [B1] to [B57], wherein in each cyclic peptide compound, amino acids composing the cyclic peptide compound do not have any of a methylthio group, a thiol group, an indole skeleton, or a substituted or unsubstituted hydroxyphenyl group in their side chains.
[B59]
The library according to any of [B1] to [B58], wherein a molecular weight in each cyclic peptide compound is 500 to 2000 on average.
[B60]
The library according to any of [B1] to [B59], wherein a molecular weight of a peptide moiety other than a nucleic acid linking portion in each cyclic peptide compound is 500 to 2000 on average.
[B61]
The library according to any of [B1] to [B60], wherein the library is for use in identifying an orally administrable compound or a precursor thereof.
[B62]
The library according to any of [B1] to [B61], wherein the library is a library for use in identifying a cyclic peptide compound that is capable of specifically binding to a target molecule.
[B63]

The library according to any of [B1] to [B62], wherein the library is for use in identifying a compound that is capable of specifically binding to a target molecule.
[B64]
The library according to any of [B1] to [B63], wherein the library has a diversity of $1 \times 10^2$ or more.
[B64-1]
The library according to [B64], wherein the library has a diversity of $1 \times 10^3$ or more.
[B64-2]
The library according to [B64], wherein the library has a diversity of $1 \times 10^4$ or more.
[B64-3]
The library according to [B64], wherein the library has a diversity of $1 \times 10^6$ or more.
[B64-4]
The library according to [B64], wherein the library has a diversity of $1 \times 10^8$ or more.
[B64-5]
The library according to [B64], wherein the library has a diversity of $1 \times 10^{10}$ or more.
[B65]
The library according to any of [B1] to [B63], wherein the library comprises $1 \times 10^2$ or more types of the cyclic peptide compound optionally linked to a nucleic acid.
[B65-1]
The library according to [B65], wherein the library comprises $1 \times 10^3$ or more types of the cyclic peptide compound optionally linked to a nucleic acid.
[B65-2]
The library according to [B65], wherein the library comprises $1 \times 10^4$ or more types of the cyclic peptide compound optionally linked to a nucleic acid.
[B65-3]
The library according to [B65], wherein the library comprises $1 \times 10^6$ or more types of the cyclic peptide compound optionally linked to a nucleic acid.
[B65-4]
The library according to [B65], wherein the library comprises $1 \times 10^8$ or more types of the cyclic peptide compound optionally linked to a nucleic acid.
[B65-5]
The library according to [B65], wherein the library comprises $1 \times 10^{10}$ or more types of the cyclic peptide compound optionally linked to a nucleic acid.
[B66]
The library according to any of [B1] to [B65-5], wherein the library comprises at least one selected from the cyclic peptide compounds listed in Table 15 (i.e., pd001 to pd168).
[B67]
The library according to any of [B1] to [B65-5], wherein the library comprises all of the cyclic peptide compounds listed in Table 15 (i.e., pd001 to pd168).
[B68]
The library according to any of [B1] to [B67], wherein a cyclic peptide compound contained in the library is a cyclic peptide compound linked to a nucleic acid (cyclic peptide-nucleic acid complex).
[B69]
The library according to [B68], wherein the nucleic acid in the cyclic peptide-nucleic acid complex contains a nucleic acid encoding the cyclic peptide.
[B70]
The library according to [B68], wherein the nucleic acid in the cyclic peptide-nucleic acid complex is a nucleic acid encoding the cyclic peptide.
[B70-1]
The cyclic peptide-nucleic acid complex according to any of [B68] to [B69], wherein the nucleic acid in the cyclic peptide-nucleic acid complex contains a nucleotide sequence encoding a cyclic peptide moiety that forms the cyclic peptide-nucleic acid complex.
[B71]
The library according to any of [B1] to [B70], wherein the library is a display library.
[B72]
The library according to [B71], wherein the display library is a nucleic acid display library.
[B73]
The library according to [B72], wherein the nucleic acid display library is an mRNA display library.
[C1]

A method for screening for a cyclic peptide compound that is capable of specifically binding to a target molecule, the method comprising the steps of:

(i) bringing the library according to any of [B1] to [B73] into contact with the target molecule; and
(ii) selecting a cyclic peptide compound that is capable of specifically binding to the target molecule.

[C2]
The method according to [C1], wherein the target molecule is a protein.
[C3]
The method according to [C1] or [C2], wherein in (ii), the cyclic peptide compound that is capable of specifically binding to the target molecule is selected as a pharmaceutical candidate compound.
[C4]
The method according to any of [C1] to [C3], comprising the steps of:
chemically synthesizing a cyclic peptide compound that can be a candidate; and
measuring membrane permeability of the chemically synthesized cyclic peptide compound using a Caco-2 cell.
[C5]
The method according to any one of [C1] to [C4], wherein the membrane permeability of the chemically synthesized cyclic peptide compound is determined by a value of $P_{app}$ (cm/sec).
[C6]
The method according to [C5], wherein the value of $P_{app}$ (cm/sec) is calculated by membrane permeability evaluation under the following conditions:

(a) Caco-2 cells are cultured on 96-well Transwell for 3 weeks, then a compound to be evaluated, a DMEM medium, and FaSSIF (containing 1% DMSO) are added to the Apical side, a DMEM medium is added to the Basal side, and the cells are preincubated under a 5% $CO_2$ atmosphere at 37°C with shaking at 80 rpm for 24 hours;
(b) after the preincubation, the preincubation solutions on the Apical side and the Basal side are aspirated and washed, and a FaSSIF/HBSS buffer solution (containing 1% DMSO, pH 6.0) containing the compound is added to the Apical side, and a HBSS buffer solution (containing 4% BSA, pH 7.4) is added to the Basal side;
(c) each well is shaken under a 5% $CO_2$ atmosphere at 37°C and 80 rpm, and at 180 minutes after the start of shaking, a sample on the Basal side is collected, and the permeation amount of the compound is measured by liquid chromatography-mass spectrometry (LC/MS); and
(d) from the measured permeation amount, the permeability coefficient (Caco-2 $P_{app}$ (cm/sec)) is calculated.

In the above numberings, the number cited in the dependent item includes the branch number of the number, unless otherwise mentioned. For example, the [B5] cited in the dependent item means that not only [B5] but also its branch numbers [B5-1], [B5-2], [B5-3], and [B5-4] are included. The same applies to other numberings.

[Advantageous Effects of Invention]

In one aspect, the present invention provides a guideline for inducing a cyclic peptide compound that can be a candidate for a pharmaceutical to one with excellent membrane permeability and metabolic stability. In another aspect, the present invention enables drug discovery of cyclic peptides that are formed from 13 or 14 amino acid residues which have been difficult to achieve drug-likeness despite the benefit of acquiring interactions with targets. In still another aspect, a screening method for obtaining a cyclic peptide hit compound with excellent drug-likeness can be provided by designing a library of cyclic peptide compounds based on the present invention.

[Brief Description of Drawing]

[Figure 1] Figure 1 is a schematic diagram showing an example of a method for producing a peptide compound using a solid-phase method.

[Description of Embodiments]

Hereinafter, non-limiting preferred embodiments of the present disclosure will be described.
Any element described in Examples mentioned below is intended to be described so that it is naturally considered to be described in an equivalent manner in the "Description of Embodiments", without being bound by any restrictions of patent practices, customs, laws, or the like attempting to narrowly interpret the content described in the Examples in the country intended for prosecuting the present patent application.

In the present disclosure, any combination of all or part of one or more elements described herein is also intended to be described as being included in the present disclosure and naturally understood by those skilled in the art, as long as it does not contradict technically based on the technical knowledge of those skilled in the art.

The following abbreviations are used herein. Ala (alanine), Arg (arginine), Asn (asparagine), Asp (aspartic acid), Cys (cysteine), Glu (glutamic acid), Gln (glutamine), Gly (glycine), His (histidine), Ile (isoleucine), Leu (leucine), Lys (lysine), Met (methionine), Phe (phenylalanine), Pro (proline), Ser (serine), Thr (threonine), Trp (tryptophan), Tyr (tyrosine), and Val (valine). In addition to these, the abbreviations listed in Table 1 are used.

The term "alkyl" as used herein is a monovalent group derived from an aliphatic hydrocarbon by removal of any one hydrogen atom, and has a subset of hydrocarbyl or hydrocarbon group structures that do not contain a hetero atom (which is an atom other than carbon and hydrogen atoms) or an unsaturated carbon-carbon bond, and contain hydrogen and carbon atoms in the backbone. The alkyl includes not only a linear form but also a branched form. Preferred examples of the alkyl include alkyl having 1 to 20 carbon atoms ($C_1$-$C_{20}$; hereinafter, "$C_p$-$C_q$" means that the number of carbon atoms is p to q), preferably $C_1$-$C_{10}$ alkyl, and more preferably $C_1$-$C_6$ alkyl. Specific examples of the alkyl include methyl, ethyl, n-propyl, i-propyl, n-butyl, s-butyl, t-butyl, isobutyl (also referred to as 2-methylpropyl), n-pentyl, s-pentyl (also referred to as 1-methylbutyl), t-pentyl (also referred to as 1,1-dimethylpropyl), neopentyl (also referred to as 2,2-dimethylpropyl), isopentyl (also referred to as 3-methylbutyl), 3-pentyl (also referred to as 1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The term "alkoxy" as used herein means an oxy group to which the "alkyl" as defined above is bonded. As the alkoxy, a $C_1$-$C_6$ alkoxy is preferred, and a $C_1$-$C_4$ alkoxy is more preferred. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The "haloalkoxy" as used herein means a group in which one or more hydrogen atoms of the "alkoxy" defined above are replaced with halogen. As haloalkoxy, halo $C_1$-$C_6$ alkoxy is preferred, and fluoro $C_1$-$C_6$ haloalkoxy is more preferred. Specific examples of the haloalkoxy include difluoromethoxy, trifluoromethoxy, 2,2-difluoroethoxy, 2,2,2-trifluoroethoxy, 3-fluoropropoxy, and 2,2-difluoropropoxy.

The term "alkenyl" as used herein is a monovalent group having at least one double bond (two adjacent $SP^2$ carbon atoms). Depending on the configuration of the double bond and a substituent (if present), the geometry of the double bond can assume entgegen (E) or zusammen (Z) and cis or trans conformations. The alkenyl includes not only a linear form but a branched form. Preferred examples of the alkenyl include a $C_2$-$C_{10}$ alkenyl, and more preferred examples thereof include a $C_2$-$C_6$ alkenyl. Specific examples of the alkenyl include vinyl, allyl, 1-propenyl, 2-propenyl, 1-butenyl, 2-butenyl (which includes cis and trans), 3-butenyl, pentenyl, 3-methyl-2-butenyl, and hexenyl.

As used herein, the term "alkynyl" is a monovalent group having at least one triple bond (two adjacent SP carbon atoms). The alkynyl includes not only a linear form but a branched form. Preferred examples of the alkynyl include a $C_2$-$C_{10}$ alkynyl, and more preferably a $C_2$-$C_6$ alkynyl. Specific examples of the alkynyl include ethynyl, 1-propynyl, propargyl, 3-butynyl, pentynyl, hexynyl, 3-phenyl-2-propynyl, 3-(2-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein means a saturated or partially saturated cyclic monovalent aliphatic hydrocarbon group and includes a monocyclic ring, a bicyclo ring, and a spiro ring. The cycloalkyl is preferably a $C_3$-$C_8$ cycloalkyl or a $C_3$-$C_7$ cycloalkyl, and more preferably a $C_3$-$C_6$ cycloalkyl. Specific examples of the cycloalkyl include cyclopropyl, cyclobutyl, cyclopentyl, cyclohexyl, cycloheptyl, cyclooctyl, bicyclo[2.2.1]heptyl, and spiro[3.3]heptyl.

The term "aryl" as used herein means a monovalent aromatic hydrocarbon ring, that is, an aromatic hydrocarbon ring group. Preferred examples of the aryl include a $C_6$-$C_{10}$ aryl. Specific examples of the aryl include phenyl and naphthyl (e.g., 1-naphthyl and 2-naphthyl).

The term "aryl group" as used herein may include the following "heteroaryl".

The term "heteroaryl" as used herein means a monovalent aromatic cyclic group containing a carbon atom as well as 1 to 5 heteroatoms, that is, an aromatic heterocyclic group. The ring may be a monocyclic or fused to another ring, and may be partially saturated. The number of atoms composing the ring of heteroaryl is preferably 5 to 10 (5- to 10-membered heteroaryl), and more preferably 5 to 7 (5- to 7-membered heteroaryl).

Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, and imidazopyridyl, pyrazolopyridyl, imidazopyridyl, triazolopyridyl, pyrrolopyrazinyl, and furopyridyl.

The "aralkyl (arylalkyl)" as used herein means a group in which at least one hydrogen atom of the "alkyl" as defined herein is replaced with the "aryl" as defined herein. The aralkyl is preferably $C_7$-$C_{14}$ aralkyl or $C_5$-$C_{10}$ aryl-$C_1$-$C_6$ alkyl, more preferably $C_7$-$C_{10}$ aralkyl. Specific examples of the aralkyl include benzyl, phenethyl, and 3-phenylpropyl.

The term "alkylene" as used herein means a divalent group derived from the "alkyl" as used herein by further removal of any one hydrogen atom, and is preferably $C_4$-$C_8$ alkylene. Examples of the alkylene specifically include $-CH_2-$,

$-(CH_2)_2-$, $-(CH_2)_3-$, $-CH(CH_3)CH_2-$, $-C(CH_3)_2-$, $-(CH_2)_4-$, $-CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$, $-CH_2CH(CH_2CH_3)-$, $-(CH_2)_5-$, $-CH(CH_3)CH(CH_2CH_3)-$, $-(CH_2)_6-$, $-(CH_2)_7-$, and $-(CH_2)_8-$. The alkylene is preferably $C_1$-$C_8$ alkylene, more preferably $C_4$-$C_8$ alkylene.

The "arylene" as used herein means a divalent group derived from the "aryl" defined herein by the further removal of any one hydrogen atom. The arylene may be a monocyclic ring or a condensed ring. The number of atoms composing the ring of arylene is not particularly limited and is preferably 6 to 10 ($C_6$-$C_{10}$ arylene). Examples of the arylene specifically include 1,2-phenylene, 1,3-phenylene, 1,4-phenylene, 1,2-naphthylene, 1,3-naphthylene, and 1,4-naphthylene.

The "heteroarylene" as used herein means a divalent group derived from the "heteroaryl" defined herein by the further removal of any one hydrogen atom. The heteroarylene may be a monocyclic ring or a condensed ring. The number of atoms composing the ring is not particularly limited and is preferably 5 to 10 (5- to 10-membered heteroarylene). Specific examples of the heteroarylene include pyrroldiyl, imidazoldiyl, pyrazoldiyl, pyridinediyl, pyridazinediyl, pyrimidinediyl, pyrazinediyl, triazoldiyl, triazinediyl, isooxazoldiyl, oxazoldiyl, oxadiazoldiyl, isothiazoldiyl, thiazoldiyl, thiadiazoldiyl, furanediyl, and thiophenediyl.

The term "alkylene arylene" as used herein means a divalent group in which the alkylene and the arylene are bonded at any position, and the alkylene is attached to the basic skeleton side. Specific examples of the alkylene arylene include $-C_1$-$C_6$ alkylene-$C_6$-$C_{10}$ arylene.

The term "arylene alkylene" as used herein means a divalent group in which the arylene and the alkylene are bonded at any position, and the arylene is attached to the basic skeleton side. Specific examples of the arylene alkylene include $-C_6$-$C_{10}$ arylene-$C_1$-$C_6$ alkylene.

The term "alkylene heteroarylene" as used herein means a divalent group in which the alkylene and the heteroarylene are bonded at any position, and the alkylene is attached to the basic skeleton side. Specific examples of the alkylene heteroarylene include $-C_1$-$C_6$ alkylene-5-to 10-membered heteroarylene.

The term "heteroarylene alkylene" as used herein means a divalent group in which the heteroarylene and the alkylene are bonded at any position, and the heteroarylene is attached to the basic skeleton side. Specific examples of the heteroarylene alkylene include -5- to 10-membered heteroarylene-$C_1$-$C_6$ alkylene.

The term "active ester group" as used herein refers to a group containing a carbonyl group that reacts with an amino group to form an amide bond, where the carbonyl group is bound to, for example, OBt, OAt, OSu, OPfp, SR1, or the like, and being capable of promoting the reaction with an amino group.

The "reaction auxiliary group" is a group introduced near a functional group to be bound to activate the binding reaction of the functional group for selectively inducing reactions at a desired position. For example, the reaction auxiliary group can be introduced on to either the carbonyl group or the amino group, or both to facilitate a reaction between the carbonyl group and the amino group. Examples of such a reaction auxiliary group include an SH group. This type of reaction auxiliary group can be removed along with the binding reaction or after the binding reaction.

The term "amino acid" as used herein refers to a carboxylic acid having an amino group, and includes a natural amino acid and a non-natural amino acid. The term "amino acid" as used herein may mean an amino acid residue. The "natural amino acid" means any of the L-type amino acids selected from Gly, Ala, Ser, Thr, Val, Leu, Ile, Phe, Tyr, Trp, His, Glu, Asp, Gln, Asn, Cys, Met, Lys, Arg, and Pro. The term "non-natural amino acid" means an amino acid other than natural amino acids. Examples of the non-natural amino acid include a β-amino acid, a γ-amino acid, an L-type amino acid (excluding the aforementioned natural amino acids), a D-type amino acid, an N-substituted amino acid, an α,α-disubstituted amino acid, and an amino acid having a side chain different from that of natural amino acids. Modified amino acids herein may be denoted as "MeAsp-pip" and the like. "MeAsp-pip" refers to an amino acid where the N-terminal amino group of aspartic acid is substituted with a methyl group (i.e., N-methylated), and the C-terminal carboxy group is amidated with piperidine. In similar notation, the three-letter abbreviation of the amino acid is shown in the center, with the letter at the left side indicating the substituent of the N-terminal amino group, and the letter at the right side indicating the substituent of the C-terminal carboxy group. Notations without letters on the left and right sides indicate amino and carboxy groups, respectively. "bAla" represents β-alanine, and "gMeAbu" represents 4-(methylamino)butanoic acid. β-amino acid and γ-amino acid are represented with the letters 'b' and 'g', respectively, on the far left.

As the amino acid as used herein, any steric configuration is acceptable. The selection of a side chain of the amino acid is not particularly limited, and the side chain is freely selected from, in addition to a hydrogen atom, for example, an alkyl group, an alkenyl group, an alkynyl group, an aryl group, a heteroaryl group, an aralkyl group, a heteroaralkyl group, a cycloalkyl group, and a spiro-bonded cycloalkyl group. A substituent may be further added to each of the substituents. Such a substituent is not limited and can be one or two or more substituents each independently freely selected from any substituents including a halogen atom, an oxygen atom, a nitrogen atom, a sulfur atom, a boron atom, a silicon atom, or a phosphorus atom. That is, examples of the side chain include an optionally substituted alkyl group, alkoxy group, alkoxyalkyl group, alkenyl group, alkynyl group, aryl group, heteroaryl group, aralkyl group, or cycloalkyl group that further has a substituent that is optionally added. Examples of the substituent that can be optionally added to a side chain of amino acids include deuterium, cyano, nitro, and amino carbonyl. In one non-limiting embodiment, the amino acid as used herein may be a compound having a carboxyl group and an amino group in the same molecule. Even in this case,

compounds in which the nitrogen atom of the amino group and any atom of the side chain together form a ring, such as proline, hydroxyproline, and azetidine-2-carboxylic acid, are also included in the amino acid.

The term "backbone of an amino acid" as used herein means, in the case of an α-amino acid, a chain portion composed of the amino group, the α-carbon, and the carboxy group; in the case of a β-amino acid, a chain portion composed of the amino group, the β-carbon, the α-carbon, and the carboxyl group; and in the case of a γ-amino acid, a chain portion composed of the amino group, the γ-carbon, the β-carbon, the α-carbon, and the carboxy group. As used herein, in the case of an amino acid with two carboxy groups in the amino acid, such as aspartic acid, which can be considered either as an α-amino acid or as a β-amino acid, the amino acid is defined as an amino acid with the shorter amino acid chain composed of an amino group, carbons, and a carboxy group. That is, in the case of aspartic acid, it is defined as an "α-amino acid". Also, the term "α-amino acid skeleton" means a chain portion composed of the amino group, the α-carbon, and the carboxyl group; the term "β-amino acid skeleton" means a chain portion composed of the amino group, the β-carbon, the α-carbon, and the carboxy group; and the term "γ-amino acid skeleton" means a chain portion composed of the amino group, the γ-carbon, the β-carbon, the α-carbon, and the carboxy group. For example, aspartic acid also falls under the category of "amino acid having a β-amino acid skeleton" because it has a β-amino acid skeleton. Furthermore, glutamic acid also falls under the category of "amino acid having a γ-amino acid skeleton" because it has a γ-amino acid skeleton.

The backbone amino group of the amino acid may be unsubstituted ($NH_2$ group) or may be substituted (i.e., a -NHR group wherein R represents alkyl, alkenyl, alkynyl, aryl, heteroaryl, aralkyl, or cycloalkyl optionally having a substituent, and one or two non-adjacent methylene groups in any of these groups may be substituted by an oxygen atom, a carbonyl group (-C(=O)-), or a sulfonyl group ($-SO_2-$); and a carbon chain bonded to a N atom and a carbon atom at position α may form a ring, as in proline). The substituent of the R is selected in the same manner as in the substituent for the amino acid side chain mentioned above. In the case of a substituted backbone amino group, the R is included in the "side chain of the amino acid" as used herein. As used herein, such an amino acid having the substituted backbone amino group is referred to as the "N-substituted amino acid". The "N-substituted amino acid" as used herein is preferably an N-alkyl amino acid, and may be an $N-C_1-C_6$ alkyl amino acid or an $N-C_1-C_4$ alkyl amino acid. Preferred N-substituted amino acids may be N-methyl amino acid or N-ethyl amino acid, and more preferably N-methyl amino acid.

The term "side chain" as used herein refers to a portion other than the backbone structure and is used in the context of a side chain of an amino acid, a side chain of a cyclic portion of a cyclic peptide compound, or the like. However, in the cyclic peptide-nucleic acid complex mentioned below, the nucleic acid portion and the nucleic acid linking portion are not contained in the side chain.

The "amino acid" composing a peptide compound includes all isotopes corresponding to each. The isotope of the "amino acid" is a form in which at least one atom is replaced with an atom having the same atomic number (the number of protons) and a different mass number (the sum of numbers of protons and neutrons). Examples of the isotope included in the "amino acid" composing the peptide compound of the present invention include an isotope of a hydrogen atom, a carbon atom, a nitrogen atom, an oxygen atom, a phosphorus atom, a sulfur atom, a fluorine atom, a chlorine atom, or the like, including, for example, $^{2}H$, $^{3}H$; $^{13}C$, $^{14}C$; $^{15}N$; $^{17}O$, $^{18}O$, $^{31}P$, $^{32}P$; $^{35}S$; $^{18}F$; $^{36}Cl$.

Examples of the halogen atom include fluoro (-F), chloro (-Cl), bromo (-Br), and iodo (-I). Examples of the substituent derived from a halogen atom include, in addition to a halogen atom, an alkyl group, a cycloalkyl group, an alkenyl group, an alkynyl group, an aryl group, a heteroaryl group, and an aralkyl group which may be substituted with one or more halogen atoms.

Examples of the substituent include a halogen atom, a hydroxy group (-OH), a carboxy group, an oxo group (=O), a sulfhydryl group (-SH, also known as a thiol group), a thiocarboxy group, an oxy group (-O-), a thioether group (-S-), a sulfoxide group (-S(=O)-), a sulfone group ($-S(=O)_2-$), a sulfo group ($-SO_3H$), an azide group ($-N_3$), and a cyano group (-CN). The substituent may be a combination of two or more of these which forms ester, thioester, amide, carbamate, an alkylthio group (-SR), sulfonamide, sulfamide, a thiocarboxylic acid group (-C(=O)-SH), a keto acid ($-C(=O)-CO_2H$), or a urea group.

The alkoxy group (-OR) is selected from an alkyl alkoxy group, a cycloalkyl alkoxy group, an alkenyl alkoxy group, an alkynyl alkoxy group, an aryl alkoxy group, a heteroaryl alkoxy group, an aralkyl alkoxy group, and the like. Examples of the substituent derived from an oxo group include a hydrocarbonyl group (-CHO, yielding an aldehyde as a compound), an alkylcarbonyl group (yielding a ketone as a compound), a cycloalkylcarbonyl group, an alkenylcarbonyl group, an alkynylcarbonyl group, an arylcarbonyl group, a heteroarylcarbonyl group, and an aralkylcarbonyl group. Examples of the substituent forming an ester include a carbonyloxy group (-O-C(=O)-) and an alkoxy carbonyl group (-C(=O)O-). The alkoxy carbonyl group is selected from an alkyloxy carbonyl group, a cycloalkyloxy carbonyl group, an alkenyloxy carbonyl group, an alkynyloxy carbonyl group, an aryloxy carbonyl group, a heteroaryloxy carbonyl group, an aralkyloxy carbonyl group, and the like. The carbonyloxy group is selected from an alkyl carbonyloxy group, a cycloalkyl carbonyloxy group, an alkenyl carbonyloxy group, an alkynyl carbonyloxy group, an aryl carbonyloxy group, a heteroaryl carbonyloxy group, an aralkyl carbonyloxy group, and the like.

Examples of the substituent that forms a thioester include a carbonylthio group (-S-C(=O)-) and an alkylthiocarbonyl group (-C(=O)-SR). The substituent that forms a thioester is selected, for example, from an alkylcarbonylthio group, a cycloalkylcarbonylthio group, an alkenylcarbonylthio group, an alkynylcarbonylthio group, an arylcarbonylthio group, a heteroarylcarbonylthio group, an aralkylcarbonylthio group, and the like, or from an alkylthiocarbonyl group, a cycloalkylthiocarbonyl group, an alkenylthiocarbonyl group, an alkynylthiocarbonyl group, an arylthiocarbonyl group, a heteroarylthiocarbonyl group, and an aralkylthiocarbonyl group.

Examples of the substituent that forms an amide include an alkylcarbonylamino group (-NH-C(=O)-R), a cycloalkylcarbonylamino group, an alkenylcarbonylamino group, an alkynylcarbonylamino group, a cycloalkylcarbonylamino group, an arylcarbonylamino group, a heteroarylcarbonylamino group, and an aralkylcarbonylamino group, or include an alkylaminocarbonyl group (-C(=O)-NHR), a cycloalkylaminocarbonyl group, an alkenylaminocarbonyl group, an alkynylaminocarbonyl group, an arylaminocarbonyl group, a heteroarylaminocarbonyl group, and an aralkylaminocarbonyl group. Further examples include compounds in which the H atom bonded to the N atom is replaced with an alkyl group, a cycloalkyl group, an alkenyl group, an alkynyl group, an aryl group, a heteroaryl group, or an aralkyl group.

Examples of the substituent that forms a carbamate include an alkyl oxycarbonylamino group (-NH-CO-OR), a cycloalkyl oxycarbonylamino group, an alkenyl oxycarbonylamino group, an alkynyl oxycarbonylamino group, an aryl oxycarbonylamino group, a heteroaryl oxycarbonylamino group, and an aralkyl oxycarbonylamino group. Further examples include compounds in which the H atom bonded to the N atom is replaced with an alkyl group, a cycloalkyl group, an alkenyl group, an alkynyl group, an aryl group, a heteroaryl group, or an aralkyl group.

Examples of the substituent that forms a sulfonamide include an alkylsulfonylamino group (-NH-$SO_2$-R), a cycloalkylsulfonylamino group, an alkenylsulfonylamino group, an alkynylsulfonylamino group, a cycloalkylsulfonylamino group,an arylsulfonylamino group, a heteroarylsulfonylamino group, and an aralkylsulfonylamino group, or include an alkylaminosulfonyl group (-$SO_2$-NHR), a cycloalkylaminosulfonyl group, an alkenylaminosulfonyl group, an alkynylaminosulfonyl group, an arylaminosulfonyl group, a heteroarylaminosulfonyl group, and an aralkylaminosulfonyl group. Further examples include compounds in which the H atom bonded to the N atom is replaced with an alkyl group, a cycloalkyl group, an alkenyl group, an alkynyl group, an aryl group, a heteroaryl group, or an aralkyl group.

Examples of the substituent that forms a sulfamide include an N-alkylsulfamoylamino group (-NH-$SO_2$-NHR), an N-cycloalkylsulfamoylamino group, an N-alkenylsulfamoylamino group, an N-alkynylsulfamoylamino group, an N-cycloalkylsulfamoylamino group, an N-arylsulfamoylamino group, an N-heteroarylsulfamoylamino group, and an N-aralkylsulfamoylamino group. Furthermore, a compound in which hydrogen atoms bonded to a nitrogen atom are replaced with two substituents selected from an alkyl group, a cycloalkyl group, an alkenyl group, an alkynyl group, an aryl group, a heteroaryl group, and an aralkyl group, which may be the same or different, and which may optionally form a ring.

The substituent that forms a thioether is selected from an alkylthio group, a cycloalkylthio group, an alkenylthio group, an alkynylthio group, an arylthio group, a heteroarylthio group, an aralkylthio group, and the like. The substituent that forms a sulfoxide group (-S(=O)-R) is selected from an alkylsulfinyl group, a cycloalkylsulfinyl group, an alkenylsulfinyl group, an alkynylsulfinyl group, an arylsulfinyl group, a heteroaryl sulfinyl group, an aralkyl sulfinyl group, and the like. The substituent that forms a sulfone group (-S$(=O)_2$-R) is selected from an alkylsulfonyl group, a cycloalkylsulfonyl group, an alkenylsulfonyl group, an alkynylsulfonyl group, an arylsulfonyl group, a heteroarylsulfonyl group, an aralkylsulfonyl group, and the like.

Examples of the substituent that forms a primary amine include an amino group (-$NH_2$). Examples of the substituent that forms a secondary amine (-NH-R) include an alkylamino group, a cycloalkylamino group, an alkenylamino group, an alkynylamino group, an arylamino group, a heteroarylamino group, and an aralkylamino group. Examples of the substituent that forms a tertiary amine (-NR($R^1$)) such as alkyl(aralkyl)amino group include any two substituents selected from an alkyl group, a cycloalkyl group, an alkenyl group, an alkynyl group, an aryl group, a heteroaryl group, and an aralkyl group, which may be the same or different, and which may optionally form a ring. Examples of the substituent that forms an amidino group (-C(=NR)-N$R^1R^2$) include an amidino group (-C(=NH)-$NH_2$) and a group in which three substituents on the nitrogen atoms are replaced by three selected from an alkyl group, a cycloalkyl group, an alkenyl group, an alkynyl group, an aryl group, a heteroaryl group, and an aralkyl group, which may be the same or different, such as an alkyl(aralkyl)(aryl) amidino group. Examples of the substituents that forms a guanidino group (-NR-C(=N$R^3$)-N$R^1R^2$) include a guanidine group (-NH-C(=NH)-$NH_2$) and a group in which R, $R^1$, $R^2$, and $R^3$ are replaced by four substituents selected from an alkyl group, a cycloalkyl group, an alkenyl group, an alkynyl group, an aryl group, a heteroaryl group, and an aralkyl group, which may be the same or different, and which may optionally form a ring.

Examples of the substituent that forms a urea group include an amino carbamoyl group (-NR-CO-N$R^1R^2$). R, $R^1$, and $R^2$ are any three substituents , which may be the same or different, selected from a hydrogen atom, an alkyl group, a cycloalkyl group, an alkenyl group, an alkynyl group, an aryl group, a heteroaryl group, and an aralkyl group and which may optionally be taken together to form a ring.

Examples of the functional group derived from a boron atom include alkylborane (-BR($R^1$)) and alkoxyborane (-B(OR)(O$R^1$)). These two substituents are any two substituents, which may be the same or different, selected from an alkyl group, a cycloalkyl group, an alkenyl group, an alkynyl group, an aryl group, a heteroaryl group, and an aralkyl group and which may optionally be taken together to form a ring.

In this way, various functional groups containing an O atom, an N atom, an S atom, a B atom, a P atom, an Si atom, and a halogen atom commonly used in low-molecular-weight compounds may be present in one or two or more. In other words, one of these substituents such as an alkyl group, a cycloalkyl group, an alkenyl group, an alkynyl group, an aryl group, a heteroaryl group, and an aralkyl group may be further substituted with one or more additional substituents. The conditions in which functional groups meet all of these requirements are defined as the free selection of substituents. In the case of β- or γ-amino acids, any stereochemical configuration is permitted, similarly to α-amino acids, and the selection of side chains is also not particularly limited, as in the case of α-amino acids. The amino group moiety of the backbone of the amino acid may be free ($-NH_2$ group) or N-alkylated such as N-methylated (-NHR group, wherein R represents an optionally substituted alkyl group, alkenyl group, alkynyl group, aryl group, heteroaryl group, aralkyl group, or cycloalkyl group, and the carbon atom bound to the N atom and the carbon atom from the side chain (e.g., at the α-position) may together form a ring as in proline. The substituents can be freely selected, and examples thereof include a halogen atom, an ether group, and a hydroxy group.).

The term "translation amino acid" or "translatable amino acid" as used herein refers to those "amino acids" that have a side chain capable of being translated. In one non-limiting embodiment, the amino acid used herein may be a translatable amino acid.

The term "drug-likeness" or "drug-like" as used herein refers to the properties of excellent metabolic stability and membrane permeability.

The term "drug-like amino acid" as used herein refers to an amino acid with the same skeleton as "amino acid," namely, α, β, and y amino acids, where one of the two hydrogen atoms of the backbone amino group ($NH_2$ group) and one or two hydrogen atoms of the methylene group ($-CH_2-$ group) may be substituted with an alkyl group, a cycloalkyl group, an alkenyl group, an alkynyl group, an aryl group, a heteroaryl group, an aralkyl group, and the like. The group in which a hydrogen atom of the $-CH_2-$ group is replaced with the above-mentioned group forms a side chain. These substituents include those that are further substituted with a substituent that functions as a component of drug-like peptide compounds. These are preferably selected from the substituents separately defined above. For example, it may be substituted by one or more substituents selected from a hydroxy group, an alkoxy group, an amide group, a sulfonyl group, a sulfinyl group, a halogen atom, a hydroxyamino group, an amino hydroxy group, and the like. Furthermore, it may correspond to either L-amino acid or D-amino acid, or α,α-dialkylamino acid. The drug-like amino acid does not necessarily have to be translatable. The drug-like amino acid includes a side chain portion of a peptide obtained from "translation amino acids" (for example, if a hit compound is obtained with D-tyrosine, a chemically modified D-type amino acid derived from D-tyrosine, and if a hit compound is obtained with β-alanine, a chemically modified β-amino acid derived from β-alanine) and all amino acids that can be chemically synthesized through structural optimization of the N-substituted portion by chemical conversion of N-methylamino acids. These amino acids function as components of drug-like peptide compounds, and thus are selected from a range that ensures the resulting peptide compounds obtained through post-translational chemical modifications exhibit drug-like properties. For example, as mentioned below, lysine with an aminoalkyl group is not included in drug-like amino acids when the amino group is not involved in post-translational modifications. However, when using the amino group of lysine as a reactive functional group for post-translational modifications (such as a cross unit), the lysine unit is included as a unit of drug-like amino acids. In this way, whether an amino acid corresponds to a "drug-like amino acid" is determined by the functional group after being converted by post-translation modifications. Examples of the substituent that may serve as such a substituent include, from among those defined separately above, an ester group, a thioester group, a sulfhydryl group, or a protected thiol group, an amino group, a monosubstituted amino group or di-substituted amino group, a protected amino group, a substituted sulfonylamino group, an alkylborane group, an alkoxyborane group, an azido group, a keto acid group, a thiocarboxy group, a phosphoryl ester group, and an acyloxyamino group. Details will be described later, but in one embodiment of the present disclosure, an indole skeleton and/or a substituted or unsubstituted hydroxyphenyl group are not included in the drug-like amino acids in the present disclosure.

The term "substituted hydroxyphenyl group" as used herein means a group in which at least one hydrogen atom of the aromatic ring of the hydroxyphenyl group is replaced with a substituent. The substituent is selected in the same manner as in the substituent for the amino acid side chain mentioned above. Examples of the substituted hydroxyphenyl group include, but are not intended to be limited to, a 3-fluoro-4-hydroxyphenyl group. The hydrogen atom of the aromatic ring as used herein does not include H of the hydroxy group (-OH) in the hydroxyphenyl group. For example, a methoxyphenyl group is not included in either the "substituted hydroxyphenyl group" or the "unsubstituted hydroxyphenyl group" as used herein.

The term "unsubstituted hydroxyphenyl group" as used herein means an unsubstituted hydroxyphenyl group. The substituted hydroxyphenyl group and the unsubstituted hydroxyphenyl group may be collectively referred to as a "substituted or unsubstituted hydroxyphenyl group".

The phrase "does not have a substituted or unsubstituted XX group" as used herein means that neither a substituted XX group nor an unsubstituted XX group is present.

The term "translation-based synthesis" as used herein means translating and synthesizing a peptide compound

from a nucleic acid (e.g., DNA, RNA) that encodes the peptide compound. Translation is the process of obtaining a linear peptide by repeating amide or ester bond reactions using mRNA as a template through the action of ribosomes.

The term "post-translation modification" refers to a chemical reaction that occurs after translation, independently of the action of ribosomes, either spontaneously or upon addition of another reagent, such as a cyclization reaction and a deprotection reaction.

The term "consist substantially of" as used herein means that the proportion of the theoretical number of variations of the recited peptide compounds and/or nucleic acids encoding them contained in a library to the theoretical total number of variations of the peptide compounds contained in the library and/or nucleic acids encoding them may be, but is not limited to, 90% or more, 93% or more, 95% or more, 97% or more, 98% or more, or 99% or more.

The term "substantially free of' as used herein means that the listed components (such as, but not limited to, cyclic peptide compounds, amino acids, or nucleic acids) are not included, or even if they are included, the components do not negatively affect the effects in one embodiment of the present invention or in amounts or in embodiments that do not give negative impact. For example, components listed therein may be contained as long as they are components that do not negatively affect the effects in one embodiment of the present invention or in amounts or in embodiments that do not have a negative impact.

The "negative impact" used herein in the context relating to an effect of an invention refers to an impact that negates the effect of the invention. For example, in cases where the original effectiveness of an invention is assumed to be 100%, a "negative impact" can be considered if its effectiveness drops to 30%, 20%, 10%, or 5% or less. For example, in a case where the hit rate that should be originally obtained by panning is 1%, a negative impact can be considered if the hit rate falls to 0.3% or less. Furthermore, in cases where the proportion of a hit compound without Trp that should be originally obtained by panning is 100%, a negative impact can be considered if the proportion of the hit compound without Trp falls to 30% or less. However, the negative impact is not limited to these cases.

Peptide compound

While not intended to be limited, the term "peptide compound" in the present disclosure includes a linear peptide compound and a cyclic peptide compound. The peptide compound in the present disclosure also includes a peptide, a complex of peptide and nucleic acid (peptide-nucleic acid complex), a complex of peptide and ribosome and nucleic acid, and the like. While not intended to be limited, the peptide compounds of the present disclosure include a linear peptide, a cyclic peptide, a complex of a linear peptide moiety and a nucleic acid (linear peptide-nucleic acid complex), and a complex of a cyclic peptide moiety and a nucleic acid (cyclic peptide-nucleic acid complex). As used herein, the part of the peptide in the peptide-nucleic acid complex may be referred to as the "peptide moiety". The peptide compound of the present disclosure may contain an α-hydroxycarboxylic acid in place of some of the constituent amino acids. When a peptide compound contains an α-hydroxycarboxylic acid, the bond between each residue in the peptide compound may be a bond other than an amide bond, such as an ester bond. When counting the number of amino acid residues in a peptide compound containing an α-hydroxycarboxylic acid, the number of amino acid residues is counted including the number of α-hydroxycarboxylic acids. Examples of the α-hydroxycarboxylic acid include, in addition to glycolic acid, D-lactic acid, and L-lactic acid, compounds in which the amino group of the aforementioned "α-amino acid" is replaced by a hydroxy group.

The "nucleic acid" in the present disclosure includes DNA, mRNA, and tRNA. As used herein, the "amino acid" composing a peptide compound is sometimes simply referred to as "amino acid residue". The "peptide compound" as used herein may also include a pharmaceutically acceptable salt thereof.

The term "peptide compound" or "peptide moiety" as used herein refers to a peptide in which 2 to 100, 3 to 50, or 4 to 30 amino acids are linked by an amide bond and/or an ester bond. In such cyclic peptide compounds, the carboxy group present in the backbone or side chain of the corresponding linear peptide compound is cyclized with an amino group or hydroxy group to form a cyclic portion. The bonding mode of cyclization is not particularly limited, but, for example, a covalent bond such as an amide bond, a carbon-carbon bond, a disulfide bond, an ester bond, a thioester bond, a thioether bond, a lactam bond, a bond via a triazole structure, or a bond via a fluorophore structure is preferred. Among them, cyclization via an amide bond tends to provide cyclic peptide compounds with higher metabolic stability.

The "cyclization reaction" as used herein refers to a reaction that forms a cyclic portion by linking the carboxy group present in the backbone or side chain of a linear peptide compound with an amino group or hydroxy group. When a cyclic peptide compound in the present disclosure is chemically modified, the chemical modification may be performed on the linear peptide before the cyclization reaction, or the chemical modification may be performed on the cyclic peptide obtained after the cyclization reaction.

Scheme A is an example of the cyclization reaction. A white circle (○) unit (cross unit), a black circle (●) unit (cyclic portion backbone unit), a square (■) unit (linear portion backbone unit), and a triangle (A) unit (cyclic N-terminal unit) each represent the amino acid residue composing the peptide moiety. Each unit is classified based on its function, and these amino acids may be the same as or different from each other.

[Formula 1]

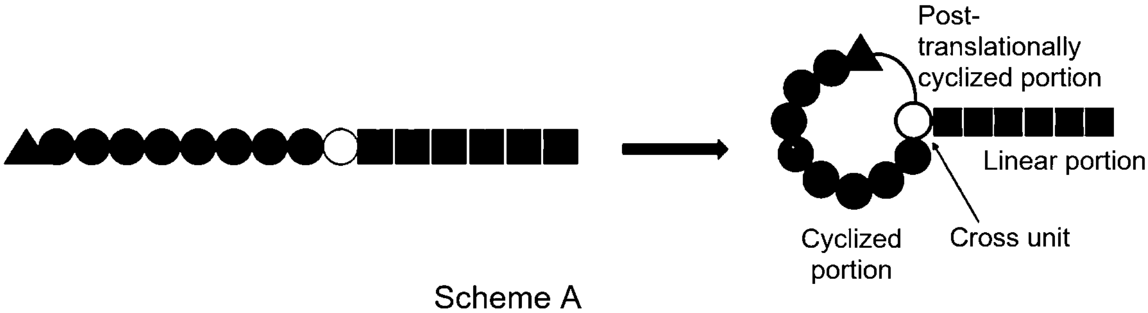


Scheme A

The "unit" as used herein refers to one of the following: an amino acid before cyclization after the synthesis of the linear peptide compound in the present disclosure, an amino acid after cyclization, or an amino acid at the completion of chemical modification after cyclization. Examples of the amino acid residue at the completion of chemical modification after cyclization include an amino acid residue translated from one tRNA and subsequently chemically converted or structurally modified through post-translational chemical modification.

In scheme A, the cyclic portion is a moiety consisting of one triangular unit, eight black circle units, and one white circle unit, while the linear portion is a moiety consisting of six square units. The curved portion of scheme A is a moiety to be cyclized (the moiety is also referred to herein as "cyclized portion" or "post-translationally cyclized portion").

In one embodiment, the peptide moiety of the peptide compound in the present disclosure requires a reactive functional group on the triangular or cross units in scheme A. Thus, drug-like amino acids are not necessarily selected for triangular or cross units in peptide compounds before cyclization.

In one embodiment, even when a triangular or cross unit in the peptide compound before cyclization is not a drug-like amino acid or is originally an unfavorable amino acid, it is possible to make the triangular or cross unit a drug-like amino acid or favorable amino acid by performing chemical modification after cyclization.

In one embodiment, for a triangular unit in the peptide moiety before cyclization (pre-cyclization-N-terminal unit) of the peptide compound in the present disclosure, an amino acid residue having a thiol group in the vicinity of the amino group, wherein both of the thiol group and the amino group are protected by protecting groups can be used.

In one embodiment, for a cross unit at the peptide moiety before cyclization of the peptide compound in the present disclosure, it is preferable to use an amino acid having a functional group capable of cyclization (second reaction point) by reacting with a functional group possessed by the amino acid of the triangular unit (first reaction point) in the side chain. Since the amino and carboxy groups of the backbone are used to form a covalent bond with other amino acid residues in translation-based synthesis and a third functional group is necessary for cyclization in the cross unit, it is preferable that the cross unit has three or more functional groups in total. Among these functional groups, for the cyclization reaction, it is preferable to use a functional group of the side chain moiety of the cross unit.

The cross unit can be incorporated at any position in the peptide moiety before cyclization as long as the position allows the cyclization. However, it is preferable that the cross unit is incorporated at the position in which the number of amino acids in the cyclic portion after cyclization or post-translation modification after cyclization is 13 or 14. In other words, it is preferable that the cross unit of the peptide compound in the present disclosure is incorporated at a position at least 12 amino acid residues (e.g., 12 or 13 amino acid residues) toward the C-terminus from the triangular unit.

The black circle units and square units are selected from amino acids, preferably from drug-like amino acids.

<u>- Cyclic peptide compound</u>

The cyclic peptide compound of the present invention is a compound formed by amino acids bonded through an amide or ester bond, and has a cyclic portion. The term "cyclic portion" of a cyclic peptide compound as used herein refers to a cyclic structure formed by the linkage of 13 or 14 amino acid residues or $\alpha$-hydroxycarboxylic acids through an amide bond, an ester bond, or a carbon-carbon covalent bond. It is preferable that cyclic peptide compound consists of 30 or less residues in total of the cyclic portion and the linear portion. Given the requirement to achieve both excellent membrane permeability and metabolic stability (drug-likeness), it is preferable that the number of amino acids composing the cyclic portion is 13 or 14. In one non-limiting embodiment, the cyclic portion of the cyclic peptide compound is formed by subjecting a peptide compound obtained by translation-based synthesis to a cyclization reaction. The cyclic peptide compound of the present invention may be further chemically modified. Examples of the chemically modified cyclic peptide compound include a cyclic peptide compound linked to a nucleic acid (cyclic peptide-nucleic acid complex). The nucleic acid may be linked to the cyclic peptide compound via a linker.

The cyclic portion of the cyclic peptide compound may have a side chain (also referred to as a "linear portion")

branching from the backbone that constitutes the cyclic structure, and the linear portion has at least one amide bond and/or ester bond. When the cyclic peptide compound of the present invention has a linear portion, it can be described as in scheme A-1 or A-2, for example. For example, when the cyclic peptide compound has a linear portion, it may be a peptide compound in which the cyclic portion includes amino acids such as Asp or Glu, and the carboxy group of the backbone or side chain of the Asp or Glu is linked to the amino group of the linear portion by an amide bond. The term "linear portion" as used herein contains natural amino acids and non-natural amino acids (including chemically modified or skeleton-transformed amino acids). When the total number of amino acids in the cyclic and linear portions is 13 or 14 residues, the membrane permeability tends to be higher. When the total number of amino acids in the cyclic and linear portions is 13 residues or more, the metabolic stability tends to be higher. The number of amino acids in the linear portion is preferably 0 to 8 residues, more preferably 0 to 3 residues, and most preferably 0 (that is, the linear portion is absent).

[Formula 2]

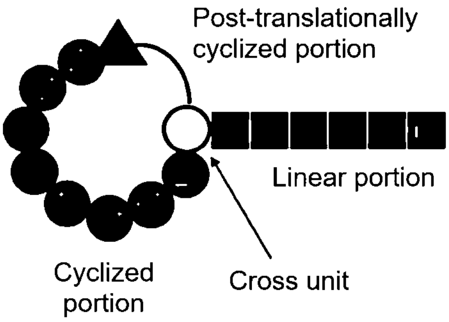


Scheme A-1

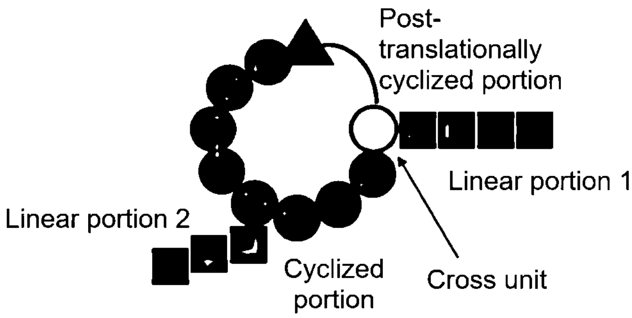


Scheme A-2

The term "number of amino acids" as used herein refers to the total number of amino acid residues or $\alpha$-hydroxycarboxylic acids composing a peptide, and means the number of amino acids or $\alpha$-hydroxycarboxylic acids generated upon cleavage of amide bonds, ester bonds, and bonds of cyclized portion that link the amino acids.

The term "amino acids composing the cyclic portion" as used herein refers to amino acids present on the backbone of the cyclic portion among the amino acids resulting from the cleavage of amide bonds, ester bonds, and bonds in the cyclized portion present in a cyclic peptide compound. The amino acid or $\alpha$-hydroxycarboxylic acid corresponding to the cross unit shown in the above scheme A-1 or A-2 is included in the amino acid that composes the cyclic portion.

In the cyclic peptide-nucleic acid complex, a cyclic peptide compound (peptide moiety) and a nucleic acid are linked directly or via a linker. In the cyclic peptide-nucleic acid complex, the nucleic acid is linked to the linear portion and not to the cyclic portion. As used herein, the entire linear portion to which the nucleic acid is linked is referred to as the "nucleic acid linking portion", and the parts of the peptide moiety other than a nucleic acid linking portion are referred to as the "peptide moiety other than a nucleic acid linking portion". In peptide compounds that are not linked to a nucleic acid, there is no nucleic acid linking portion, thus the entire peptide moiety is referred to as the "peptide moiety other than a nucleic acid linking portion". For example, in the aforementioned scheme A-2, when a nucleic acid is linked to the tip of the linear portion 1, the part other than the linear portion 1, namely the combined portion of the cyclic portion and linear portion 2, becomes the "peptide moiety other than a nucleic acid linking portion". In linear peptide-nucleic acid complexes, the entire linear peptide serves as the nucleic acid linking portion, thus there is no "peptide moiety other than a nucleic acid linking portion".

In one non-limiting embodiment, the cyclic peptide compound has a cyclic portion,

(1) the cyclic portion is composed of 13 or 14 amino acid residues,
(2) among the amino acid residues composing the cyclic portion, the number of N-substituted amino acid residues is 7 or more, the number of amino acid residues having the same side chain is 3 or less, and the number of natural amino acid residues is 4 or less, and
(3) ClogP/total amide bond (ClogP/total AB) is 1.0 to 1.8.

The number of amino acids composing the cyclic peptide compound (referred to as a peptide moiety when the cyclic peptide compound is linked to a nucleic acid) in the present disclosure is not particularly limited, but is preferably 30 or less. When a cyclic peptide compound is linked to a nucleic acid, the chemical structure of the cyclic peptide compound can be classified into a nucleic acid linking portion and a peptide moiety other than the nucleic acid linking portion. When the number of amino acids composing the peptide moiety other than the nucleic acid linking portion is 13 or 14, or when the number of amino acids composing the cyclic portion is 13 or 14, it tends to be possible to achieve both excellent membrane

permeability and metabolic stability.

In one non-limiting embodiment, the number of amino acids (number of units) of the linear portion is preferably 0 to 8, more preferably 0 to 5, and most preferably 0 to 3.

Among amino acid residues composing the cyclic portion, the number of amino acid residues having the same side chain is, for example, but not limited to, 5 or less, 4 or less, 3 or less, or 2 or less, preferably 4 or less, and more preferably 3 or less. In other words, the amino acids that compose the cyclic portion can include up to five amino acids having the same side chain.

The number of amino acids composing the cyclic portion is not limited, but examples include 13 or more, 14 or less, 13 to 14, 13 or 14. Given the requirement to achieve both excellent membrane permeability and metabolic stability, it is preferable that the number of amino acids composing the cyclic portion is 13 to 14, 13, or 14.

In one non-limiting embodiment, when the proportion of the number of N-substituted amino acids to the total number of amino acids composing the cyclic portion is 50% or more, 55% or more, 60% or more, 65% or more, 70% or more, or 75% or more, a cyclic peptide compound exhibiting higher membrane permeability can be obtained.

In one non-limiting embodiment, examples of the number of N-substituted amino acids composing the cyclic portion include, but not limited to, 7 or more, 8 or more, 9 or more, 10 or more, 14 or less, 13 or less, 12 or less, 11 or less, 7 to 14, 7 to 13, 7 to 12, 7 to 11, 7 to 10, 7 to 9, 8 to 14, 8 to 13, 8 to 12, 8 to 11, 8 to 10, 8 to 9, 9 to 14, 9 to 13, 9 to 12, 9 to 11, 9 to 10, 10 to 14, 10 to 13, 10 to 12, or 10 to 11. Given the requirement to achieve both excellent membrane permeability and metabolic stability, it is preferable that the number of N-substituted amino acids composing the cyclic portion is 7 or more, or 7 to 13, more preferably 8 or more, 9 or more, 10 or more, 7 to 10, or 8 to 10.

The cyclic peptide compound in the present disclosure is a compound with ClogP/total amide bond (ClogP/total AB) of 1.0 to 1.8. As used herein, the "total AB" is calculated as follows. When the cyclic peptide compound is not linked to a nucleic acid, the sum of all the amide bonds that make up the entire cyclic peptide compound (the number of amide bonds in both the backbone and side chain of each amino acid) is used as the "total AB". On the other hand, when a cyclic peptide compound is linked to a nucleic acid, the sum of the amide bonds of the backbone and side chains of the amino acids composing the peptide moiety other than a nucleic acid linking portion of the cyclic peptide compound is used as "total AB". When the bond between the nucleic acid linking portion and the peptide moiety other than a nucleic acid linking portion is an amide bond, that amide bond is also included in the calculation. ClogP/total AB is the value calculated by dividing ClogP by total AB. The cyclic peptide compound linked to a nucleic acid (cyclic peptide-nucleic acid complex) can be used as a component composing a display library used for screening, but a drug candidate compound that is expected to be employed in practice is solely of parts where the nucleic acid is not linked. Thus, when considering drug-likeness (for example, high membrane permeability), the properties of the peptide moiety other than the nucleic acid linking portion could become important.

In one non-limiting embodiment, the ClogP of the cyclic peptide compound in the present disclosure is preferably 4 or more and 25 or less to have good membrane permeability, and more preferably, 10 or more, 11 or more, 12 or more, or 13 or more, and 25 or less, 24 or less, 23 or less, 22 or less, 21 or less, or 20 or less, and further examples include 10 to 25, 11 to 24, 12 to 23, 13 to 22, 13 to 21, 13 to 20, 14 to 20, and 15 to 20. ClogP is a computer calculated partition coefficient and can be determined according to the principles described in "CLOGP Reference Manual Daylight Version 4.9 (release date: August 1, 2011, https://www.daylight.com/dayhtml/doc/clogp/)". Examples of the method for calculating ClogP include calculating ClogP using Daylight Version 4.95 (release date: August 1, 2011, ClogP algorithm version 5.4, database version 28, https://www.daylight.com/dayhtml/doc/release_notes/index.html) of Daylight Chemical Information Systems, Inc.

The principle described in "CLOGP Reference Manual Daylight Version 4.9 (release date: August 1, 2011, https://www.daylight.com/dayhtml/doc/clogp/)" is as described in paragraph [0101] of International Publication No. WO 2024/214825.

As used herein, when the cyclic peptide compound is not linked to a nucleic acid, the ClogP of the entire cyclic peptide compound is calculated. When the cyclic peptide compound is linked to a nucleic acid, the ClogP of the peptide moiety other than the nucleic acid linking portion is calculated as the "ClogP of the cyclic peptide compound". The "ClogP of the peptide moiety other than the nucleic acid linking portion" is a value of calculated ClogP for a hypothetical peptide in which the nucleic acid linking portion of the cyclic peptide compound is replaced with piperidine (pip). That is, the hypothetical peptide is a cyclic peptide in which, in the amide bond between the carboxy group of the backbone or side chain of the amino acids composing the cyclic portion (for example, amino acid residues having a carboxy group on the side chain such as Asp or Glu) and the amino group of the nucleic acid linking portion, piperidine (pip) is bound in place of the amino group of the nucleic acid linking portion. The cyclic peptide compound linked to a nucleic acid (cyclic peptide-nucleic acid complex) can be used as a component composing a display library used for screening, but a drug candidate compound that is expected to be employed in practice is solely of parts where the nucleic acid is not linked. Thus, when considering drug-likeness (for example, high membrane permeability), the properties of the peptide moiety other than a nucleic acid linking portion could become important.

In one non-limiting embodiment, the ClogP/total AB of the cyclic peptide compound in the present disclosure is

preferably 1.0 or more, more preferably 1.1 or more, and most preferably 1.2 or more. In addition, the ClogP/total AB of the cyclic peptide compound in the present disclosure is preferably 1.8 or less, 1.7 or less, 1.6 or less, 1.5 or less, and 1.4 or less. Examples of preferred ClogP/total AB include 1.0 to 1.8, 1.0 to 1.7, 1.0 to 1.6, 1.0 to 1.5, 1.0 to 1.4, 1.0 to 1.3, 1.0 to 1.2, 1.1 to 1.6, 1.1 to 1.5, 1.1 to 1.4, 1.1 to 1.3, or 1.1 to 1.2.

In one non-limiting embodiment, when the ClogP/totalAB of the cyclic peptide compound in the present disclosure is 1.0 or more and the number of N-substituted amino acids is 7 or more, the compound exhibits more excellent membrane permeability. When the ClogP/total AB of the cyclic peptide compound in the present disclosure is 1.1 or more and the number of N-substituted amino acids is 8 or more, the compound further enhanced membrane permeability.

In one non-limiting embodiment, the cyclic peptide compound contained in the present disclosure may have a hydrogen bond donor (proton donor) in the cyclic portion. The hydrogen bond donor is a group that provides a necessary hydrogen atom to form a hydrogen bond, and forms a hydrogen bond with a hydrogen bond acceptor (proton acceptor) in the same compound or in nearby compounds (including water molecules). The hydrogen bond donor is a group that contains a hydrogen atom covalently bonded to an atom having relatively high electronegativity, and examples include a hydroxy group, a carboxy group, an amino group, an unsubstituted amide group, and a monosubstituted amide group. The hydrogen bond acceptor is, for example, lone pairs on carbonyl oxygen in carboxy groups, esters, and amides, or amine nitrogen. When an intramolecular hydrogen bond is formed, the conformation of the peptide compound is fixed, thus the affinity with the target molecule can be enhanced. When an intermolecular hydrogen bond with the target molecule is formed, the affinity with the target molecule can be enhanced. Examples of the number of hydrogen bond donors include 8 or less, 7 or less, 6 or less, 5 or less, 4 or less, 3 or less, 2 or less, or 1 or less. When the number of hydrogen bond donors is 5 or less, 4 or less, or 3 or less, a better balance between excellent membrane permeability and metabolic stability can be achieved. The lower limit of the number of the hydrogen bond donors is not particularly limited, but examples include 0 or more, 1 or more, and 2 or more. Examples of the number of the hydrogen bond donors include 0 to 8, 0 to 7, 0 to 6, 0 to 5, 0 to 4, 0 to 3, 0 to 2, 0 to 1, 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 9, 3 to 8, 3 to 7, 3 to 6, 3 to 5, or 3 to 4.

To increase the hit rate of peptide compounds that have higher membrane permeability and can specifically bind to a target molecule, it is preferable that the side chains of the amino acids composing the cyclic portion of the cyclic peptide compounds include a group selected from drug-like substituents. The drug-like substituents can be selected from, for example, an optionally substituted alkyl group, cycloalkyl group, alkenyl group, alkynyl group, aralkyl group, aryl group, and heteroaryl group, which may be further substituted with, for example, a halogen atom, an amide group (-CO-NRR' or -NR-CO-R'), a sulfonyl group ($-SO_2-R$), or an alkoxy group (-OR) as substituents, wherein, R and R' are each independently selected from an optionally substituted alkyl group, cycloalkyl group, alkenyl group, alkynyl group, aryl group, heteroaryl group, and aralkyl group, or in the case of an amide group, R and R' together with the nitrogen atom to which they are attached may form a 4- to 8-membered saturated heterocycle, wherein the 4- to 8-membered saturated heterocycle may be substituted with at least one group independently selected from the group consisting of a halogen atom, an oxo group, a $C_1$-$C_6$ alkyl group, and a 4- to 7-membered heterocyclyl group.

However, since biological membranes are composed of phospholipids, polar functional groups that are highly prone to ionization under physiological conditions (around pH = 7) such as alkylamino groups and alkylguanidino groups are not very suitable for achieving high membrane permeability. It is preferable that the cyclic peptide compound in the present disclosure does not contain these functional groups.

In one non-limiting embodiment, the side chain of at least one amino acid composing the cyclic portion of the cyclic peptide compound in the present disclosure is composed of at least one selected from the group consisting of an alkyl group, a cycloalkyl group, an aralkyl group, and an amide group. Herein, an arbitrary atom of the alkyl group and the nitrogen atom of the amino group of the amino acid may be taken together to form a ring.

In one non-limiting embodiment, when the side chain of at least one amino acid composing the cyclic portion of the cyclic peptide compound in the present disclosure is an alkyl group, the alkyl group may be a $C_1$-$C_6$ alkyl group optionally substituted with at least one group selected from the group consisting of a halogen atom, a hydroxy group, a $C_3$-$C_8$ cycloalkyl group, and a $C_1$-$C_6$ alkoxy group.

In one non-limiting embodiment, when the side chain of at least one amino acid composing the cyclic portion of the cyclic peptide compound in the present disclosure is a cycloalkyl group, the cycloalkyl group may be a $C_3$-$C_{10}$ cycloalkyl group optionally substituted with at least one group selected from the group consisting of a halogen atom, a hydroxy group, a $C_1$-$C_6$ alkyl group, a $C_1$-$C_6$ haloalkyl group, a $C_1$-$C_6$ alkoxy group, and a $C_1$-$C_6$ haloalkoxy group.

In one non-limiting embodiment, when the side chain of at least one amino acid composing the cyclic portion of the cyclic peptide compound in the present disclosure is an aralkyl group, the aralkyl group may be a $C_7$-$C_{14}$ aralkyl group optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

In one non-limiting embodiment, when the side chain of at least one amino acid composing the cyclic portion of the cyclic peptide compound in the present disclosure is an aralkyl group, the aralkyl group may be an optionally

substituted $C_5$-$C_{10}$ aryl $C_1$-$C_6$ alkyl group, in which the $C_5$-$C_{10}$ aryl group is optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

In one non-limiting embodiment, when the side chain of at least one amino acid composing the cyclic portion of the cyclic peptide compound in the present disclosure is an amide group, the amide group may be represented by formula: $-CONR^1R^2$, wherein $R^1$ and $R^2$ are each independently a hydrogen atom or a $C_1$-$C_6$ alkyl group, or $R^1$ and $R^2$, together with a nitrogen atom to which they are attached, form a 4- to 8-membered saturated heterocyclic ring, and the 4- to 8-membered saturated heterocyclic ring is optionally substituted with at least one group independently selected from the group consisting of a halogen atom, an oxo group, a $C_1$-$C_6$ alkyl group, and a 4- to 7-membered heterocyclyl group.

In one non-limiting embodiment, the cyclic peptide compound in the present disclosure may be a cyclic peptide compound that contains at least one amide bond formed between a carboxy group contained in a side chain of an amino acid and a nitrogen atom of an amino group of another amino acid.

In one non-limiting embodiment, the cyclic peptide compound in the present disclosure may contain an amino acid selected from the group consisting of Asp-pip (see formula (i)) and MeAsp-pip (see formula (ii)).

[Formula 3]

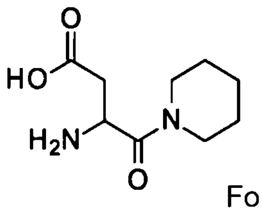

Formula (i)

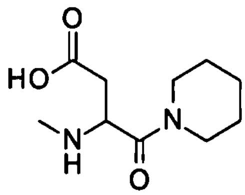

Formula (ii)

In one non-limiting embodiment, at least one amino acid composing the cyclic portion of the cyclic peptide compound in the present disclosure may be an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu. At least one amino acid composing the cyclic portion of the cyclic peptide compound in the present disclosure may be each independently an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.

In one non-limiting embodiment, the amino acids composing the cyclic portion of the cyclic peptide compound in the present disclosure may be, but are not particularly limited to, an $\alpha$-amino acid or an amino acid having a $\beta$-amino acid skeleton or a $\gamma$-amino acid skeleton. The number of $\alpha$-amino acids composing the cyclic portion of the cyclic peptide compound may be, for example, but is not limited to, 10 to 14, 10 to 13, 10 to 12, 10 to 11, 10, 11, 12, 13, or 14. The number of amino acids having a $\beta$-amino acid skeleton composing the cyclic portion of the cyclic peptide compound may be, but is not limited to, 0 to 4, 0 to 3, 0 to 2, 0 to 1, 1 to 4, 1 to 3, 1 to 2, 2 to 4, 2 to 3, 3 to 4, 0, 1, 2, 3, or 4. The number of amino acids having a $\gamma$-amino acid skeleton composing the cyclic portion of the cyclic peptide compound may be, but is not limited to, 0 to 4, 0 to 3, 0 to 2, 0 to 1, 1 to 4, 1 to 3, 1 to 2, 2 to 4, 2 to 3, 3 to 4, 0, 1, 2, 3, or 4.

In one non-limiting embodiment, the amino acids composing the cyclic portion of the cyclic peptide compound in the present disclosure may include, but are not particularly limited to, an $\alpha$-amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu). The $\alpha$-amino acids are each independently an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu).

In one non-limiting embodiment, the amino acids composing the cyclic portion of the cyclic peptide compound in the present disclosure may include, but are not particularly limited to, an amino acid having a $\beta$-amino acid skeleton selected from the group consisting of Asp-pip, bAla, bMeAla, D-3-Abu, D-3-MeAbu, and MeAsp-pip. The amino acids having a $\beta$-amino acid skeleton may be each independently an amino acid selected from the group consisting of Asp-pip, bAla, bMeAla, D-3-Abu, D-3-MeAbu, and MeAsp-pip.

In one non-limiting embodiment, the amino acids composing the cyclic portion of the cyclic peptide compound in the present disclosure may include or may be, but are not particularly limited to, gMeAbu.

In one non-limiting embodiment, the number of natural amino acids among amino acids composing the cyclic portion of the cyclic peptide compound in the present disclosure is, for example, 0 to 6, 0 to 5, 0 to 4, 0 to 3, 0 to 2, 0 to 1, 1 to 6, 1 to 5, 1 to 4, 1 to 3, 1 to 2, 2 to 6, 2 to 5, 2 to 4, 2 to 3, 3 to 6, 3 to 5, 3 to 4, 4 to 6, 4 to 5, and 5 to 6, and preferably 1 to 4.

In one non-limiting embodiment, the amino acids composing the cyclic portion of the cyclic peptide compound in

the present disclosure may include a natural amino acid selected from the group consisting of Gly, Ala, Val, Leu, Ile, Thr, Phe, and Pro. The natural amino acids may be each independently selected from the group consisting of Gly, Ala, Val, Leu, Ile, Thr, Phe, and Pro.

In one non-limiting embodiment, the number of non-natural amino acids among the amino acids composing the cyclic portion of the cyclic peptide compound in the present disclosure is preferably 7 or more, more preferably 8 or more, and most preferably 9 or more. Examples of the preferable number of non-natural amino acids include 14 or less, 13 or less, 12 or less, 11 or less, and 10 or less.

In one non-limiting embodiment, the amino acids composing the cyclic portion of the cyclic peptide compound in the present disclosure may include, but are not particularly limited to, a non-natural amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu). The non-natural amino acids may be each independently an amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).

In one non-limiting embodiment, the value of Caco-2 $P_{app}$ (cm/sec) of the cyclic peptide compound in the present disclosure may be, for example, $1.0 \times 10^{-4}$ or less, $9.0 \times 10^{-5}$ or less, $8.0 \times 10^{-5}$ or less, $7.0 \times 10^{-5}$ or less, $6.0 \times 10^{-5}$ or less, $5.0 \times 10^{-5}$ or less, $4.0 \times 10^{-5}$ or less, $3.0 \times 10^{-5}$ or less, $2.0 \times 10^{-5}$ or less, $1.0 \times 10^{-5}$ or less, $9.0 \times 10^{-6}$ or less, $8.0 \times 10^{-6}$ or less, $7.0 \times 10^{-6}$ or less, $6.0 \times 10^{-6}$ or less, or $5.99 \times 10^{-6}$ or less. In one non-limiting embodiment, the value of Caco-2 $P_{app}$ (cm/sec) of the cyclic peptide compound in the present disclosure may be, for example, $1.0 \times 10^{-9}$ or more, $1.0 \times 10^{-8}$ or more, $1.0 \times 10^{-7}$ or more, $0.4 \times 10^{-6}$ or more, $1.0 \times 10^{-6}$ or more, or $1.0 \times 10^{-5}$ or more, preferably $0.4 \times 10^{-6}$ or more, and more preferably $1.0 \times 10^{-6}$ or more. In one non-limiting embodiment, the range of the value of Caco-2 $P_{app}$ (cm/sec) of the cyclic peptide compound in the present disclosure may be, for example, $1.0 \times 10^{-9}$ or more and $1.0 \times 10^{-4}$ or less, $1.0 \times 10^{-8}$ or more and $1.0 \times 10^{-4}$ or less, $1.0 \times 10^{-7}$ or more and $1.0 \times 10^{-4}$ or less, $0.4 \times 10^{-6}$ or more and $1.0 \times 10^{-4}$ or less, and $0.4 \times 10^{-6}$ or more and $1.0 \times 10^{-5}$ or less.

- <u>Membrane permeability of peptide compound</u>

The value of Caco-2 $P_{app}$ (cm/sec) is a value that serves as an indicator of the membrane permeability in the cell membrane, and can be calculated through membrane permeability evaluation under the following conditions.

(a) Caco-2 cells are cultured on 96-well Transwell for 3 weeks, then a compound to be evaluated, a DMEM medium, and FaSSIF (containing 1% DMSO) are added to the Apical side, a DMEM medium is added to the Basal side, and the cells are preincubated under a 5% $CO_2$ atmosphere at 37°C with shaking at 80 rpm for 24 hours;
(b) after the preincubation, the preincubation solutions on the Apical side and the Basal side are aspirated and washed, and a FaSSIF/HBSS buffer solution (containing 1% DMSO, pH 6.0) containing the compound is added to the Apical side, and a HBSS buffer solution (containing 4% BSA, pH 7.4) is added to the Basal side;
(c) each well is shaken under a 5% $CO_2$ atmosphere at 37°C and 80 rpm, and at 180 minutes after the start of shaking, a sample on the Basal side is collected, and the permeation amount of the compound is measured by liquid chromatography-mass spectrometry (LC/MS); and
(d) from the measured permeation amount, the permeability coefficient (Caco-2 $P_{app}$ (cm/sec)) is calculated.

The permeability coefficient (Caco-2 $P_{app}$ (cm/sec)) is calculated using a numerical expression 1.
[Expression 1]

$$P_{app} = \frac{1}{C_1(0) \times S} \times \frac{dQ}{dt} \qquad \text{(Expression 1)}$$

dQ/dt represents the permeation rate of drug (the amount of drug appearing on the Basal side per unit time), S represents the total surface area of cells, and $C_1(0)$ represents the concentration of drug added on the Apical side.

Note that, in the above measurement, a Pgp inhibitor, such as Zosquidar, can be added to each of the FaSSIF/HBSS buffer solution (1% DMSO) (pH 6.0) and the HBSS buffer solution (4% BSA) (pH 7.4). In addition, as the concentration of the compound to be evaluated on the donor side used in the calculation of the permeability coefficient in the above step (d), the concentration of the compound when initially added can be used, or the concentration of the compound measured by collecting the solution on the Apical side before the start of pre-incubation or the start of shaking in the above step (c) can be used. In particular, when the preincubation is performed, it is preferable to use the concentration of the compound on the Apical side collected before pre-incubation.

In one non-limiting embodiment, when measuring the membrane permeability of the peptide compounds in the

present disclosure, it is preferable to use a peptide compound that does not have a nucleic acid portion serving as a template for the peptide moiety as the test substance. In one non-limiting embodiment, as described later, panning is performed in the state of the peptide-nucleic acid complex, and then a peptide-nucleic acid complex that can specifically bind to the target molecule is selected, and a peptide compound can be synthesized based on the nucleotide sequence of the nucleic acid portion of the selected peptide-nucleic acid complex. Preferred examples include measuring the membrane permeability using such a synthesized peptide compound as the test substance. In one non-limiting embodiment, a derivative of the peptide compound encoded by the nucleotide sequence may be synthesized, and the membrane permeability may be measured using the derivative as a test substance, or a derivative may be synthesized from the test substance based on the results of a membrane permeability, and the membrane permeability of the derivative may be measured.

In one non-limiting embodiment, the cyclic peptide compound in the present disclosure may be selected from the compounds pd001 to pd168 listed in Table 15.

<u>- Peptide-Nucleic Acid Complex</u>

In one embodiment, the peptide compound in the present disclosure may be a complex of a peptide and a nucleic acid (peptide-nucleic acid complex). The peptide-nucleic acid complex may form a complex by binding the nucleic acid to the C-terminus of the peptide moiety. The peptide-nucleic acid complex in the present disclosure is classified into the linear portion to which the nucleic acid is linked, that is a nucleic acid linking portion, and the peptide moiety other than the nucleic acid linking portion. In the cyclic peptide-nucleic acid complex, the nucleic acid is linked to the linear portion and not to the cyclic portion. The method of bonding the peptide and the nucleic acid is not particularly limited, but they may be bonded via a linker. When the peptide compound and the nucleic acid are bonded via a linker, the linker is considered as a part of the nucleic acid linking portion. In one embodiment, the nucleic acid forming a complex with the peptide moiety may be DNA or RNA, preferably RNA, and more preferably mRNA. In one embodiment, the nucleic acid that forms a complex with the peptide may be a template used for the translation-based synthesis of the peptide moiety, where mRNA is preferred as the template. In one more specific embodiment, preferred examples include a peptide compound in which the C-terminus of the peptide moiety is linked to the 3' end of the mRNA serving as a template via a linker containing an antibiotic puromycin or the like, which is an analog of aminoacyl tRNA.

The peptide-nucleic acid complex can be produced, for example, by the method described in International Publication No. WO 2013/100132. Specifically, the peptide-nucleic acid complex can be produced by a method that includes the following steps.

A) acylating dinucleotide pdCpA or pCpA with a desired amino acid or amino acid analog to provide aminoacyl-pdCpA or aminoacyl-pCpA,
B) providing a tRNA lacking the 3' terminal CA,
C) linking the aminoacyl pdCpA or aminoacyl pCpA in step A with the CA-deficient tRNA in step B to provide an initiator tRNA acylated with a desired amino acid or amino acid analog,
D) providing a cell-free translation system which contains the initiator tRNA in step C) and does not contain at least one of methionine, a methionyl-tRNA synthetase (MetRS), an initiator tRNA for methionine translation, a formyl donor, and a methionyl-tRNA transferase,
E) providing a template DNA encoding a peptide sequence containing an ATG translation initiation codon downstream of a promoter, followed by a cysteine codon UGU or UGC, and downstream thereof, a codon corresponding to the anticodon of the tRNA in step C),
F) providing mRNA from the template DNA in step E),
G) attaching a linker to the 3' end of mRNA in step F), and
H) adding the mRNA attached to the linker in step G) to a cell-free translation system in step D), and translating to provide a pre-cyclic peptide-mRNA complex.

When the peptide is a cyclic peptide, a step of cyclizing the pre-cyclic peptide-mRNA complex in step H) may be further included. In step H), a desulfurization reaction can be performed as needed. Additionally, after step G), a step of synthesizing cDNA using a primer that anneals to the 3' region of mRNA can be included.

In steps A to C, RNA can be synthesized by preparing a template DNA that encodes a desired tRNA sequence and disposes a T7, T3 or SP6 promoter upstream and transcribing with RNA polymerases adapted for promoters, such as T7 RNA polymerase and T3, and SP6 RNA polymerase. A generated tRNA of interest can also be extracted by extracting and purifying tRNA from the cells and using probes of complementary sequences of tRNA sequence. At this time, the cells transformed with an expression vector of tRNA of interest can be used as a source. RNA of the sequence of interest can also be synthesized by chemical synthesis. For example, an aminoacyl tRNA can be obtained by binding tRNA in which CA is removed from the CCA sequence at the 3' end obtained in this way to an aminoacylated pdCpA prepared separately with

an RNA ligase (pdCpA method). Alternatively, it is also possible to prepare a full-length tRNA and perform aminoacylation by flexizyme, which is a ribozyme that charges tRNA with active esters of various non-natural amino acids.Acylated tRNA can also be obtained by using the method described later.

In steps E to H, DNA in which a desired sequence is placed downstream of a promoter such as a T7 promoter is first chemically synthesized, then used as a template to obtain a double-stranded DNA by a primer extension reaction. The obtained double-stranded DNA is transcribed as a template into mRNA using RNA polymerase such as T7 RNA polymerase. By attaching a linker containing puromycin or the like to the 3' end of the mRNA obtained through transcription and then translating the mRNA into a protein using a known cell-free translation system like the PURE system, the puromycin is incorporated into the protein, thereby the mRNA and its encoded protein are linked via the puromycin. In this way, a peptide-mRNA complex where the mRNA and the coded product (peptide) are associated can be constructed.

- Metabolic stability of peptide compound

In one non-limiting embodiment, it is preferable that the peptide compound of the present disclosure have good metabolic stability. For good metabolic stability, it is preferable that the number of amino acids contained in the peptide compound be 13 to 14. When the number of amino acids contained in the peptide compound is 13 to 14, the conditions for having the membrane permeability described above do not affect the metabolic stability of the peptide compound. In one embodiment, it is preferable that the peptide compound does not have a thioether bond that can be easily oxidized. In one embodiment, it is preferable that the peptide compound does not have a methylthio group since the methylthio group can be easily oxidized and potentially hinder metabolic stability.

The metabolic stability of the peptide compound of the present invention can be confirmed using known methods, such as using hepatocytes, intestinal cells, liver microsomes, intestinal microsomes, liver S9 mix, or the like. Specifically, for example, the stability of the peptide compound in liver microsomes can be confirmed by measuring it according to the description in the literature by LL von Moltke et al. (Midazolam hydroxylation by human liver microsomes in vitro: inhibition by fluoxetine, norfluoxetine, and by azole antifungal agents. J Clin Pharmacol, 1996, 36(9), 783-791). More specifically, the metabolic stability can be confirmed according to the method described in Example 3.

Regarding the metabolic stability, for example, if the intrinsic hepatic clearance ($CL_h$ int (μL/min/mg protein)) value when the stability in liver microsomes is measured according to the above-mentioned method is 150 or less, it can be considered that metabolic stability sufficient for use as an oral pharmaceutical is achieved, and the value is preferably 100 or less. In the cases that the peptide compound is a drug to be metabolized by CYP3A4, it is preferable that the value is 78 or less to avoid intestinal metabolism in humans (Non Patent Literature: M. Kato et al. The intestinal first-pass metabolism of substances ofCYP3A4 and P-glycoprotein-quantitative analysis based on information from the literature. Drug Metab. Pharmacokinet. 2003, 18(6), 365-372.), and it is more preferable that the value is 35 or less (assuming FaFg = 1 and protein binding rate = 0%) to show a bioavailability of about 30% or more in humans.

- Nucleic acid

In one non-limiting embodiment, the peptide compound in the present disclosure may be linked to a nucleic acid, preferably via a linker. Preferred examples of the nucleic acid in the present disclosure include a nucleic acid that encodes the peptide compound in the present disclosure. In one embodiment, the term "nucleic acid encoding a peptide compound" refers to a nucleic acid that is used or has been used as a template in the translation-based synthesis of the peptide compound.

- Library

The library in the present disclosure includes a library of the peptide compounds in the present disclosure and a library of peptide-nucleic acid complexes. The library in the present disclosure may further include linear peptide compounds and linear peptide-nucleic acid complexes. As for the library in the present disclosure, a library of cyclic peptide compounds or a library of cyclic peptide-nucleic acid complexes is preferred, particularly a library of cyclic peptide-mRNA complexes is preferred. According to the library in the present disclosure, among cyclic peptide compounds having a cyclic portion formed of 13 or 14 amino acid residues, compounds that excel in drug-likeness and have high affinity for target molecules can be efficiently found. As the library, a display library is preferred. Examples of the display library include a display-utilizing library. Among them, an mRNA display library, a DNA display library, and a ribosome display library are preferred, and an mRNA display library is more preferred. When the library in the present disclosure is a display library, nucleic acids linked to enriched peptide compounds by panning can be amplified by PCR or the like, and then transcribed and translated, allowing for easy synthesis of peptide compounds (Yanagawa, H. et al. FEBS Lett., 1997, 414, 405-408. and Szostak J.W. et al. Proc. Natl. Acd. Sci. USA, 1997, 94,12297-12302.).

The peptide compound in the present disclosure may be a peptide compound encoded by a nucleic acid. When

the peptide compound in the present disclosure is linked to a nucleic acid, the peptide compound may be a peptide compound encoded by the linked nucleic acid or a peptide compound obtained by further modifying the peptide compound. In other words, the term "(cyclic) peptide compound encoded by nucleic acid" includes not only peptide compounds that are directly synthesized by translating the nucleic acid as a template, but also peptides that are synthesized by further modification after translation. For example, when a linear peptide is synthesized by translating a nucleic acid, and then a cyclic peptide is synthesized through subsequent cyclization steps, the cyclic peptide may be referred to as a cyclic peptide compound encoded by the nucleic acid.

For a library of the peptide compound that may be linked to a nucleic acid and/or nucleic acids encoding them, the term "consist substantially of" as used herein means that the proportion of the theoretical number of variations of the recited peptide compounds and/or nucleic acids encoding them contained in a library to the theoretical total number of variations of the peptide compounds and/or nucleic acids encoding them contained in the library may be, but is not limited to, 90% or more, 93% or more, 95% or more, 97% or more, 98% or more, or 99% or more.

In one non-limiting embodiment, the proportion of the number of cyclic peptide compounds in which the number of amino acid residues composing the cyclic portion is 13 or 14 to the total number of cyclic peptide compounds contained in the library in the present disclosure may be, but is not limited to, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more.

In one non-limiting embodiment, the library in the present disclosure may be a library substantially consisting of cyclic peptide compounds in which the number of amino acid residues composing the cyclic portion of the cyclic peptide compound is 13 or 14.

In one non-limiting embodiment, the proportion of the number of cyclic peptide compounds in which the number of amino acid residues composing the cyclic peptide compound is 13 or 14 to the total number of cyclic peptide compounds contained in the library in the present disclosure may be, but is not limited to, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more.

In one non-limiting embodiment, the library in the present disclosure may be a library consisting substantially of cyclic peptide compounds in which the number of amino acid residues composing the cyclic peptide compound is 13 or 14.

In one non-limiting embodiment, the average of the number of amino acids (the number of units) of the linear portion other than a nucleic acid linking portion in each cyclic peptide compound contained in the library in the present disclosure is preferably 0 to 8, more preferably 0 to 5, and most preferably 0.

In one non-limiting embodiment, the number of amino acids composing a peptide moiety other than a nucleic acid linking portion in each cyclic peptide compound contained in the library in the present disclosure is, for example, but not limited to, 13 or more, 14 or less, 13 to 14, 13, or 14. Given the requirement to achieve both excellent membrane permeability and metabolic stability, it is preferable that the number of amino acids composing the cyclic portion is 13, 14, or 13 to 14.

In one non-limiting embodiment, the average of the number of amino acids composing a peptide moiety other than a nucleic acid linking portion in each cyclic peptide compound contained in the library in the present disclosure is, for example, but not limited to, 13 or more, 14 or less, 13, or 14. Given the requirement to achieve both excellent membrane permeability and metabolic stability, it is preferable that the average of the number of amino acids composing the cyclic portion is 13 to 14.

In one non-limiting embodiment, the average of the number of N-substituted amino acids among the number of amino acids composing the cyclic portion, in each cyclic peptide compound contained in the library in the present disclosure is, for example, 7 or more, 8 or more, 9 or more, 10 or more, 14 or less, 13 or less, 12 or less, 11 or less, 7 to 14, 7 to 13, 7 to 12, 7 to 11, 7 to 10, 7 to 9, 8 to 14, 8 to 13, 8 to 12, 8 to 11, 8 to 10, 8 to 9, 9 to 14, 9 to 13, 9 to 12, 9 to 11, 9 to 10, 10 to 14, 10 to 13, 10 to 12, or 10 to 11, preferably 7 or more, 8 or more, 7 to 13, or 8 to 13, more preferably 7 to 10 or 8 to 10.

In one non-limiting embodiment, the proportion of the number of cyclic peptide compounds in which the number of N-substituted amino acids composing the cyclic portion is 7 to 10, among the cyclic peptide compounds contained in the library in the present disclosure is, for example, 10% or more, 20% or more, 30% or more, 40% or more, 50% or more, 60% or more, 70% or more, 80% or more, or 90% or more, preferably 50% or more or 60% or more, more preferably 70% or more, and most preferably 80% or more.

In one non-limiting embodiment, the proportion of the number of cyclic peptide compounds in which the number of N-substituted amino acids composing the cyclic portion is 8 to 13, among the cyclic peptide compounds contained in the library in the present disclosure is, for example, 10% or more, 20% or more, 30% or more, 40% or more, 50% or more, 60% or more, 70% or more, 80% or more, or 90% or more, preferably 50% or more or 60% or more, more preferably 70% or more, and most preferably 80% or more.

In one non-limiting embodiment, the proportion of the number of cyclic peptide compounds in which the number of N-substituted amino acids composing the cyclic portion is 8 to 10, among the cyclic peptide compounds contained in the library in the present disclosure is, for example, 10% or more, 20% or more, 30% or more, 40% or more, 50% or more, 60%

or more, 70% or more, 80% or more, or 90% or more, preferably 50% or more or 60% or more, more preferably 70% or more, and most preferably 80% or more.

In cyclic peptide compounds contained in the library in the present disclosure, the average proportion of the number of N-substituted amino acids among the number of amino acids composing the cyclic portion is, for example, 50% or more, 55% or more, 60% or more, 65% or more, 70% or more, 75% or more, or 80% or more, preferably 50% or more, and more preferably 60% or more. The N-substituted amino acid mentioned here is preferably an N-alkyl amino acid, more preferably an N-$C_1$-$C_6$ alkyl amino acid, further preferably an N-methyl amino acid or N-ethyl amino acid, and most preferably an N-methyl amino acid. That is, in one non-limiting embodiment, the number of the N-substituted amino acids is illustrated as the average of the number of N-alkyl amino acids, the number of N-$C_1$-$C_6$ alkyl amino acids, the number of N-ethyl amino acids, or the number of N-methyl amino acids.

In one non-limiting embodiment, the average of ClogP/totalAB of the cyclic peptide compound contained in the library in the present disclosure is preferably 1.0 or more, more preferably 1.1 or more, more preferably 1.2 or more, and preferably 1.8 or less, 1.7 or less, 1.6 or less, 1.5, or 1.4 or less. The average of ClogP/total AB of the cyclic peptide compound contained in the library in the present disclosure may be 1.0 to 1.8, 1.0 to 1.7, 1.0 to 1.6, 1.0 to 1.5, 1.0 to 1.4, 1.0 to 1.3, 1.0 to 1.2, 1.1 to 1.6, 1.1 to 1.5, 1.1 to 1.4, 1.1 to 1.3, or 1.1 to 1.2. The "ClogP/total AB of the cyclic peptide compound" is the value calculated as described above. If the ClogP/total AB of each cyclic peptide compound contained in the library in the present disclosure is calculated, and the average value is within the aforementioned range, the probability that the hit compounds in the screening are compounds with excellent membrane permeability increases.

In one non-limiting embodiment, the average of ClogP of the cyclic peptide compound contained in the library in the present disclosure is preferably 10 or more, 11 or more, 12 or more, or 13 or more, and preferably 25 or less, 24 or less, 23 or less, 22 or less, 21 or less, or 20 or less. The average of ClogP of the cyclic peptide compound contained in the library in the present disclosure may be 10 to 25, 11 to 24, 12 to 23, 13 to 22, 13 to 21, 13 to 20, 14 to 20, or 15 to 20. The "ClogP of the cyclic peptide compound" is the value calculated as described above. If the ClogP of each cyclic peptide compound contained in the library in the present disclosure is calculated, and the average value is within the aforementioned range, the probability that the hit compounds in the screening are compounds with excellent membrane permeability increases.

In one non-limiting embodiment, the cyclic peptide compound contained in the library in the present disclosure may have a hydrogen bond donor (proton donor). The average of the number of the hydrogen bond donor is, for example, 8 or less, 7 or less, 6 or less, 5 or less, 4 or less, 3 or less, 2 or less, or 1 or less, preferably 5 or less, more preferably 4 or less, and most preferably 3 or less. The lower limit of the average of the number of the hydrogen bond donors is not particularly limited, but examples include 0 or more, 1 or more, or 2 or more. Examples of the average of the number of the hydrogen bond donors include 0 to 8, 0 to 7, 0 to 6, 0 to 5, 0 to 4, 0 to 3, 0 to 2, 0 to 1, 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 9, 3 to 8, 3 to 7, 3 to 6, 3 to 5, or 3 to 4. If the number of hydrogen bond donors of each cyclic peptide compound contained in the library in the present disclosure is calculated, and the average value is within the aforementioned range, the probability that the hit compounds in the screening are compounds with excellent membrane permeability increases.

In one embodiment, the cyclic peptide compound contained in the library in the present disclosure may have a hydrogen bond donor (proton donor) in the cyclic portion. The hydrogen bond donor is a group that provides the necessary hydrogen atom to form a hydrogen bond, and forms a hydrogen bond with a hydrogen bond acceptor (proton acceptor) in the same compound or in nearby compounds (including water molecules). The hydrogen bond donor is a group that contains a hydrogen atom covalently bonded to an atom having relatively high electronegativity, and examples include a hydroxy group, a carboxy group, an amino group, an unsubstituted amide group, and a monosubstituted amide group. The hydrogen bond acceptor is, for example, lone pairs on carbonyl oxygen in carboxy groups, esters, and amides, or amine nitrogen. When an intramolecular hydrogen bond is formed, the conformation of the peptide compound is fixed, thus the affinity with the target molecule can be enhanced. When an intermolecular hydrogen bond with the target molecule is formed, the affinity with the target molecule can be enhanced. The average of the number of the hydrogen bond donor in the cyclic portion is, for example, 8 or less, 7 or less, 6 or less, 5 or less, 4 or less, 3 or less, 2 or less, or 1 or less, preferably 5 or less, more preferably 4 or less, and most preferably 3 or less. The lower limit of the average of the number of the hydrogen bond donors is not particularly limited, but examples include 0 or more, 1 or more, or 2 or more. Examples of the average of the number of the hydrogen bond donors include 0 to 8, 0 to 7, 0 to 6, 0 to 5, 0 to 4, 0 to 3, 0 to 2, 0 to 1, 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 9, 3 to 8, 3 to 7, 3 to 6, 3 to 5, or 3 to 4.

In one non-limiting embodiment, the cyclic peptide compound contained in the library of the present disclosure may include a plurality of amino acids having the same side chain as the amino acids that compose the cyclic portion. Here, the term "include amino acids having the same side chain" means that a plurality of amino acids having the same side chain are included in the amino acids that compose the entire cyclic peptide compound or the cyclic portion of the cyclic peptide compound. The "amino acids having the same side chain" are determined based on identity or non-identity of the side chains, and substituents on the nitrogen atom of the amino group in the backbone are not considered. For example, alanine and N-methylalanine are considered "amino acids having the same side chain". Specifically, when the cyclic portion of the cyclic peptide compound contains two Leu amino acids, the cyclic peptide compound includes a plurality of

amino acids having the same side chain as the amino acids composing the cyclic portion. Among amino acids composing the cyclic portion, the average of the number of amino acids having the same side chain is, for example, 5 or less, 4 or less, 3 or less, 2 or less, or 1 or less, preferably 4 or less, and more preferably 3 or less.

In one non-limiting embodiment, the side chain of at least one amino acid composing the cyclic portion of the cyclic peptide compound contained in the library in the present disclosure is composed of at least one selected from the group consisting of an alkyl group, a cycloalkyl group, an aralkyl group, and an amide group. Herein, an arbitrary atom of the alkyl group and the nitrogen atom of the amino group of the amino acid may be taken together to form a ring.

In one non-limiting embodiment, when the side chain of at least one amino acid composing the cyclic portion of the cyclic peptide compound contained in the library in the present disclosure is an alkyl group, the alkyl group may be a $C_1$-$C_6$ alkyl group optionally substituted with at least one group selected from the group consisting of a halogen atom, a hydroxy group, a $C_3$-$C_8$ cycloalkyl group, and a $C_1$-$C_6$ alkoxy group.

In one non-limiting embodiment, when the side chain of at least one amino acid composing the cyclic portion of the cyclic peptide compound contained in the library in the present disclosure is a cycloalkyl group, the cycloalkyl group may be a $C_3$-$C_{10}$ cycloalkyl group optionally substituted with at least one group selected from the group consisting of a halogen atom, a hydroxy group, a $C_1$-$C_6$ alkyl group, a $C_1$-$C_6$ haloalkyl group, a $C_1$-$C_6$ alkoxy group, and a $C_1$-$C_6$ haloalkoxy group.

In one non-limiting embodiment, when the side chain of at least one amino acid composing the cyclic portion of the cyclic peptide compound contained in the library in the present disclosure is an aralkyl group, the aralkyl group may be a $C_7$-$C_{14}$ aralkyl group optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

In one non-limiting embodiment, when the side chain of at least one amino acid composing the cyclic portion of the cyclic peptide compound contained in the library in the present disclosure is an aralkyl group, the aralkyl group may be an optionally substituted $C_5$-$C_{10}$ aryl $C_1$-$C_6$ alkyl groupin which the $C_5$-$C_{10}$ aryl group is optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

In one non-limiting embodiment, when the side chain of at least one amino acid composing the cyclic portion of the cyclic peptide compound contained in the library in the present disclosure is an amide group, the amide group may be represented by formula: - $CONR^1R^2$, wherein $R^1$ and $R^2$ are each independently a hydrogen atom or a $C_1$-$C_6$ alkyl group, or $R^1$ and $R^2$, together with a nitrogen atom to which they are attached, form a 4- to 8-membered saturated heterocyclic ring, and the 4- to 8-membered saturated heterocyclic ring is optionally substituted with at least one group independently selected from the group consisting of a halogen atom, an oxo group, a $C_1$-$C_6$ alkyl group, and a 4- to 7-membered heterocyclyl group.

In one non-limiting embodiment, at least one amino acid composing the cyclic portion in each cyclic peptide compound contained in the library in the present disclosure may be an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu. At least one amino acid composing the cyclic portion may be each independently an amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.

In one non-limiting embodiment, the amino acids composing the cyclic portion of the cyclic peptide compound contained in the library in the present disclosure are not particularly limited, and may be selected from the group consisting of an $\alpha$-amino acid, an amino acid having a $\beta$-amino acid skeleton, and an amino acid having a $\gamma$-amino acid skeleton. The average of the number of $\alpha$-amino acids composing the cyclic portion of the cyclic peptide compound contained in the library is, for example, but not limited to, 10 to 14, 10 to 13, 10 to 12, 10 to 11, 10, 11, 12, 13, or 14. The average of the number of amino acids having a $\beta$-amino acid skeleton composing the cyclic portion of the cyclic peptide compound contained in the library is not limited, but for example, 0 to 4, 0 to 3, 0 to 2, 0 to 1, 1 to 4, 1 to 3, 1 to 2, 2 to 4, 2 to 3, 3 to 4, 0, 1, 2, 3, or 4. The average of the number of $\gamma$-amino acids composing the cyclic portion of the cyclic peptide compound contained in the library is not limited, but for example, 0 to 4, 0 to 3, 0 to 2, 0 to 1, 1 to 4, 1 to 3, 1 to 2, 2 to 4, 2 to 3, 3 to 4, 0, 1, 2, 3, or 4. To achieve high membrane permeability, it is preferable that the average of the number of $\alpha$-amino acids composing the cyclic portion is 10 to 14, the number of amino acids having a $\beta$-amino acid skeleton is 0 to 2, and the number of $\gamma$-amino acids is 0 to 2.

In one non-limiting embodiment, the amino acids composing the cyclic portion of the cyclic peptide compound contained in the library in the present disclosure may include, but are not particularly limited to, an $\alpha$-amino acid selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu). The $\alpha$-amino acids may be each independently selected from the group consisting of Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, and Ser(tBu).

In one non-limiting embodiment, the amino acids composing the cyclic portion of the cyclic peptide compound

contained in the library in the present disclosure may include, but are not particularly limited to, an amino acid having a β-amino acid skeleton selected from the group consisting of Asp-pip, bAla, bMeAla, D-3-Abu, D-3-MeAbu, and MeAsp-pip. The amino acids having a β-amino acid skeleton may be each independently selected from the group consisting of Asp-pip, bAla, bMeAla, D-3-Abu, D-3-MeAbu, and MeAsp-pip.

In one non-limiting embodiment, the amino acids composing the cyclic portion of the cyclic peptide compound contained in the library in the present disclosure may include or may be, but are not particularly limited to, gMeAbu.

In one non-limiting embodiment, the average of the number of natural amino acids among the number of amino acids composing the cyclic portion in the cyclic peptide compounds contained in the library in the present disclosure is, for example, 1 or more, 2 or more, 3 or more, 7 or less, 6 or less, 5 or less, 4 or less, 1 to 7, 1 to 6, 1 to 5, 1 to 4, 2 to 7, 2 to 6, 2 to 5, 2 to 4, 3 to 7, 3 to 6, 3 to 5, or 3 to 4, preferably 1 or more, 7 or less, or 1 to 7, more preferably 1 or more, 6 or less, 5 or less, 1 to 6, or 1 to 5, and most preferably 1 or more, 4 or less, or 1 to 4. In the cyclic peptide compounds contained in the library in the present disclosure, the average of the proportion of the number of natural amino acids among the number of amino acids composing the cyclic portion may be 10% or more or 20% or more, and may be 50% or less, 40% or less, or 30% or less. The average of the proportion of the number of natural amino acids among the number of amino acids composing the cyclic portion may be 10 to 50%, 15 to 50%, 20 to 50%, 25 to 50%, 30 to 50%, 40 to 50%, 10 to 40%, 15 to 40%, 20 to 40%, 25 to 40%, 30 to 40%, 10 to 30%, 15 to 30%, or 20 to 30%.

In one non-limiting embodiment, the amino acids composing the cyclic portion of the cyclic peptide compound contained in the library in the present disclosure may include a natural amino acid selected from the group consisting of Gly, Ala, Val, Leu, Ile, Thr, Phe, and Pro. The natural amino acid may be each independently selected from the group consisting of Gly, Ala, Val, Leu, Ile, Thr, Phe, and Pro.

In one non-limiting embodiment, the average of the number of non-natural amino acids composing the cyclic portion among the cyclic peptide compounds contained in the library in the present disclosure is preferably 7 or more, more preferably 8 or more, 9 or more, or 10 or more. In the cyclic peptide compounds contained in the library in the present disclosure, the average of the proportion of the number of non-natural amino acids among the number of amino acids composing the cyclic portion may be 50% or more, 55% or more, 60% or more, 65% or more, 70% or more, 75% or more, or 80% or more, and may be 100% or less, 95% or less, 90% or less, 85% or less, 80% or less, 75% or less, or 70% or less. The average of the proportion of the number of non-natural amino acids among the number of amino acids composing the cyclic portion may be 50 to 100%, 50 to 95%, 50 to 90%, 50 to 85%, 50 to 80%, 50 to 75%, 50 to 70%, 55 to 100%, 55 to 95%, 55 to 90%, 55 to 85%, 55 to 80%, 55 to 75%, 55 to 70%, 60 to 100%, 60 to 95%, 60 to 90%, 60 to 85%, 60 to 80%, 60 to 75%, 60 to 70%, 70 to 100%, 70 to 95%, 70 to 90%, 70 to 85%, or 70 to 80%.

In one non-limiting embodiment, the cyclic peptide compounds contained in the library, or the amino acids composing the cyclic portion of the cyclic peptide compound in the present disclosure may include, but are not particularly limited to, a non-natural amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu). The non-natural amino acid may be each independently selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).

In one non-limiting embodiment, each cyclic peptide compound contained in the library in the present disclosure may be a cyclic peptide compound that does not have a substituted or unsubstituted hydroxyphenyl group on the side chain of an amino acid contained in the peptide moiety other than a nucleic acid linking portion. In one embodiment, the library in the present disclosure may be a library that substantially does not contain a cyclic peptide compound having a substituted or unsubstituted hydroxyphenyl group on the side chain in the peptide moiety other than a nucleic acid linking portion. Since the presence of a substituted or unsubstituted hydroxyphenyl group on the side chain in the peptide moiety other than a nucleic acid linking portion of a cyclic peptide compound can hinder membrane permeability, it is preferable that the cyclic peptide compound does not have a substituted or unsubstituted hydroxyphenyl group.

In one non-limiting embodiment, each cyclic peptide compound contained in the library in the present disclosure may be a cyclic peptide compound that does not have an indole skeleton on the side chain in the peptide moiety other than a nucleic acid linking portion. In one embodiment, the library in the present disclosure may be a library that substantially does not contain a cyclic peptide compound having an indole skeleton on the side chain in the peptide moiety other than a nucleic acid linking portion. Since the presence of an indole skeleton on the side chain in the peptide moiety other than a nucleic acid linking portion of a cyclic peptide compound can hinder membrane permeability, it is preferable that the cyclic peptide compound does not have an indole skeleton.

In one non-limiting embodiment, the library in the present disclosure may be a library consisting substantially of cyclic peptide compounds that do not have a methylthio group on the side chain in the peptide moiety other than a nucleic acid linking portion. In one embodiment, the library in the present disclosure may be a library that substantially does not contain a cyclic peptide compound having a substituted or unsubstituted methylthio group on the side chain in the peptide moiety other than a nucleic acid linking portion. In one embodiment, it is preferable that the cyclic peptide compound does not have a methylthio group since the methylthio group can be easily oxidized and potentially hinder metabolic stability.

In one non-limiting embodiment, the library in the present disclosure may be a library consisting substantially of cyclic peptide compounds that do not have a thiol group on the side chain in the peptide moiety other than a nucleic acid linking portion. In one embodiment, the library in the present disclosure may be a library that substantially does not contain a cyclic peptide compound having a substituted or unsubstituted thiol group on the side chain in the peptide moiety other than a nucleic acid linking portion. In one embodiment, it is preferable that the cyclic peptide compound does not have a thiol group since the thiol group can be easily oxidized and potentially hinder metabolic stability.

In one non-limiting embodiment, the average of the molecular weight of the cyclic peptide compounds contained in the library in the present disclosure may be 500 to 2,000. In one embodiment, the average of the molecular weights of cyclic peptide compounds contained in the library in the present disclosure may be 500 to 2,000. As used herein, when the cyclic peptide compound is not linked to a nucleic acid, the molecular weight of the entire cyclic peptide compound is calculated. When the cyclic peptide compound is linked to a nucleic acid, the molecular weight of the peptide moiety other than the nucleic acid linking portion is calculated as the "molecular weight of the cyclic peptide compound". The "molecular weight of the peptide moiety other than the nucleic acid linking portion" is a value of calculated molecular weight for a hypothetical peptide in which the nucleic acid linking portion of the cyclic peptide compound is replaced with piperidine (pip). That is, the hypothetical peptide is a cyclic peptide in which, in the amide bond between the carboxy group of the backbone or side chain of the amino acids composing the cyclic portion (for example, amino acid residues having a carboxy group on the side chain such as Asp or Glu) and the amino group of the nucleic acid linking portion, piperidine (pip) is bound in place of an amino group of the nucleic acid linking portion. The cyclic peptide compound linked to a nucleic acid (cyclic peptide-nucleic acid complex) can be used as a component composing a display library used for screening, but a drug candidate compound that is expected to be employed in practice is solely of parts where the nucleic acid is not linked. Thus, when considering drug-likeness (for example, high membrane permeability), the properties of the peptide moiety other than a nucleic acid linking portion could become important.

In the library in the present disclosure, when determining the "theoretical (total) number of variations" of cyclic peptide compounds, those that cannot actually be produced as cyclic peptide compounds are not included in the theoretical (total) number. For example, in cases such as the following (1) and (2), the relevant amino acids are not synthesized via translation, thus the peptide compounds containing such amino acids are not included in the theoretical (total) number: (1) when, even if amino acids are added to the translation solution as materials for the peptide compound, the nucleic acids encoding the same amino acids are not contained as a template; (2) when using a translation mixture that contains a nucleotide sequence of nucleic acids serving as a template for the peptide compound, but does not contain corresponding amino acids. In addition, if there are by-products or unreacted substances in the process of producing the cyclic peptide compound, these are not included in the theoretical (total) number.

For example, using a total of 20 types of natural amino acids and a codon table that corresponds a set of codon units to one type of amino acid, when synthesizing an 11-residue peptide via translation from a nucleotide sequence where the codon units are randomly arranged, if the initiator amino acid is fixed to methionine, the theoretical number of variations is $20^{10}$.

As used herein, whether the average value of the cyclic peptide compounds contained in the library falls within a specific range can be determined by summing the values of each cyclic peptide compound actually contained in the library, and then dividing the total by the number of all cyclic peptide compounds contained in the library (measured number-average value). However, if the number of cyclic peptide compounds in the library is large, making it difficult to calculate the measured number-average value using the methods described above, the measured number-average value is substituted with a theoretical number-average value. The theoretical number-average value is a value calculated by assuming all types of cyclic peptide compounds that could theoretically be contained in the library, summing their respective values, and then dividing this total value by the theoretical number of types of cyclic peptide compounds that could theoretically be contained in the library.

The library in the present disclosure contains a plurality of cyclic peptide compounds or nucleic acids encoding these. The number of variations (types) of cyclic peptide compounds contained in a library herein is referred to as "diversity". The diversity of cyclic peptide compounds or nucleic acids encoding these contained in the library in the present disclosure is not particularly limited, and examples thereof include $10^2$ or more, $10^3$ or more, $10^4$ or more, $10^5$ or more, $10^6$ or more, $10^7$ or more, $10^8$ or more, $10^9$ or more, $10^{10}$ or more, $10^{11}$ or more, and $10^{12}$ or more. These diversities are not limited to measured values, and may be theoretical values.

The cyclic peptide compounds contained in the library in the present disclosure may include at least one selected from the cyclic peptide compounds pd001 to pd168 listed in Table 15, or may include all of them.

Although the features of the cyclic peptide compounds contained in the library in the present disclosure have been described so far, these descriptions can also be applied to the features of the cyclic peptide compounds in which the number of amino acid residues composing the cyclic portion is 13 or 14 contained in the library in the present disclosure. For example, the description in the paragraph regarding the proportion of the number of cyclic peptide compounds in which the number of N-substituted amino acids composing the cyclic portion is 7 to 10 among the cyclic peptide compounds contained in the library in the present disclosure can also be applied as an explanation for the proportion of the number of

cyclic peptide compounds in which the number of N-substituted amino acids composing the cyclic portion is 7 to 10 among the cyclic peptide compounds in which the number of amino acid residues composing the cyclic portion is 13 or 14 contained in the library in the present disclosure.

- Membrane Permeability

In one non-limiting embodiment, the library in the present disclosure contains cyclic peptide compounds with relatively high membrane permeability, and it is even possible to easily obtain peptide compounds with even higher membrane permeability by further derivatizing the cyclic peptide compounds. In addition, when screening is performed using the library in the present disclosure, the cyclic peptide compounds that have been hit as compounds capable of specifically binding to the target molecules may have relatively high membrane permeability. The term "high membrane permeability" as used herein means that the membrane permeability coefficient ($P_{app}$) as measured by the method for measuring membrane permeability in the present disclosure is $4.0 \times 10^{-7}$ cm/s or more, preferably $8.0 \times 10^{-7}$ cm/s or more, or $9.0 \times 10^{-7}$ cm/s or more, more preferably $1.0 \times 10^{-6}$ cm/s or more, more preferably $3.0 \times 10^{-6}$ cm/s or more, and most preferably $5.0 \times 10^{-6}$ cm/s or more. In one non-limiting embodiment, the library in the present disclosure may be a library for identifying orally administrable compounds or their precursors. The "orally administrable" herein means that the membrane permeability coefficient ($P_{app}$) is $0.4 \times 10^{-6}$ cm/s or more, preferably $1.0 \times 10^{-6}$ cm/s or more, and more preferably $5.0 \times 10^{-6}$ cm/s or more. The term "precursor" as used herein refers to a compound prior to chemical modifications for optimization or the like, and examples include a hit compound from panning, prior to any optimization.

- Hit rate of peptide compounds capable of specifically binding to target molecules

In one non-limiting embodiment, the library in the present disclosure can be used to identify compounds capable of specifically binding to target molecules. In one embodiment, the library in the present disclosure, compared to conventional libraries, has a higher number of amino acids composing the peptide compounds. This increases the diversity of the amino acids and results in a higher hit rate of peptide compounds capable of specifically binding to target molecules. Namely, by using the library in the present disclosure, not only the peptide compounds with high binding affinity to target molecules can be obtained, but also it is possible to obtain a large number of peptide compounds capable of specifically binding to the target molecules. Thus, it becomes possible to select the desired compound from among the large number of selected peptide compounds, considering other factors such as metabolic stability and membrane permeability. The term "hit rate" as used herein refers to the proportion of the number of variations of hit compounds obtained by panning among the number of variations of compounds contained in the library. The number of variations of the compounds contained in the library may be a theoretical number.

In one non-limiting embodiment, peptide compounds that are not present in the library as cyclic peptide compounds for obtaining hit compounds (for example, peptide compounds that clearly have no binding ability to the target molecule before panning) are not considered as peptide compounds composing the library in the present disclosure.

By using cyclic peptide compounds enriched through panning, it is possible to identify groups of amino acids that may contribute to binding with the target molecule by analyzing the frequency of occurrence of amino acids.

- Display library

The display library is a library in which a peptide, which is a phenotype, and RNA or DNA encoding the peptide, which is a genotype, are associated. This library can be used to identify a peptide compound capable of specifically binding to a target molecule. For example, peptides that bind to a desired target can be enriched by contacting a library with the desired fixed target and washing away molecules that do not bind to the target (panning method). By analyzing the genetic information associated with peptides selected through such a process, it is possible to clarify the sequence of peptides that bind to the target. For example, methods of utilizing the antibiotic puromycin, an analog of aminoacyl tRNA, which is non-specifically linked to a protein during mRNA translation extension by the ribosome are reported as mRNA display (Proc Natl Acad Sci USA. 1997;94:12297-302. RNA-peptide fusions for the in vitro selection of peptides and proteins. Roberts RW, Szostak JW.) or in vitro virus(FEBS Lett. 1997;414:405-8. In vitro virus: bonding of mRNA bearing puromycin at the 3'-terminal end to the C-terminal end of its encoded protein on the ribosome in vitro. Nemoto N, Miyamoto-Sato E, Husimi Y, Yanagawa H.).

When a linker such as puromycin is linked to the 3' end of the library of mRNA obtained by transcription from a DNA library including a promoter such as a T7 promoter, and the mRNA is translated into a protein in a cell-free translation system, the puromycin is mistakenly incorporated into a protein by the ribosome as an amino acid, and the mRNA and the protein encoded thereby are linked to yield a library in which the mRNA and its product are associated. In this process, high efficiency is achieved because it does not include transformation such as with E. coli, and a large display library can be

constructed. It is possible to identify the sequence of the bound protein by synthesizing cDNA from mRNA which is tagged to molecules enriched and selected in panning and containing genetic information, subjecting the cDNA to PCR amplification, and analyzing the nucleotide sequence.

Examples of the known display library utilizing cell-free translation systems include, in addition to mRNA display, a cDNA display that is a library of cDNA encoding a peptide to which a complex of a peptide and puromycin is attached (Nucleic Acids Res. 2009;37(16):e108. cDNA display: a novel screening method for functional disulfide-rich peptides by solid-phase synthesis and stabilization of mRNA-protein fusions. Yamaguchi J, Naimuddin M, Biyani M, Sasaki T, Machida M, Kubo T, Funatsu T, Husimi Y, Nemoto N.), a ribosome display utilizing a relatively stable complex of ribosome and a translation product during mRNA translation (Proc Natl Acad Sci U S A. 1994;91:9022-6. An in vitro polysome display system for identifying ligands from very large peptide libraries. Mattheakis LC, Bhatt RR, Dower WJ.), a covalent display in which bacteriophage endonuclease P2A forms a covalent bond with DNA (Nucleic AcidsRes. 2005;33:e10. Covalent antibody display--an in vitro antibody-DNA library selection system. Reiersen H, Lobersli I, Loset GA, Hvattum E, Simonsen B, Stacy JE, McGregor D, Fitzgerald K, Welschof M, Brekke OH, Marvik OJ.) and a CIS display utilizing the replication initiation protein RepA of the plasmid of the microorganism to bind to the replication initiation point ori (Proc Natl Acad Sci U S A. 2004;101:2806-10. CIS display: In vitro selection of peptides from libraries of protein-DNA complexes. Odegrip R, Coomber D, Eldridge B, HedererR, Kuhlman PA, Ullman C, FitzGerald K, McGregor D.). Also known is in vitro compartmentalization in which, for each molecule of DNA that constitutes a DNA library, a transcriptional translation system is encapsulated in a water-in-oil emulsion or liposome, and a translation reaction is performed (Nat Biotechnol. 1998;16:652-6. Man-made cell-like compartments for molecular evolution. Tawfik DS, Griffiths AD.). As appropriate, the above methods can be used using a known method.

- Nucleic Acid Library

The "nucleic acid" in the present invention can include a deoxyribonucleic acid (DNA), a ribonucleic acid (RNA), a nucleotide derivative having an artificial base, and a peptide nucleic acid (PNA). The nucleic acid in the present invention can be any of these nucleic acids or a mixture of them as long as the genetic information of interest is retained. That is, DNA-RNA hybrid nucleotides or chimeric nucleic acids in which different nucleic acids such as DNA and RNA are linked in a single strand are also included in the nucleic acids in the present invention.

The peptide compounds contained in the library can be synthesized through translation from the template nucleic acid. One non-limiting embodiment of the present disclosure is a library of nucleic acids serving as a template. Examples of the nucleic acid library include an mRNA library and a DNA library. A nucleic acid library can be obtained by adding bases to the positions where amino acid residues are not fixed on a peptide sequence and synthesizing them. For example, a nucleic acid library can be synthesized as a mixture of A, T, G, C in the case of DNA library, and A, U, G, C in the case of RNA library, of 3 times the number of 4 bases (N) or a mixture of 2 bases, such as N for the first and second letters of the codon and W, M, K, and S for the third letter. When the types of amino acids introduced are reduced to be 16 or less, there is a method of setting the base at the third letter to be one type of base. In addition, a codon unit corresponding to three letters of the codon is prepared, and the frequency of occurrence of amino acid residues may be freely adjusted by mixing it in any proportion and using it for synthesis. Each nucleic acid in the nucleic acid library may be linked to puromycin at its 3' end. By translating the library of nucleic acids linked to puromycin at the 3' end, the peptide compounds encoded by the nucleic acids are synthesized and the resulting peptide compound is linked to the corresponding nucleic acid, enabling the synthesis of a display library.

By translating these nucleic acid libraries using a cell-free translation system, it is possible to synthesize a library of corresponding cyclic peptides. When a cell-free translation system is used, it is preferable to include a sequence encoding a spacer downstream of the nucleic acid of interest. Examples of the spacer sequence include, but are not limited to, a sequence including glycine and serine. It is also preferable that a linker formed by polymers (e.g., five polymers) of RNA, DNA, hexaethylene glycol (spc18) or the like is included between a compound incorporated into a peptide and a nucleic acid library during translation by the ribosome such as puromycin or a derivative thereof.

- Screening method

In one non-limiting embodiment, peptide compounds capable of specifically binding to the target molecule can be selected through screening using the library in the present disclosure. The screening method in the present disclosure is not particularly limited, and for example, an mRNA display method can be used.

In one non-limiting embodiment, the screening method in the present disclosure involves contacting the library of cyclic peptide compounds in the present disclosure with a target molecule, and washing away peptide compounds that do not bind to the target molecule, thereby enriching peptide compounds that bind to the target molecule (panning). In one embodiment, it is possible to identify the amino acid sequence of the bound peptide by synthesizing cDNA from mRNA, which is a tag containing the nucleotide sequence information of a peptide compound selected in this way, subjecting it to

PCR amplification, and analyzing the nucleotide sequence. In one embodiment, an mRNA library may be obtained by transcribing the cDNA amplified above. Using this mRNA library as a template, a library of peptide compounds may be produced again. This library is enriched with peptide compounds that can bind to a target molecule, allowing for further enrichment of peptide compounds capable of specifically binding to the target molecule by performing panning again using this library. By repeating this process multiple times, the desired peptide compound can be further enriched. In one embodiment, the amino acid sequence can be identified based on the nucleotide sequence information contained in the peptide compound enriched in this manner, and a peptide compound capable of specifically binding to the target molecule can be produced. In one embodiment, the screening method according to the present disclosure can be performed in vitro.

In one non-limiting embodiment, the peptide compounds obtained by the screening method according to the present disclosure may be optimized by chemical modification or the like by known methods. The term "optimization" as used herein means chemically modifying the structure of each amino acid in a compound obtained through screening and the like to create a more drug-like peptide compound, to create a peptide compound with stronger activity against a pharmacological target, and/or to create a peptide compound with reduced toxicity.

In one non-limiting embodiment, the screening method in the present disclosure includes the following steps:

(a) bringing the peptide compound contained in the library in the present disclosure into contact with a target molecule; and

(b) selecting a peptide compound that is capable of binding to the target molecule.

In one embodiment, the screening method in the present disclosure may enrich peptide compounds that can specifically bind to the target molecule by repeating the steps (a) and (b) described above two or more times (cycles). In this case, in the step (a) from the second time onward, it is possible to use the library of peptide compounds selected in the previous cycle. This allows for an increased enrichment rate of the target peptide compound with each additional cycle. The number of repetitions may be, for example, 2 cycles or more, 3 cycles or more, 4 cycles or more, 5 cycles or more, 6 cycles or more, 7 cycles or more, 8 cycles or more, 9 cycles or more, or 10 cycles or more. By repeating the cycle, peptide compounds that bind to the target can be enriched.

In one non-limiting embodiment, the screening method in the present disclosure can select a peptide compound that binds to the target molecule as a pharmaceutical candidate compound in the step (b) described above.

In one non-limiting embodiment, the screening method in the present disclosure further includes the following steps:

(c) chemically synthesizing a peptide compound that can be a candidate; and

(d) measuring membrane permeability of the chemically synthesized peptide compound using a Caco-2 cell.

- Target molecule

The target molecules used in the screening method in the present disclosure are not particularly limited, and examples thereof include proteins, peptides, nucleic acids, sugars, lipids, and the like, but it is preferred that the targeted molecules are proteins. In one embodiment, according to the screening method in the present disclosure, it is possible to obtain peptide compounds that inhibit protein-protein interactions (PPIs). Although not intended to be bound by any specific theory, it is believed that there is a site called a "hot spot" on the surfaces of the two proteins involved in PPI, which enhances the binding strength of PPI. Examples of PPI include hydrophobic interactions, electrostatic interactions, van der Waals forces, a hydrogen bonding, photo-crosslinking, and salt bridges. Through such PPIs, phosphorylation of proteins, conformational changes or localization, and the formation of protein-protein complexes (for example, multi-subunit formation) can occur, potentially involving protein modification, transport, folding changes, signal transduction, and the like. Although not intended to be bound by any specific theory, it is considered that the side chains of the amino acids composing the peptide contained in the library in the present disclosure can inhibit PPI by entering this site. In other words, it is believed that according to the library and screening methods in the present disclosure, peptide compounds inhibiting PPI can be obtained regardless of the type of target molecules.

In one embodiment, when the library used in the screening method in the present disclosure contains cyclic peptide compounds (cyclic peptide-nucleic acid complexes) to which nucleic acids are linked, the actual expected pharmaceutical candidate compound may consist solely of a portion other than the nucleic acids and the nucleic acid linking portion (for example, the cyclic portion). The target molecule can be selected based on the presence or absence, or strength of interaction with the cyclic peptide moiety. In the cyclic peptide-nucleic acid complexes, when the nucleic acid portion contains a nucleotide sequence encoding for the cyclic peptide portion, the nucleic acid portion can facilitate understanding of the chemical structure and synthesis of the cyclic peptide portion and contribute to the efficiency of screening.

The target molecules in vivo are not particularly limited in their location either. In one embodiment, according to

the library and screening method in the present disclosure, it is possible to obtain cyclic peptide compounds with high membrane permeability, or cyclic peptide compounds that can easily enhance membrane permeability through derivatization, thereby allowing the targeting of intracellular proteins.

In one non-limiting embodiment, the target molecule used in the screening method in the present disclosure is used by being fixed on a carrier. The carrier is not particularly limited as long as the target molecule can be fixed, but examples thereof include beads or resins. The target molecule can be fixed on a carrier by known methods.

The target molecule of the screening method in the present disclosure is not particularly limited. The library in the present disclosure includes peptide compounds capable of specifically binding to various target molecules, which, in one embodiment, can enable drug discovery for tough targets that have been considered difficult for drug development.

- Method for producing peptide compound and library

In one embodiment, the cyclic peptide compound and the library containing such cyclic peptide compounds in the present disclosure can be produced, for example, by chemical synthesis, translation-based synthesis, or a combination thereof.

- Chemical synthesis method for peptide compound

As chemical synthesis methods for the peptide compounds in the present disclosure, examples include liquid-phase synthesis, solid-phase synthesis using Fmoc synthesis or Boc synthesis, and combinations thereof. In Fmoc synthesis, amino acids in which the backbone amino group is protected by an Fmoc group, the side chain functional group is protected by a protecting group that is not cleaved by bases like piperidine as needed, and the backbone carboxylic acid is not protected, are used as the basic units. As for the basic units, there are no specific limitations as long as they are combinations having Fmoc-protected amino groups and carboxylic acid groups. For example, a dipeptide may be used as a basic unit. The basic unit placed at the N-terminus may be one other than Fmoc amino acid. For example, it may be a Boc amino acid, or it may be a carboxylic acid analogue that does not have an amino group. The backbone carboxylic acid group is immobilized on the solid phase by a chemical reaction with a functional group of the solid phase carrier. Next, the Fmoc group is deprotected using a base such as piperidine or DBU, and the newly formed amino group is condensed with a protected amino acid having a carboxylic acid of a basic unit subsequently added, to form a peptide bond. In condensation reactions, various combinations are possible, such as the combination of DIC and HOBt, the combination of DIC and HOAt, and the combination of HATU and DIPEA. By repeating the deprotection of the Fmoc group followed by peptide bond formation reaction, the desired peptide sequence can be generated. Once the desired sequence is obtained, cleavage from the solid phase and deprotection of any side chain functional groups introduced as needed are performed. It is also possible to perform structural transformation or cyclization of the peptide before cleavage from the solid phase. Cleavage from the solid phase and deprotection can be performed under the same conditions, for example, of 90 : 10 TFA/$H_2O$, or the deprotection can be performed under separate conditions as needed. Cleavage from the solid phase can be performed with a weak acid such as 1% TFA in some cases, while in others, it is possible to use Pd as a protecting group and leverage the orthogonality of both chemical reactions. During or at the end of these processes, steps such as cyclization can also be performed. For example, it is possible to condense the side chain carboxylic acid with the N-terminal backbone amino group, or to condense the side chain amino group with the C-terminal backbone carboxylic acid. In this case, orthogonality in the reaction is required either between the C-terminal carboxylic acid and the side chain carboxylic acid to be cyclized, or between the N-terminal backbone amino group or hydroxy group and the side chain amino group to be cyclized, and as mentioned above, the protecting groups are selected considering the orthogonality of protecting groups. The reaction product obtained in this manner can be purified using a reverse-phase column or a molecular sieve column. Details of these are described in sources such as the "Solid Phase Synthesis Handbook" issued by Merck KGaA on May 1, 2002.

- Translation-based synthesis method of peptide compound

The peptide compound in the present disclosure may be synthesized by a translation-based synthesis method. In the present disclosure, the "translation-based synthesis method" refers to a method of synthesizing a peptide compound by recognizing a nucleotide sequence of mRNA with tRNA, rather than synthesizing exclusively by organic synthesis techniques. Examples of the translation-based synthesis method for a peptide compound include a method using a cell-free translation system, which includes a synthesis method using a reconstituted cell-free translation system. It is preferable to use a reconstituted cell-free translation system as it allows for the exclusion of undesirable factors.

In one aspect, the present disclosure provides a translation-based synthesis method of a peptide compound containing at least one, two or more, or three or more amino acids selected from the group consisting of the amino acids described in (A).

(A) Gly, Ala, Val, Leu, Ile, Thr, Phe, Pro, Abu, D-Ala, D-MeAla, D-MeVal, D-Val, Hph, MeAbu, MeAla, MeGly, MeIle, MeLeu, MePhe, MeThr, MeVal, nBuGly, Ser(tBu), Asp-pip, bAla, bMeAla, D-3-Abu, D-3-MeAbu, MeAsp-pip, and gMeAbu.

Without limitation, such a translation-based synthesis method may include the following (i) and (ii):

(i) a step of preparing a tRNA bound to at least one, two or more, or three or more amino acids selected from the group consisting of the amino acids described in (A); and
(ii) a step of translating a nucleic acid containing at least one codon corresponding to an anticodon of the tRNA in a cell-free translation system to obtain the peptide compound.

In one non-limiting embodiment, the translation-based synthesis method in the present disclosure may be a translation-based synthesis method of a cyclic peptide compound.

- Cell-free translation system

The term "cell-free translation system" as used herein is not limited as long as it contains ribosomes extracted from cells, groups of protein factors involved in translation, tRNAs, amino acids, energy sources such as ATPs, and regeneration systems thereof that can be combined to translate an mRNA into a protein. The system in which the translation-based synthesis reaction is in progress may be included in the "cell-free translation system" herein. The cell-free translation system herein may comprise a nucleic acid that serves as a template for translating a peptide, and may additionally comprise an initiation factor, an elongation factor, a dissociation factor, an aminoacyl tRNA synthetase, and the like. These factors can be obtained by purification from extracts of various cells. Examples of the cells for purifying factors can include prokaryotic cells and eukaryotic cells. Examples of the prokaryotic cells can include Escherichia coli cells, extreme thermophile cells, or Bacillus subtilis cells. As the eukaryotic cells, those made from yeast cells, wheat germ, rabbit reticulocytes, plant cells, insect cells, or animal cells are known. As the tRNA and aminoacyl tRNA synthetase (ARS), not only naturally occurring ones, but also artificial tRNAs and artificial aminoacyl tRNA synthetases that recognize a non-natural amino acid can be used. By using the artificial tRNA and artificial aminoacyl tRNA synthetase, it is possible to synthesize a peptide in which a non-natural amino acid is site-specifically introduced. If necessary, RNA polymerase such as T7 RNA polymerase can be added to the cell-free translation system to perform transcription from template DNA.

PURESYSTEM(R) (manufactured by BioComber Co., Ltd., Japan) is a reconstituted cell-free translation system in which protein factors, energy regeneration system enzymes, and ribosomes necessary for translation in E. coli are extracted and purified, and mixed with tRNA, amino acids, ATP, GTP, and the like. PURESYSTEM(R) not only has a low impurity content, but also allows easy preparation of systems that do not contain protein factors or amino acids that are desired to be eliminated, because it is a reconstitution system ((i) Nat Biotechnol. 2001; 19: 751-5. Cell-free translation reconstituted with purified components. Shimizu Y, Inoue A, Tomari Y, Suzuki T, Yokogawa T, Nishikawa K, Ueda T. (ii) Methods Mol Biol. 2010; 607: 11-21. PURE technology. Shimizu Y, Ueda T.).

For example, many methods in which a stop codon is used as a codon that introduces a non-natural amino acid have been reported. However, by using PURESYSTEM described above, a synthetic system excluding natural amino acids and ARS can be constructed. This allows a non-natural amino acid to be associated with a codon encoding the natural amino acid to be removed (J Am Chem Soc. 2005; 127: 11727-35. Ribosomal synthesis of non-natural peptides. Josephson K, Hartman MC, Szostak JW.). Furthermore, by breaking the degeneracy of the codon, a non-natural amino acid can be added without excluding natural amino acids (Kwon I, et al. Breaking the degeneracy of the genetic code. J Am Chem Soc. 2003, 125, 7512-3.). Peptides containing N-methylamino acids can be synthesized in ribosomes by using cell-free translation systems such as PURESYSTEM.

More specifically, translation-based synthesis can be carried out by adding mRNA to a known cell-free translation system like PURESYSTEM, which is a mixture of components appropriately selected from protein factors necessary for E. coli translation (methionyl-tRNA transformylase, EF-G, RF1, RF2, RF3, RRF, IF1, IF2, IF3, EF-Tu, EF-Ts, ARS (selected from AlaRS, ArgRS, AsnRS, AspRS, CysRS, GlnRS, GluRS, GlyRS, HisRS, IleRS, LeuRS, LysRS, MetRS, PheRS, ProRS, SerRS, ThrRS, TrpRS, TyrRS, ValRS as needed)), ribosomes, amino acids, creatine kinase, myokinase, inorganic pyrophosphatase, nucleoside diphosphate kinase, tRNA derived from E. coli, creatine phosphate, potassium glutamate, HEPES-KOH pH7.6, magnesium acetate, spermidine, dithiothreitol, GTP, ATP, CTP, UTP, and the like. When T7 RNA polymerase is added, transcription and translation from a template DNA containing a T7 promoter can also be performed in a coupled manner. In addition, a peptide compound containing a non-natural amino acid can be obtained by translation-based synthesis by adding a group of desired aminoacyl tRNAs or a group of non-natural amino acids (e.g., F-Tyr) that are allowed by aminoacyl tRNA synthetases (ARS) to the system (Kawakami T, et al. Ribosomal synthesis of polypeptoids and peptoid-peptide hybrids. J Am Chem Soc. 2008, 130, 16861-3., Kawakami T, et al. Diverse backbone-cyclized peptides via codon reprogramming. Nat Chem Biol. 2009, 5, 888-90.). Furthermore, a peptide compound containing a non-natural

amino acid can also be obtained by translation-based synthesis by adding a modified ARS to a system instead of or in addition to natural ARS, and adding a group of non-natural amino acids to the system. Alternatively, mutants of ribosomes and EF-Tu or the like can be used to increase the efficiency of translation introduction of the non-natural amino acid (Dedkova LM, et al. Construction of modified ribosomes for incorporation of D-amino acids into proteins. Biochemistry 2006, 45, 15541-51., Doi Y, et al. Elongation factor Tu mutants expand amino acid tolerance of protein biosynthesis system. J Am Chem Soc. 2007, 129, 14458-62., Park HS, et al. Expanding the genetic code of Escherichia coli with phosphoserine. Science 2011, 333, 1151-4.).

In one non-limiting embodiment, the cell-free translation system in the present disclosure may be a translation system for producing peptide compounds, preferably a translation system for producing cyclic peptide compounds.

- tRNA

In the translational incorporation of a non-natural amino acid into a peptide, aminoacylation of a tRNA that is orthogonal and efficiently incorporated by the ribosome ((i) Biochemistry 2003; 42: 9598-608. Adaptation of an orthogonal archaeal leucyl-tRNA and synthetase pair for four-base, amber, and opal suppression. Anderson JC, Schultz PG., (ii) Chem Biol. 2003; 10: 1077-84. Using a solid-phase ribozyme aminoacylation system to reprogram the genetic code. Murakami H, Kourouklis D, Suga H.) is required. The following five methods can be used as a method for aminoacylating tRNA.

Inside cells, aminoacyl tRNA synthetase (ARS) is provided for each amino acid individually as an enzyme for aminoacylation of tRNA. Thus, as the first method, a method of using certain ARS that allows non-natural amino acids such as N-Me His, and a method of preparing and utilizing a modified aminoacyl tRNA synthetase that allows non-natural amino acids can be used ((i) Proc.Natl.Acad.Sci. U S A. 2002; 99: 9715-20. An engineered Escherichia coli tyrosyl-tRNA synthetase for site-specific incorporation of a non-natural amino acid into proteins in eukaryotic translation and its application in a wheat germ cell-free system. Kiga D, Sakamoto K, Kodama K, Kigawa T, Matsuda T, Yabuki T, Shirouzu M, Harada Y, Nakayama H, Takio K, Hasegawa Y, Endo Y, Hirao I, Yokoyama S. (ii) Science 2003; 301: 964-7. An expanded eukaryotic genetic code. Chin JW, Cropp TA, Anderson JC, Mukherji M, Zhang Z, Schultz PG. Chin, JW. (iii) Proc.Natl.Acad.Sci. U S A. 2006; 103: 4356-61. Enzymatic aminoacylation of tRNA with non-natural amino acids. Hartman MC, Josephson K, Szostak JW.). Second, a method of aminoacylating tRNA and then chemically modifying the amino acid in a test tube can be used (J Am Chem Soc. 2008; 130: 6131-6. Ribosomal synthesis of N-methyl peptides. Subtelny AO, Hartman MC, Szostak JW.). Third, binding tRNA in which CA is removed from the CCA sequence at the 3' end to an aminoacylated pdCpA prepared separately with an RNA ligase can be performed to obtain an aminoacyl tRNA (Biochemistry 1984; 23: 1468-73. T4 RNA ligase mediated preparation of novel "chemically misacylated" tRNAPheS. Heckler TG, Chang LH, Zama Y, Naka T, Chorghade MS, Hecht SM.). Aminoacylation by flexizyme, which is a ribozyme that makes active esters of various non-natural amino acids carried on tRNA, can also be used (J Am Chem Soc. 2002; 124: 6834-5. Aminoacyl-tRNA synthesis by a resin-immobilized ribozyme. Murakami H, Bonzagni NJ, Suga H.). Fourth, a method of ultrasonically mixing tRNA and an amino acid active ester in a cationic micelle can also be used (Chem Commun (Camb). 2005; (34): 4321-3. Simple and quick chemical aminoacylation of tRNA in cationic micellar solution under ultrasonic agitation. Hashimoto N, Ninomiya K, Endo T, Sisido M.). Fifth, aminoacylation is also possible by adding an amino acid active ester bound to PNA complementary to near the 3' end of tRNA to tRNA (J Am Chem Soc. 2004; 126: 15984-9. In situ chemical aminoacylation with amino acid thioesters linked to a peptide nucleic acid. Ninomiya K, Minohata T, Nishimura M, Sisido M.).

More specifically, aminoacyl tRNA can be produced by using the following method. RNA can be synthesized by preparing a template DNA that encodes a desired tRNA sequence and disposes a T7, T3 or SP6 promoter upstream and transcribing with RNA polymerases adapted for promoters, such as T7 RNA polymerase and T3, and SP6 RNA polymerase. A generated tRNA of interest can also be extracted by extracting and purifying tRNA from the cells and using probes of complementary sequences of tRNA sequence. At this time, the cells transformed with an expression vector of tRNA of interest can be used as a source. RNA of the sequence of interest can also be synthesized by chemical synthesis. For example, an aminoacyl tRNA can be obtained by binding tRNA in which CA is removed from the CCA sequence at the 3' end obtained in this way to an aminoacylated pdCpA or pCpA prepared separately with an RNA ligase (pdCpA method, pCpA method). The tRNA is useful in the production of peptide compounds. Alternatively, full-length tRNA is prepared and aminoacylation by flexizyme, which is a ribozyme that makes active esters of various non-natural amino acids carried on tRNA, can be performed. Aminoacyl tRNA can also be produced using natural ARS or a modified one thereof, although this is not intended to be limiting. When natural ARS or a modified one thereof is used, it is not necessary to have a large amount of previously-prepared aminoacyl tRNA in the translation system, because the aminoacyl tRNA once consumed in the translation system can be regenerated by natural ARS or a modified one thereof. Such modified ARS are described in WO 2016/148044. These methods for producing aminoacyl tRNAs can also be used in appropriate combination.

- Method for cyclizing peptide moiety

The cyclic peptide compound in the present disclosure has a cyclic portion. The cyclization method of the peptide compound is not particularly limited, but for example, a cyclization reaction can be performed after synthesizing the peptide compound by chemical synthesis or translation-based synthesis. Examples of the cyclization include cyclization using an amide bond, a carbon-carbon bond, a thioether bond, a disulfide bond, an ester bond, a thioester bond, a lactam bond, a bond via a triazole structure, or a bond via a fluorophore structure. The synthesis step of the peptide compound and the cyclization reaction steps may be performed separately or continuously. Cyclization can be performed by the methods known to those skilled in the art, such as those described in WO 2013/100132, WO 2008/117833, or WO 2012/074129.

The cyclized portion may be, but is not limited to, the bonding between the N-terminus and the C-terminus of the peptide, the bonding between the N-terminus and a side chain of another amino acid residue of the peptide, the bonding between the C-terminus and a side chain of another amino acid residue of the peptide, or the bonding between the side chains of amino acid residues, and two or more of these may be used in combination. When cyclizing a peptide-nucleic acid complex in which a nucleic acid is linked to the C-terminus, it may involve the bonding between the N-terminus of the peptide and the side chain of another amino acid residue, or between the side chains of amino acid residues.

In one non-limiting embodiment, the cyclic peptide compound in the present disclosure can be prepared by the following method. Examples include the production method including the following steps:

(1) a step of synthesizing a non-cyclic peptide compound composed of amino acid residues, or amino acid residues and N-terminal carboxylic acid analogue, by translating from the nucleic acids that encode the peptide compound, wherein the non-cyclic peptide compound includes an amino acid residue having a reaction point in one of the side chains on the C-terminus, and an amino acid residue or N-terminal carboxylic acid analogue having another reaction point on the N-terminus; and

(2) binding the reaction point of the amino acid residue or N-terminal carboxylic acid analogue on the N-terminus and the reaction point of the amino acid residue at the side chain on the C-terminus to form an amide bond, a carbon-carbon bond, or a thioether bond.

These steps (1) and (2) may be performed separately or continuously.

One non-limiting embodiment of the method for cyclizing peptide compounds via an amide bond is a method of translating a peptide compound that has cysteine or a cysteine analog at the N-terminus and an active ester on the side chain of the amino acid at the C-terminus, and cyclizing it using native chemical ligation. The translation-based synthesis method of the peptide compound that has cysteine or a cysteine analog at the N-terminus, which is a precursor to this cyclic peptide compound, is not particularly limited.

In one embodiment, to synthesize a peptide compound having an amino acid other than Met or Nle at the N-terminus of the peptide compound, the following three methods, for example, can be used.

The first method is a method of cleaving the N-terminal amino acid or N-terminal peptide of a peptide initiated with formylmethionine or Met using an enzyme. Examples of the enzyme include peptide deformylase (PDF) and/or methionine aminopeptidase (MAP).

The second method is a method of using a reconstituted cell-free translation system, removing Met from the system in advance, placing an amino acid that is desired to be positioned at the N-terminus at the second residue from the N-terminus, and performing translation-based synthesis by reading through the start codon Met. This method is defined as the initiation readthrough (iRT) method.

The third method is a method of performing translation-based synthesis by adding an aminoacyl tRNA prepared by aminoacylating an amino acid that is desired to be positioned at the N-terminus as the initiator tRNA in advance to a reconstituted cell-free translation system that does not contain Met or initiator methionine tRNA. This method is defined as the initiation suppression (iSP) method.

In one non-limiting embodiment, the C-terminal moiety of the cyclic peptide compound in the present disclosure may be chemically modified, rather than remaining as a carboxylic acid. For example, the carboxylic acid moiety may be reacted with piperidine or the like to be converted to piperidinamide or the like.

[Examples]

The present invention will be described in more detail on the basis of following Examples, which should not be construed as limiting the present invention. All starting materials and reagents were obtained from commercial suppliers, or synthesized in accordance with known methods.

In the Examples, the following abbreviations were used.

AA: ammonium acetate

DBU: 1,8-diazabicyclo[5.4.0]-7-undecene
DCE: 1,2-dichloroethane
DCM: dichloromethane
DIC: N,N'-diisopropylcarbodiimide
DIPEA: N,N-diisopropylethylamine
DMF: N,N-dimethylformamide
DMSO: dimethyl sulfoxide
Fmoc: 9-fluorenylmethyloxycarbonyl
HATU: O-(7-aza-1H-benzotriazol-1-yl)-N,N,N',N'-tetramethyluronium hexafluorophosphate
HFIP: 1,1,1,3,3,3-hexafluoro-2-propanol
HOAt: 1-hydroxy-7-azabenzotriazole
NMP: N-methyl-2-pyrrolidone
TBME: t-butyl methyl ether
TFE: 2,2,2-trifluoroethanol
TIPS: triisopropylsilane
Trt: triphenylmethyl
oxyma: ethyl cyano(hydroxyimino)acetate

The reaction solvents used for peptide synthesis and solid-phase synthesis were those for peptide synthesis (purchased from KANTO CHEMICAL CO., INC., FUJIFILM Wako Pure Chemical Corporation, or WATANABE CHEMICAL INDUSTRIES, LTD.). For example, DCM, DMF, DMSO, and DBU (2% DMF solution) were used. In reactions where water was not used as a solvent, dehydrating solvents or anhydrous solvents (purchased from KANTO CHEMICAL CO., INC., FUJIFILM Wako Pure Chemical Corporation, or the like) were used.

Example 1. Synthesis of compound such as amino acids for use in peptide synthesis

For peptide synthesis described herein, amino acids shown in Tables 1, 2, and 3, and amino acid-support resins were used.

Example 1-1. Fmoc-amino acid purchased and used as such

The Fmoc-amino acids shown in Table 1 were purchased from commercial suppliers. As used herein, when -OH is described in addition to the abbreviation of an amino acid in Table 1, it means that the C-terminus of that amino acid is a carboxyl group. For example, Gly-OH means that the C-terminus of glycine is a carboxy group, and Fmoc-Gly-OH means that the N-terminal amino group of glycine is protected by a Fmoc group and the C-terminal is a carboxy group.

[Table 1]

| Abbreviation | Structure | Abbreviation | Structure |
|---|---|---|---|
| Fmoc-Abu-OH | 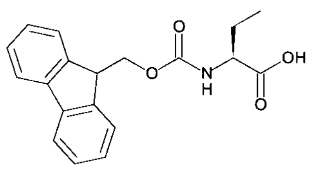 | Fmoc-Ala-OH | 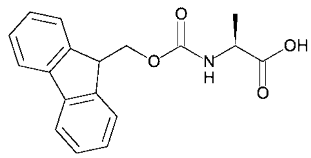 |
| Fmoc-bAla-OH | 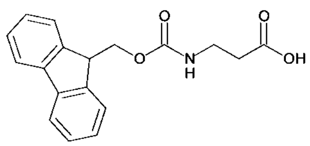 | Fmoc-bMeAla-OH | 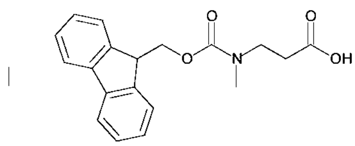 |
| Fmoc-D-3-Abu-OH | 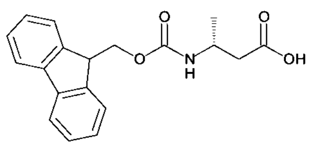 | Fmoc-D-3-MeAbu-OH | 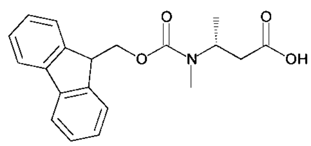 |

(continued)

| Abbreviation | Structure | Abbreviation | Structure |
|---|---|---|---|
| Fmoc-D-Ala-OH | 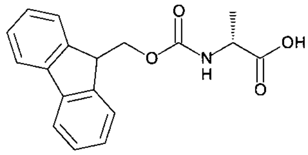 | Fmoc-D-MeAla-OH | 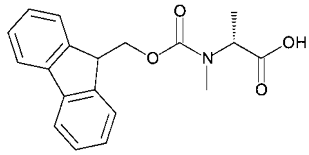 |
| Fmoc-D-MeVal-OH | 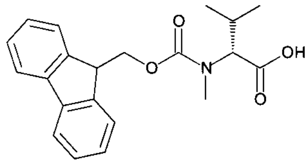 | Fmoc-D-Val-OH | 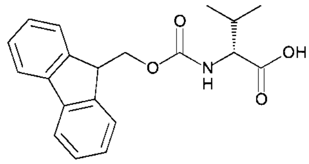 |
| Fmoc-Gly-OH | 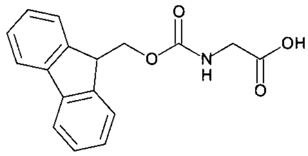 | Fmoc-gMeAbu-OH | 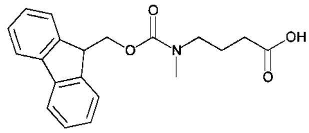 |
| Fmoc-Hph-OH | 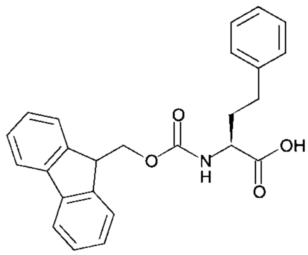 | Fmoc-Ile-OH | 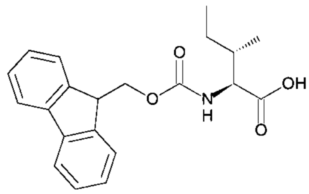 |
| Fmoc-Leu-OH | 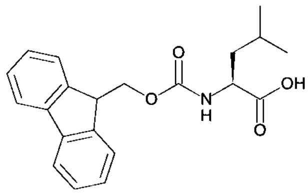 | Fmoc-MeAbu-OH | 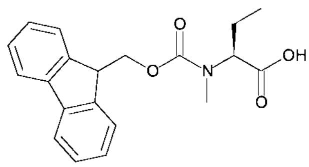 |
| Fmoc-MeAla-OH | 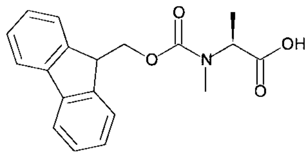 | Fmoc-MeGly-OH | 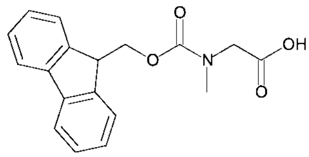 |
| Fmoc-Melle-OH | 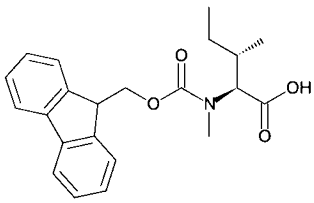 | Fmoc-MeLeu-OH | 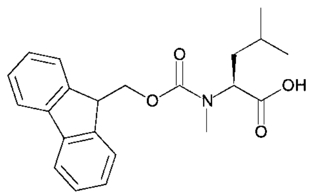 |
| Fmoc-MePhe-OH | 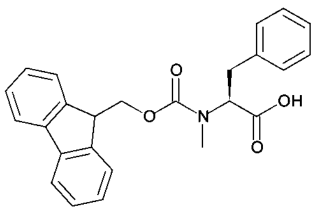 | Fmoc-MeThr-OH | 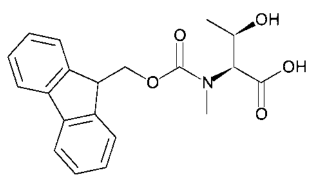 |

(continued)

| Abbreviation | Structure | Abbreviation | Structure |
|---|---|---|---|
| Fmoc-MeVal-OH | 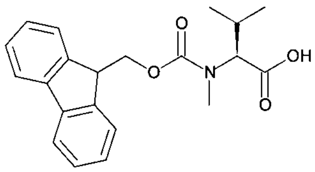 | Fmoc-nBuGly-OH | 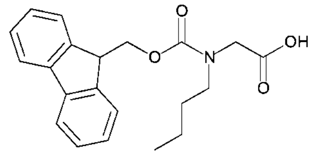 |
| Fmoc-Phe-OH | 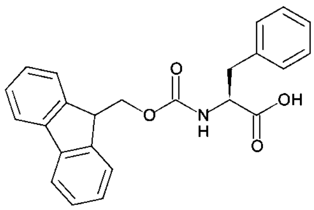 | Fmoc-Pro-OH | 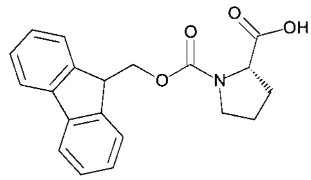 |
| Fmoc-Ser(tBu)-OH | 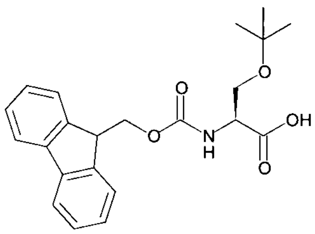 | Fmoc-Val-OH | 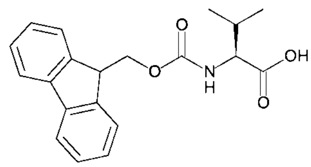 |

Example 1-2. Synthesis of Fmoc-amino acid

The Fmoc-amino acids shown in Table 2 were synthesized in accordance with the schemes shown below.

[Table 2]

| Compound No. | Abbreviation |
|---|---|
| aa001 | Fmoc-Asp-pip |
| aa002 | Fmoc-MeAsp-pip |
| aa003 | Fmoc-Thr(THP)-OH |

Compound aa001

Synthesis of (3S)-3-(9H-fluoren-9-ylmethoxycarbonylamino)-4-oxo-4-piperidin-1-ylbutanoic acid (Fmoc-Asp-pip)

[Formula 4]

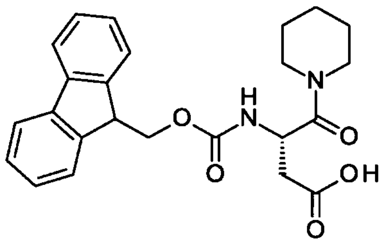

Compound aa001 was synthesized according to the method described in WO 2013/100132.

Compound aa002

Synthesis of (3S)-3-[9H-fluoren-9-ylmethoxycarbonyl(methyl)amino]-4-oxo-4-piperidin-1-ylbutanoic acid (Fmoc-MeAsp-pip)

[Formula 5]

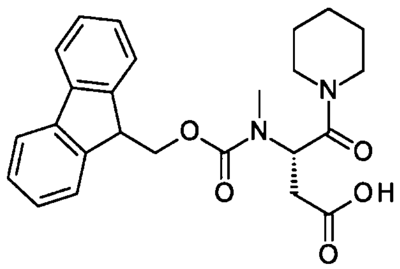

Compound aa002 was synthesized according to the method described in WO 2018/225864.

Compound aa003

Synthesis of (2S,3R)-2-(9H-fluoren-9-ylmethoxycarbonylamino)-3-(oxan-2-yloxy)butanoic acid (Fmoc-Thr(THP)-OH)

[Formula 6]

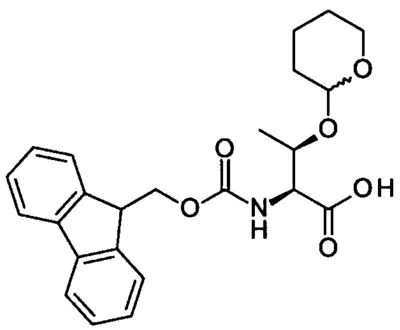

Compound aa003 was synthesized according to the method described in WO 2018/225864.

Example 1-3. Synthesis of amino acid-support resin

[Formula 7]

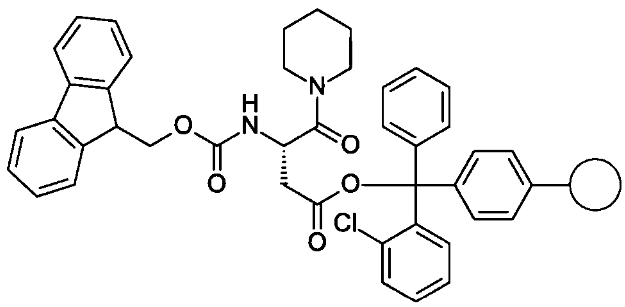

**Fmoc-Asp(O-Trt(2-Cl)resin)-pip**

As used herein, a polymer or resin moiety may be expressed by O when the compound is bound to a polymer or

resin. For the purpose of clarifying the reaction point of the resin moiety, the chemical structure of the reaction site may be shown connected to O. For example, in the above structure Fmoc-Asp (O-Trt (2-Cl)-resin)-pip, the 2-chlorotrityl group of the resin is bonded to the carboxyl group on the side chain of Asp through an ester bond. The term "pip" means piperidine, and in the above structure, the carboxyl group at the C-terminal forms an amide bond with piperidine. 2-Chlorotrityl chloride resin (1.25-1.60 mmol/g, 100-200 mesh, 1% DVB) was purchased from WATANABE CHEMICAL INDUSTRIES, LTD. and SUNRESIN Co. Ltd.

The Fmoc-amino acid-support resin shown in Table 3 was synthesized in accordance with the schemes shown below.

[Table 3]

| Compound No. | Abbreviation |
|---|---|
| rs001 | Fmoc-Asp(O-Trt(2-Cl)resin)-pip |
| rs002 | Fmoc-D-3-Abu-O-Trt(2-Cl)resin |
| rs003 | Fmoc-D-3-MeAbu-O-Trt(2-Cl)resin |
| rs004 | Fmoc-MeAsp(O-Trt(2-Cl)resin)-pip |

Compound rs001:

Synthesis of (3S)-3-(9H-fluoren-9-ylmethoxycarbonylamino)-4-oxo-4-piperidin-1-ylbutanoic acid-2-chlorotrityl resin (Fmoc-Asp(O-Trt(2-Cl)-resin)-pip)

[Formula 8]

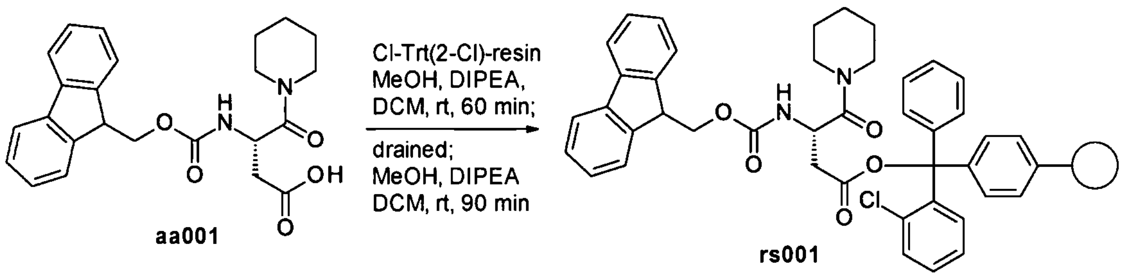


aa001 rs001

To a reaction vessel with a filter, 2-chlorotrityl chloride resin (1.60 mmol/g, 50.0 g, 80.0 mmol) and DCM (676 mL) were added, and the mixture was shaken at room temperature for 10 minutes. Nitrogen pressure was applied to remove DCM, then a solution obtained by mixing compound aa001 (16.9 g, 40.0 mmol), methanol (12.9 mL, 320 mmol), DCM (676 mL), and DIPEA (33.4 mL, 192 mmol) was added to the reaction vessel and shaken at room temperature for 60 minutes. Nitrogen pressure was applied to remove the reaction solution, then a mixture of methanol (99.7 mL, 2.46 mmol), DCM (676 mL), and DIPEA (33.4 mL, 192 mmol) was added to the reaction vessel and shaken at room temperature for 90 minutes. Nitrogen pressure was applied to remove the reaction solution, then DCM (676 mL) was added. The reaction vessel was shaken for 10 minutes, and then nitrogen pressure was applied to remove the reaction solution. The resin washing operation using DCM was repeated twice, and the obtained resin was dried overnight under reduced pressure to obtain 61.8 g of compound rs001 (support amount 0.452 mmol/g).

Synthesis of compound rs002, (3R)-3-(9H-fluoren-9-ylmethoxycarbonylamino)butanoic acid-2-chlorotrityl resin (Fmoc-D-3-Abu-O-Trt (2-Cl)resin)

[Formula 9]

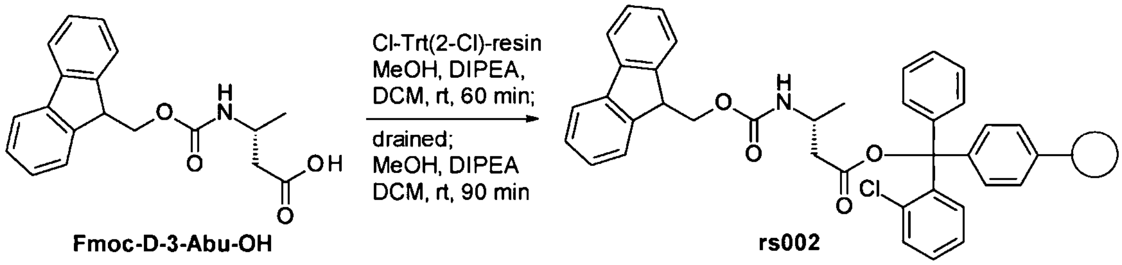


Fmoc-D-3-Abu-OH

rs002

Using 2-chlorotrityl chloride resin (1.44 mmol/g, 200 g, 289 mmol) and Fmoc-D-3-Abu-OH (47.0 g, 144 mmol) as starting materials, 230 g (support amount 0.494 mmol/g) of compound rs002 was obtained in the same manner as the synthesis of compound rs001.

Synthesis of compound rs003, (3R)-3-(9H-fluoren-9-ylmethoxycarbonyl(methyl)amino)butanoic acid-2-chlorotrityl resin (Fmoc-D-3-MeAbu-O-Trt (2-Cl)resin)

[Formula 10]

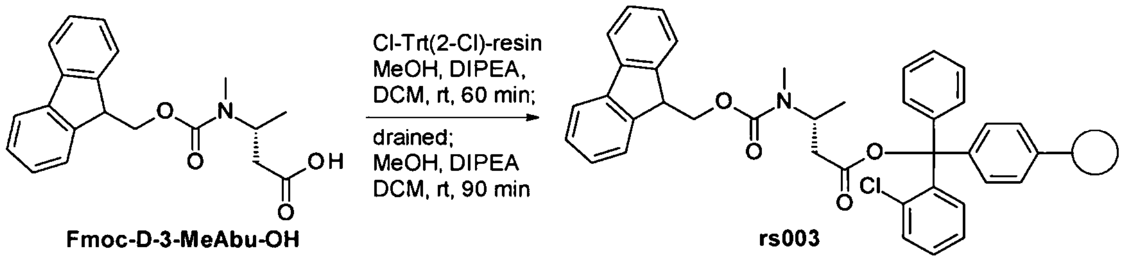


Fmoc-D-3-MeAbu-OH

rs003

Using 2-chlorotrityl chloride resin (1.25 mmol/g, 200 g, 250 mmol) and Fmoc-D-3-MeAbu-OH (42.5 g, 125 mmol) as starting materials, 228 g (support amount 0.495 mmol/g) of compound rs003 was obtained in the same manner as the synthesis of compound rs001.

Synthesis of compound rs004, (3S)-3-(9H-fluoren-9-ylmethoxycarbonyl(methyl)amino)-4-oxo-4-piperidin-1-ylbutanoic acid-2-chlorotrityl resin (Fmoc-MeAsp(O-Trt(2-Cl)-resin)-pip)

[Formula 11]

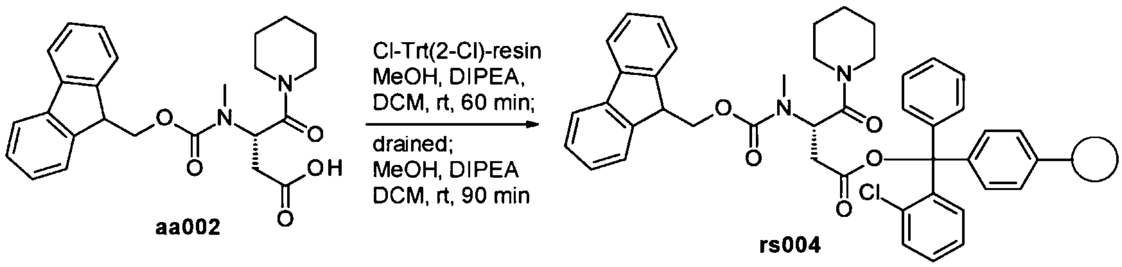


aa002

rs004

Using 2-chlorotrityl chloride resin (1.44 mmol/g, 200 g, 289 mmol) and compound aa002 (82.2 g, containing 23 w% of TBME, 145 mmol) as starting materials, 239 g (support amount 0.509 mmol/g) of compound rs004 was obtained in the same manner as the synthesis of compound rs001.

Example 2. Synthesis of cyclic peptide compounds

A cyclic peptide compound composed of 13 or 14 amino acid residues was synthesized through peptide elongation by solid phase reaction, cleavage, cyclization, side chain deprotection, and purification in accordance with the scheme shown in Fig. 1.

The peptide elongation by solid phase reaction described in this Example was performed by the Fmoc method using a peptide synthesizer (Multipep RS, manufactured by CEM Corporation (formerly Intavis AG)). Detailed procedures of operations were performed in accordance with the manual attached to the synthesizer.

Detailed synthesis conditions in the present Example are shown in the following.

Peptide elongation by solid phase reaction

An Fmoc-protected amino acid (0.6 mol/L) composing a peptide of interest, and HOAt or oxyma (0.375 mol/L) as a carboxyl group-activating agent were dissolved in NMP to prepare solution 1. In addition, DIC was mixed with DMF to be a concentration of 10% (v/v) to prepare solution 2. A solid phase reaction vessel with a filter (frit) was charged with a resin (100 mg) supporting the Fmoc amino acids prepared in Example 1-3, and was set in the peptide synthesizer. DCM (1.2 mL) was added, the mixture was left standing for 30 minutes to swell the resin, and then the solution was drained from the frit. Solutions 1 and 2 were set in the peptide synthesizer, and automated synthesis with the peptide synthesizer was started.

A solution of DBU in DMF (2%v/v, 0.7 mL per column) was added to the solid phase reaction vessel containing the resin, and the Fmoc group removal reaction was carried out at room temperature. The mixture was reacted for 4.5 minutes in deprotection at the first residue, and reacted for 10 minutes in deprotection at the second and subsequent residues, and then the solution was drained from the frit. DMF (0.7 mL) was added thereto, the mixture was left standing for 5 minutes, and then the solution was drained from the frit. This resin washing step was repeated three more times. Subsequently, solution 1 (0.3 mL) was mixed with solution 2 (0.36 mL) in a mixing vial of the synthesizer, and then the mixture was added to the resin. The solid phase reaction vessel was heated to 40°C, and the mixture was allowed to react for 2.5 hours to perform a condensation reaction of the amino group on resin with the Fmoc-protected amino acid, and then the solution was drained from the frit. Subsequently, the resin was washed 3 times with DMF (0.7 mL). The Fmoc group removal reaction, followed by the condensation reaction of the Fmoc amino acid, was taken as a cycle, and the cycle was repeated to elongate a peptide on a resin surface. After completion of the peptide elongation, a DMF solution of DBU (2%v/v, 0.7 mL) was added into the solid phase reaction vessel containing the resin, the mixture was reacted at room temperature for 15 minutes to carry out a Fmoc group removal reaction, and then the solution was drained from the frit. The obtained resin was washed four times with DMF (0.7 mL) and then washed four times with DCM (0.7 mL).

Cleavage from resin

To the obtained resin was added TFE/DCM (1/1, 2.0 mL) containing DIPEA at 0.75% (v/v), and the mixture was reacted at room temperature for 2 hours to perform a cleavage reaction of the peptide chain from the resin. After the reaction, the solution in the tube was recovered from the frit. The operation of adding TFE/DCM (1/1 (v/v), 1.0 mL) to the remaining resin and recovering the solution from the frit was carried out twice. All the obtained cleavage solutions were mixed, DMF (4.0 mL) was mixed thereto, and the solvent was then removed by distillation under reduced pressure using High-Throughput Centrifugal Evaporator (HT-12) manufactured by Genevac Company.

Cyclization

The residue obtained by the above-described method was dissolved in a mixed liquid of DMF (4.0 mL) and DCM (4.0 mL), a solution of HATU in DMF (0.5 mol/L, 1.5 equivalents) and DIPEA (1.8 equivalents) were added, the mixture was stirred at room temperature for 2 hours to carry out a condensation cyclization reaction of an amino group at the N-terminal and a carboxyl group serving as a moiety of binding to the resin, and the solvent was then removed by distillation under reduced pressure using High-Throughput Centrifugal Evaporator (HT-12) manufactured by Genevac Company. The number of equivalents was calculated with respect to a value obtained by multiplying the amount of support of the peptide on the resin used as a raw material (mmol/g) by the amount of the resin used.

Deprotection

Deprotection was performed only for Thr-containing peptides. For peptides that do not contain Thr, the deprotection was omitted.

A deprotection reaction of a Thr-side chain was performed by dissolving the residue obtained by the above-described method in a HFIP/TIPS/DCE (117/2.4/1 (v/v/v)) solution of tetramethylammonium hydrogen sulfate (0.10 mol/L,

4.0 mL) and stirring it at room temperature for 1 hour. DIPEA (0.070 mL) was added thereto, and the solvent was then removed by distillation under reduced pressure using High-Throughput Centrifugal Evaporator (HT-12) manufactured by Genevac Company.

Purification

DMSO was added to the residue obtained by the above-described method, insoluble substances were removed by filtration, and purification was then performed by preparative-HPLC to obtain an intended cyclic peptide. Waters Auto Purification System was used as a purification apparatus, YMC-Actus Triart C18 (internal diameter: 20 mm and length: 100 mm) was used as a column, and an aqueous methanol-ammonium acetate solution (50 mmol/L) was used as a mobile phase.

In accordance with the above synthesis conditions, cyclic peptides consisting of 13 or 14 amino acid residues as listed in Table 4 were synthesized. In Table 4, sequences of amino acids were shown from the N-terminus in order in columns 1 to 14, and the exact mass, LC-MS analysis conditions (LCMS cond.), observed mass spectrum (obs. mass; m/z), ion form, and retention time (Rt; min) of each compound were described. All peptides are cyclic peptides in which an amide bond between an amino group at the N-terminus and a carboxyl group at the C-terminus (carboxyl group of the side chain when the C-terminus is Asp or MeAsp) is formed. The analysis conditions of LCMS are shown in Table 5. The structural formula of each compound is shown in Table 15.

[Table 4]

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd001 | aMeAbu | MeLeu | MeIle | Phe | Pro | MeLeu | Ser(tBu) | MePhe | Thr | MeAbu | Pro | MeLeu | MeVal | Asp-pip | 1748.1 | AA50 | 1747.7 | [M-H]- | 0.92 |
| pd002 | MeAla | MeLeu | MeAbu | Val | MeAbu | MePhe | Thr | MeIle | MePhe | MeLeu | Pro | Ile | MeVal | Asp-pip | 1692.1 | AA50 | 1691.6 | [M-H]- | 0.90 |
| pd003 | MeAla | Leu | MePhe | bMeAla | MeLeu | Leu | MeAla | MeAbu | MeIle | Thr | MeGly | MeLeu | MePhe | Asp-pip | 1638.1 | AA50 | 1637.6 | [M-H]- | 0.84 |
| pd004 | MeAla | Ile | MePhe | MeVal | MeLeu | Thr | MeGly | MePhe | MeLeu | MeAla | MeLeu | Ser(tBu) | MeAbu | Asp-pip | 1696.1 | AA50 | 1695.7 | [M-H]- | 0.86 |
| pd005 | MeGly | MePhe | bAla | MeLeu | MeThr | MeLeu | MeAla | Abu | MePhe | Val | Pro | MeAla | MeVal | Asp-pip | 1580.0 | AA50 | 1579.4 | [M-H]- | 0.79 |
| pd006 | D-Ala | MeAla | MePhe | Val | MeLeu | Val | MeGly | Leu | MeLeu | Abu | Hph | MeAbu | MeVal | D-3-Abu | 1497.0 | AA50 | 1496.5 | [M-H]- | 0.84 |
| pd007 | MeAla | Leu | MeLeu | Ile | MePhe | bAla | MeLeu | Ser(tBu) | MeIle | Val | MeGly | MeLeu | Leu | D-3-Abu | 1563.1 | AA50 | 1562.6 | [M-H]- | 0.92 |
| pd008 | D-Ala | MeAla | MePhe | Val | MeLeu | Thr | MeGly | Val | MeLeu | Ser(tBu) | MePhe | Abu | MeVal | D-3-Abu | 1529.0 | AA50 | 1528.5 | [M-H]- | 0.83 |
| pd009 | MeAla | Leu | MeLeu | Ile | MePhe | Ala | MeLeu | Ser(tBu) | MeIle | Val | Thr | MeLeu | MeGly | D-3-Abu | 1551.0 | AA50 | 1550.5 | [M-H]- | 0.89 |
| pd010 | MeGly | Ile | MeLeu | Abu | MeLeu | Thr | MeGly | Leu | MePhe | Ala | MeLeu | Ser(tBu) | MeVal | D-3-Abu | 1509.0 | AA50 | 1508.5 | [M-H]- | 0.87 |
| pd011 | MeGly | Ile | MeLeu | Abu | MeLeu | Thr | MeGly | Val | Ala | MePhe | MeLeu | Ser(tBu) | MeVal | D-3-Abu | 1495.0 | AA50 | 1494.5 | [M-H]- | 0.84 |
| pd012 | D-MeAla | Val | MePhe | Ile | MeLeu | Thr | Pro | MeAla | MeLeu | Ser(tBu) | MePhe | Abu | MeVal | D-3-Abu | 1583.0 | AA50 | 1582.5 | [M-H]- | 0.87 |
| pd013 | D-Val | MeAbu | Ala | MeVal | Pro | Leu | MeGly | MeAla | Ser(tBu) | Val | MePhe | Abu | MeVal | Asp-pip | 1532.0 | AA50 | 1531.5 | [M-HI- | 0.82 |
| pd014 | D-Val | MeLeu | Ala | MeVal | Pro | Leu | MePhe | MeLeu | Ser(tBu) | Val | MeLeu | Ala | MeVal | Asp-pip | 1644.1 | AA50 | 1643.7 | [M-H]- | 0.93 |
| pd015 | bMeAla | Leu | MeLeu | MePhe | Ile | Abu | MePhe | Ser(tBu) | MeLeu | Val | MeAla | MeLeu | Val | Asp-pip | 1708.1 | AA50 | 1707.7 | [M-H]- | 0.91 |
| pd016 | gMeAbu | Leu | MeLeu | MeAla | MePhe | Abu | MePhe | Ser(tBu) | MeLeu | Abu | MeAla | Val | MeLeu | Asp-pip | 1680.1 | AA50 | 1679.7 | [M-H]- | 0.89 |
| pd017 | gMeAbu | MeLeu | bMeAla | Phe | Pro | MeLeu | Ser(tBu) | MeLeu | Ala | Ile | MeAbu | Leu | MePhe | Asp-pip | 1692.1 | AA50 | 1691.7 | [M-H]- | 0.92 |
| pd018 | MeAla | Ile | MePhe | MeLeu | Val | MeGly | Leu | MePhe | MeAla | Leu | nBuGly | MeAbu | Leu | Asp-pip | 1636.1 | AA50 | 1635.6 | [M-H]- | 0.90 |
| pd019 | D-Val | MePhe | Abu | MeVal | bMeAla | Thr | MeLeu | nBuGly | Ala | MePhe | Leu | Ala | MeVal | Asp-pip | 1596.0 | AA50 | 1595.5 | [M-H]- | 0.84 |
| pd020 | D-MeVal | MePhe | Leu | MeGly | Leu | Thr | MeLeu | MeVal | Ala | Ser(tBu) | MePhe | MeAbu | Val | Asp-pip | 1668.1 | AA50 | 1667.6 | [M-HI- | 0.88 |
| pd021 | bMeAla | Leu | MeLeu | Ile | MePhe | Abu | MePhe | Ser(tBu) | MeLeu | Thr | MeAla | MeLeu | Val | Asp-pip | 1710.1 | AA50 | 1709.7 | [M-H]- | 0.91 |
| pd022 | gMeAbu | Ala | MeLeu | Ile | Pro | Hph | MeLeu | MeAla | MePhe | Thr | Ser(tBu) | Ile | MeLeu | Asp-pip | 1708.1 | AA50 | 1707.6 | [M-H]- | 0.88 |
| pd023 | gMeAbu | Hph | MeLeu | Ile | Pro | Ala | MeLeu | Abu | MePhe | Thr | MeAla | Ile | MeLeu | Asp-pip | 1650.1 | AA50 | 1649.6 | [M-H]- | 0.86 |
| pd024 | MeAla | Leu | Thr | MePhe | bAla | MeVal | Leu | MeIle | Hph | MeGly | MeIle | bAla | MeLeu | Asp-pip | 1624.0 | AA50 | 1623.6 | [M-H]- | 0.86 |
| pd025 | MeAla | Ser(tBu) | MeVal | Ala | Ile | Pro | Ala | MePhe | MeAbu | Thr | MeLeu | MePhe | Val | Asp-pip | 1624.0 | AA50 | 1623.5 | [M-H]- | 0.85 |
| pd026 | MeAla | MeLeu | Thr | MePhe | Abu | MePhe | Ala | bMeAla | Leu | nBuGly | Ile | MeLeu | Val | Asp-pip | 1624.0 | AA50 | 1623.5 | [M-H]- | 0.85 |
| pd027 | MeAla | Leu | MeLeu | Ile | Leu | MePhe | Ala | MePhe | MeIle | Thr | MeLeu | Pro | Leu | Asp-pip | 1692.1 | AA50 | 1691.6 | [M-H]- | 0.91 |
| pd028 | MeGly | MePhe | Ile | MeLeu | MeThr | Abu | Hph | MeLeu | Abu | MeAla | Ser(tBu) | Abu | MeVal | Asp-pip | 1654.0 | AA50 | 1653.6 | [M-H]- | 0.84 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd029 | MeLeu | Ile | MeGly | Phe | Thr | MeLeu | Leu | MeVal | MeAbu | Ile | Pro | Val | MePhe | Asp-pip | 1664.1 | AA50 | 1663.6 | [M-H]- | 0.89 |
| pd030 | MeGly | MePhe | Ile | MeLeu | MeThr | Gly | Leu | MePhe | Abu | MeAla | Val | Leu | MeVal | Asp-pip | 1596.0 | AA50 | 1595.5 | [M-H]- | 0.82 |
| pd031 | MeLeu | MePhe | Ile | MeGly | Thr | MeLeu | Leu | MeVal | Abu | Ile | Pro | Ser(tBu) | MePhe | Asp-pip | 1708.1 | AA50 | 1707.6 | [M-H]- | 0.93 |
| pd032 | D-Val | MeThr | Hph | MeVal | MeLeu | Leu | MeGly | MeLeu | Val | Pro | MePhe | Leu | MeVal | Asp-pip | 1692.1 | AA50 | 1691.6 | [M-H]- | 0.88 |
| pd033 | D-MeVal | MePhe | Leu | MeVal | Abu | MeLeu | Thr | MeAla | MeLeu | Leu | MePhe | MeVal | Leu | Asp-pip | 1708.1 | AA50 | 1707.7 | [M-H]- | 0.94 |
| pd034 | bMeAla | MeLeu | Ile | MePhe | MeGly | Val | MeLeu | Ser(tBu) | MePhe | Thr | MeAla | Ile | MeLeu | Asp-pip | 1696.1 | AA50 | 1695.6 | [M-H]- | 0.89 |
| pd035 | MeAla | Leu | MeLeu | MePhe | Abu | nBuGly | Leu | MeIle | Abu | MeThr | MePhe | Ile | MeLeu | Asp-pip | 1708.1 | AA50 | 1707.7 | [M-H]- | 0.92 |
| pd036 | Ala | MeLeu | Val | MePhe | MeLeu | Ala | MePhe | Thr | MeIle | Leu | MeLeu | Pro | MeVal | Asp-pip | 1678.1 | AA50 | 1677.6 | [M-H]- | 0.89 |
| pd037 | MeAla | Leu | MeLeu | Val | bMeAla | MePhe | Ala | MePhe | MeIle | Thr | MeGly | MeLeu | Leu | Asp-pip | 1624.0 | AA50 | 1623.6 | [M-H]- | 0.82 |
| pd038 | MeGly | MePhe | Ile | bMeAla | MeThr | Abu | MeLeu | Ala | MeAbu | Hph | Pro | Ser(tBu) | MeIle | Asp-pip | 1638.0 | AA50 | 1637.5 | [M-H]- | 0.84 |
| pd039 | MeGly | MeLeu | Ile | MeLeu | Thr | MePhe | Leu | Val | MePhe | MeAla | MeVal | Gly | MeAla | Asp-pip | 1596.0 | AA50 | 1595.5 | [M-H]- | 0.82 |
| pd040 | D-Val | MeThr | Leu | MeVal | MeLeu | Hph | MeGly | MeLeu | Pro | MePhe | Val | MeAla | MeVal | Asp-pip | 1664.1 | AA50 | 1663.6 | [M-H]- | 0.87 |
| pd041 | gMeAbu | Leu | MeLeu | MeAla | MePhe | Ala | MePhe | MeAbu | MeLeu | Thr | MeAla | Ile | MeLeu | Asp-pip | 1652.1 | AA50 | 1651.6 | [M-H]- | 0.84 |
| pd042 | gMeAbu | MeLeu | MeIle | Phe | Pro | MeLeu | Ser(tBu) | MePhe | Thr | MeAbu | Pro | MeLeu | Val | Asp-pip | 1734.1 | AA50 | 1733.6 | [M-H]- | 0.91 |
| pd043 | MeAla | MeLeu | Val | MePhe | MeAbu | MePhe | Thr | MeIle | Leu | MeLeu | Pro | Ile | MeVal | Asp-pip | 1706.1 | AA50 | 1705.6 | [M-H]- | 0.92 |
| pd044 | MeAla | Leu | MePhe | MeAla | MeLeu | Ser(tBu) | MeAla | Leu | MeIle | Thr | MeGly | MeLeu | MePhe | Asp-pip | 1682.1 | AA50 | 1681.6 | [M-H]- | 0.89 |
| pd045 | MeAla | Ile | MePhe | Val | Meleu | Thr | MeGly | MePhe | MeLeu | MeAla | MeLeu | Ser(tBu) | MeAbu | Asp-pip | 1682.1 | AA50 | 1681.6 | [M-H]- | 0.88 |
| pd046 | MeGly | MePhe | Ala | MeLeu | MeThr | MeLeu | MeAla | Abu | MePhe | Val | Pro | Ala | MeVal | Asp-pip | 1566.0 | AA50 | 1565.5 | [M-H]- | 0.80 |
| pd047 | D-Val | MeThr | Meleu | MeVal | MeLeu | Hph | MeGly | MeLeu | Pro | MePhe | Val | MeAla | MeVal | Asp-pip | 1678.1 | AA50 | 1677.6 | [M-H]- | 0.87 |
| pd048 | gMeAbu | Leu | MeLeu | MeAla | MePhe | Ala | MePhe | MeAbu | MeLeu | Thr | MeAla | MeIle | MeLeu | Asp-pip | 1666.1 | AA50 | 1665.5 | [M-H]- | 0.86 |
| pd049 | D-Val | MeLeu | Leu | MeVal | MeLeu | MeGly | Thr | MePhe | Ser(tBu) | MeAbu | MeAla | Leu | MeVal | D-3-Abu | 1551.0 | AA50 | 1550.5 | [M-H]- | 0.85 |
| pd050 | gMeAbu | Val | MeAbu | Ser(tBu) | Ala | MePhe | MePhe | Ile | MeLeu | MeThr | MeAla | Ile | MeVal | D-3-Abu | 1585.0 | AA50 | 1584.4 | [M-H]- | 0.79 |
| pd051 | bMeAla | Abu | MeLeu | Ile | Pro | MePhe | MeAla | MeLeu | Hph | Ile | MeAla | Thr | MeLeu | D-3-Abu | 1553.0 | AA50 | 1552.3 | [M-H]- | 0.80 |
| pd052 | MeAla | Leu | MeAla | MeLeu | Ser(tBu) | MePhe | Val | MeLeu | MeIle | Thr | MeGly | MeLeu | Leu | D-3-Abu | 1565.1 | AA50 | 1564.4 | [M-H]- | 0.86 |
| pd053 | MeLeu | Ile | MeLeu | Abu | MeLeu | Ala | MeThr | Leu | Pro | MePhe | MeAbu | Leu | MeVal | D-3-Abu | 1547.0 | AA50 | 1546.4 | [M-H]- | 0.83 |
| pd054 | D-MeAla | Leu | MeVal | Pro | Ile | McPhe | Thr | MeLeu | MeAla | MeLeu | Ser(tBu) | MePhe | MeVal | D-3-Abu | 1625.1 | AA50 | 1624.4 | [M-H]- | 0.87 |
| pd055 | gMcAbu | MeAla | Ile | MeVal | MeAla | Abu | MePhe | Ile | MeLeu | MeThr | bMcAla | Hph | MeVal | D-3-Abu | 1541.0 | AA50 | 1540.3 | [M-H]- | 0.75 |
| pd056 | bMeAla | Hph | MeLeu | Pro | MePhe | MeAla | Leu | MeLeu | Ile | MeVal | MeAla | Thr | MeLeu | D-3-Abu | 1581.0 | AA50 | 1580.5 | [M-H]- | 0.81 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd057 | MeLeu | Ile | MeLeu | Val | MeAbu | MeLeu | MeThr | Pro | Leu | MePhe | MeAbu | Ser(tBu) | MeVal | D-3-Abu | 1613.1 | AA50 | 1618.4 | [M-H]- | 0.87 |
| pd058 | D-MeAla | Meleu | MeVal | Pro | Val | MePhe | Thr | MeLeu | MeAla | MeAbu | Ser(tBu) | MePhe | MeVal | D-3-Abu | 1597.0 | AA50 | 1596.4 | [M-H]- | 0.82 |
| pd059 | gMcAbu | MeAla | Ile | MeVal | McAla | MeAbu | MePhe | Val | Hph | MeThr | MeAla | MeLeu | MeVal | D-3-Abu | 1541.0 | AA50 | 1540.4 | [M-H]- | 0.76 |
| pd060 | bMeAla | Hph | MeLeu | Pro | MePhe | MeAla | Melle | Leu | MeLeu | MeVal | MeAla | Thr | MeLeu | D-3-Abu | 1595.0 | AA50 | 1594.3 | [M-H]- | 0.85 |
| pd061 | MeLeu | Ile | MeLeu | MeVal | MeAbu | MeLeu | MeThr | Pro | Leu | MePhe | MeAbu | Ser(tBu) | MeVal | D-3-Abu | 1633.1 | AA50 | 1632.4 | [M-H]- | 0.87 |
| pd062 | gMeAbu | MeLeu | Ile | Gly | MePhe | MePhe | bAla | MeLeu | Abu | Val | MeAla | Ile | Leu | D-3-MeAbu | 1511.0 | AA50 | 1510.3 | [M-H]- | 0.78 |
| pd063 | gMeAbu | MeLeu | Ile | Gly | MePhe | Ile | Ser(LBu) | MeLeu | Abu | Val | MePhe | MeAla | Leu | D-3-MeAbu | 1583.0 | AA50 | 1582.4 | [M-H]- | 0.82 |
| pd064 | bMeAla | Leu | MeLeu | Ile | MePhe | Hph | Melle | Val | Abu | MeLeu | Pro | Gly | MeLeu | D-3-MeAbu | 1579.1 | AA50 | 1578.4 | [M-H]- | 0.84 |
| pd065 | bMeAla | Leu | MeLeu | Val | MePhe | Hph | Melle | Ser(tBu) | MeLeu | Abu | Pro | MeLeu | MeGly | D-3-MeAbu | 1623.1 | AA50 | 1622.4 | [M-H]- | 0.87 |
| pd066 | bMeAla | MeLeu | MeLeu | Ile | MePhe | Hph | Melle | Ser(tBu) | MeLeu | Abu | Pro | MeLeu | MeGly | D-3-MeAbu | 1651.1 | AA50 | 1650.5 | [M-H]- | 0.88 |
| pd067 | MeGly | MePhe | Thr | MeLeu | Val | MeLeu | Pro | Abu | MePhe | Leu | McAla | Ser(tBu) | Val | D-3-MeAbu | 1569.0 | AA50 | 1568.4 | [M-H]- | 0.78 |
| pd068 | D-Val | MeLeu | Ser(tBu) | Val | MeLeu | Gly | MeThr | MePhe | MeAbu | MeVal | MeAla | MeVal | Abu | D-3-MeAbu | 1523.0 | AA50 | 1522.4 | [M-H]- | 0.78 |
| pd069 | Ala | MeAla | MeLeu | Ile | Melle | Val | MeLeu | MePhe | Thr | Abu | MeGly | MeAla | MeLeu | D-3-MeAbu | 1479.0 | AA50 | 1478.3 | [M-H]- | 0.77 |
| pd070 | MeGly | MePhe | Thr | MeLeu | Val | Abu | MeLeu | Pro | MePhe | Abu | MeAla | MeAbu | Val | D-3-MeAbu | 1496.9 | AA50 | 1496.3 | [M-H]-1- | 0.73 |
| pd071 | D-Val | MeLeu | MeVal | Ala | MeLeu | Gly | MeThr | MePhe | MeAbu | Ser(tBu) | MeAla | MeVal | MeAbu | D-3-MeAbu | 1509.0 | AA50 | 1508.3 | [M-H]- | 0.80 |
| pd072 | Ala | MeAla | MeLeu | Ile | MeAbu | MeVal | MeLeu | MePhe | Thr | Abu | MeGly | MeAla | MeLeu | D-3-MeAbu | 1465.0 | AA50 | 1464.3 | [M-H)- | 0.75 |
| pd073 | MeGly | MePhe | Thr | MeLeu | Val | MeAbu | MeLeu | Pro | MeAbu | Hph | MeAla | MeAbu | Val | D-3-MeAbu | 1525.0 | AA50 | 1524.4 | [M-H]- | 0.77 |
| pd074 | D-Ala | Melle | Ile | MePhe | MeAla | Val | MeLeu | MeGly | Ala | MeLeu | Val | MePhe | Val | MeAsp-pip | 1608.0 | AA50 | 1607.5 | [M-H]- | 0.81 |
| pd075 | MeAla | Leu | MeLeu | Abu | MePhe | Ile | Ala | MePhe | Melle | Gly | MeLeu | Leu | MeAla | MeAsp-pip | 1622.1 | AA50 | 1621.4 | [M-H]- | 0.85 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd076 | D-Ala | MeIle | Ile | MePhe | bMeAla | MeLeu | MeGly | Ala | MeLeu | Abu | MeAla | Val | MePhe | MeAsp-pip | 1580.0 | AA50 | 1579.3 | [M-H]- | 0.78 |
| pd077 | bMeAla | MeLeu | Ile | MePhe | nBuGly | MePhe | Ser(tBu) | MeLeu | bAla | MeAla | Ile | MeLeu | Val | MeAsp-pip | 1722.1 | AA50 | 1721.6 | [M-H]- | 0.87 |
| pd078 | MeLeu | Abu | MeVal | Pro | Val | MePhe | MeAla | MeLeu | Val | MeAbu | Ala | MeLeu | Val | MeAsp-pip | 1586.1 | AA50 | 1585.4 | [M-H]- | 0.81 |
| pd079 | MeAla | Leu | MeLeu | Ile | MePhe | Ala | MePhe | MeIle | Gly | MeLeu | MeVal | Leu | MeAla | MeAsp-pip | 1650.1 | AA50 | 1649.5 | [M-H]- | 0.87 |
| pd080 | MeGly | MeLeu | Ile | MeLeu | Val | MePhe | Leu | MePhe | MeAla | MeVal | Abu | MeAla | Gly | MeAsp-pip | 1594.0 | AA50 | 1593.4 | [M-H]- | 0.83 |
| pd081 | D-Ala | MeIle | Ile | MePhe | bMeAla | MeLeu | MeGly | Ala | MeLeu | MeAbu | bMeAla | Val | MePhe | MeAsp-pip | 1594.0 | AA50 | 1593.3 | (M-H ]- | 0.75 |
| pd082 | bMeAla | MeLeu | Ile | MePhe | nBuGly | MePhe | Ser(tBu) | MeLeu | MeAbu | MeAla | Ile | MeVal | Val | MeAsp-pip | 1736.2 | AA50 | 1735.7 | [M-H]- | 0.88 |
| pd083 | MeLeu | Ser(tBu) | MeVal | bMeAla | Ile | MePhe | MeGly | MeLeu | MeAbu | Pro | Ala | MeLeu | Abu | MeAsp-pip | 1616.1 | AA50 | 1615.5 | [M-H]- | 0.78 |
| pd084 | MeAla | MeLeu | MeLeu | Ile | MePhe | Ala | MePhe | MeIle | Gly | MeLeu | MeVal | Leu | MeAla | MeAsp-pip | 1664.1 | AA50 | 1663.6 | [M-H]- | 0.88 |
| pd085 | MeGly | MeLeu | Ile | MeLeu | Val | MePhe | Leu | MePhe | MeAla | MeVal | MeAbu | MeAla | Gly | MeAsp-pip | 1608.0 | AA50 | 1607.6 | [M-H]- | 0.83 |
| pd086 | D-Val | MePhe | MeVal | Thr | MeLeu | Leu | MeGly | Val | MeLeu | Ser(tBu) | Ala | MePhe | MeAla | MeAsp-pip | 1668.1 | AA50 | 1667.5 | [M-H]- | 0.83 |
| pd087 | MeLeu | Ser(tBu) | MeVal | Ile | Pro | Val | nBuGly | MeLeu | Thr | MeAla | Pro | Hph | Ile | MeAsp-pip | 1686.1 | AA50 | 1685.6 | [M-H]- | 0.85 |
| pd088 | MeGly | Ile | MeLeu | Hph | bMeAla | MeAla | Val | MeVal | Abu | MePhe | Thr | MeLeu | Leu | MeAsp-pip | 1638.1 | AA50 | 1637.5 | [M-H]- | 0.82 |
| pd089 | D-MeVal | MeLeu | Hph | MeVal | MeLeu | Thr | MeAla | MeLeu | Ser(tBu) | MePhe | Abu | MeVal | Leu | MeAsp-pip | 1766.2 | AA50 | 1765.8 | [M-H]- | 0.91 |
| pd090 | D-Val | MePhe | Ala | MeVal | Thr | MeLeu | Abu | MeGly | MeLeu | Ser(tBu) | MeAla | MePhe | MeGly | MeAsp-pip | 1612.0 | AA50 | 1611.5 | [M-H]- | 0.80 |
| pd091 | MeAla | Leu | MeLeu | Ser(tBu) | MePhe | Abu | MeGly | Leu | MeAbu | MeThr | MePhe | Ile | MeLeu | MeAsp-pip | 1710.1 | AA50 | 1709.7 | [M-H]- | 0.84 |
| pd092 | MeGly | Ile | MeLeu | Hph | MeLeu | MeAla | MeVal | Leu | MeLeu | Thr | MeLeu | MePhe | Val | MeAsp-oip | 1722.1 | AA50 | 1721.6 | [M-H]- | 0.90 |
| pd093 | D-MeVal | MeLeu | Hph | MeVal | MeLeu | Thr | MeAla | MeLeu | Ser(tBu) | MePhe | MeAbu | MeVal | Leu | MeAsp-pip | 1780.2 | AA50 | 1779.9 | [M-H]- | 0.92 |
| pd094 | D-Val | MePhe | bAla | MeVal | Thr | MeLeu | MeAbu | MeGly | MeLeu | Ser(tBu) | MeAla | MePhe | MeGly | MeAsp-pip | 1626.0 | AA50 | 1625.3 | [M-H]- | 0.80 |
| pd095 | MeAla | Leu | MeLeu | Ser(tBu) | MePhe | MeAbu | MeGly | Leu | MeIle | MeThr | MePhe | Ile | MeLeu | MeAsp-pip | 1752.2 | AA50 | 1751.7 | [M-H]- | 0.87 |
| pd096 | MeGly | MeIle | MeLeu | Hph | MeLeu | bMeAla | MeVal | Leu | MeLeu | Thr | bMeAla | MePhe | Val | MeAsp-pip | 1694.1 | AA50 | 1693.6 | [M-H]- | 0.81 |
| pd097 | D-Val | MeLeu | Ala | MeVal | Pro | Leu | MeGly | MeLeu | Ser(tBu) | Leu | MePhe | MeVal | Asp-pip | | 1531.0 | AA50 | 1530.6 | [M-H]- | 0.87 |
| pd098 | qMeAbu | MeLeu | MeIle | Phe | Pro | MeLeu | Ser(tBu) | MeLeu | Ala | Ile | MeAbu | MePhe | Asp-pip | | 1621.1 | AA50 | 1620.6 | [M-H]- | 0.91 |
| pd099 | MeAla | Ile | MePhe | MeLeu | Val | MeGly | Leu | MePhe | MeAla | Leu | nBuGly | MeAbu | Asp-pip | | 1523.0 | AA50 | 1522.5 | [M-H]- | 0.87 |
| pd100 | D-Val | MePhe | Leu | MeVal | MeLeu | Thr | MeLeu | nBuGly | Ser(tBu) | MePhe | Leu | MeVal | Asp-pip | | 1667.1 | AA50 | 1666.7 | [M-H]- | 0.96 |
| pd101 | bMeAla | Leu | MeLeu | Ile | MePhe | Abu | MePhe | Val | MeLeu | Thr | MeAla | MeLeu | Asp-pip | | 1567.0 | AA50 | 1566.6 | [M-H]- | 0.88 |
| pd102 | gMeAbu | MeLeu | Ile | Pro | Hph | MeLeu | Ser(tBu) | MePhe | Thr | MeAla | Ile | MeLeu | Asp-pip | | 1637.1 | AA50 | 1636.6 | [M-H]- | 0.88 |
| pd103 | MeAla | MeLeu | Thr | MePhe | Abu | MePhe | Leu | MeIle | Leu | nBuGly | Ile | MeLeu | Asp-pip | | 1609.1 | AA50 | 1608.6 | [M-H]- | 0.95 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd104 | MeAla | Leu | MeLeu | Ile | MePhe | Ala | MePhe | MeIle | Thr | MeLeu | Pro | Leu | Asp-pip | | 1579.0 | AA50 | 1578.6 | [M-H]- | 0.90 |
| pd105 | MeGly | MePhe | Ile | MeLeu | MeThr | Gly | Leu | MePhe | Abu | MeAla | Leu | MeVal | Asp-pip | | 1496.9 | AA50 | 1496.4 | [M-H]- | 0.81 |
| pd106 | MeLeu | MePhe | Ile | MeGly | Thr | MeLeu | Leu | MeVal | Abu | Pro | Ser(tBu) | MePhe | Asp-pip | | 1595.0 | AA50 | 1594.5 | [M-H]- | 0.94 |
| pd107 | D-Val | MeThr | Hph | MeVal | MeLeu | Leu | MeGly | MeLeu | Pro | MePhe | Leu | MeVal | Asp-pip | | 1593.0 | AA50 | 1592.5 | [M-H]- | 0.87 |
| pd108 | D-MeVal | MePhe | Leu | MeVal | MeLeu | Thr | MeAla | MeLeu | Leu | MePhe | MeVal | Leu | Asp-pip | | 1623.1 | AA50 | 1622.6 | [M-H]- | 0.91 |
| pd109 | gMeAbu | Leu | MeLeu | MeAla | MePhe | Abu | MePhe | Ser(tBu) | MeLeu | Thr | MeAla | MeLeu | Asp-pip | | 1597.0 | AA50 | 1596.6 | [M-H]- | 0.86 |
| pd110 | bMeAla | MeLeu | Ile | MePhe | MeGly | MeLeu | Ser(tBu) | MePhe | Thr | MeAla | Ile | MeLeu | Asp-pip | | 1597.0 | AA50 | 1596.6 | [M-H]- | 0.88 |
| pd111 | MeAla | Leu | MeLeu | MePhe | Abu | nBuGly | Leu | MeIle | MeThr | MePhe | Ile | MeLeu | Asp-pip | | 1623.1 | AA50 | 1622.6 | [M-H]- | 0.92 |
| pd112 | Ala | MeLeu | MePhe | MeLeu | Abu | MePhe | Thr | MeIle | Leu | bMeAla | Pro | MeVal | Asp-pip | | 1551.0 | AA50 | 1550.5 | [M-H]- | 0.83 |
| pd113 | MeAla | Leu | MeLeu | bMeAla | MePhe | bAla | MePhe | MeIle | Thr | MeGly | MeLeu | Leu | Asp-pip | | 1525.0 | AA50 | 1524.5 | [M-H]- | 0.82 |
| pd114 | MeGly | MePhe | Ile | MeLeu | MeThr | MeLeu | Leu | MeAbu | Hph | Pro | Ser(tBu) | MeIle | Asp-pip | | 1637.1 | AA50 | 1636.6 | [M-H]- | 0.90 |
| pd115 | MeGly | MeLeu | Ile | MeLeu | Thr | MePhe | Leu | MePhe | MeAla | MeVal | Gly | MeAla | Asp-pip | | 1496.9 | AA50 | 1496.5 | [M-H]- | 0.83 |
| pd116 | D-Val | MeThr | Leu | MeVal | MeLeu | Hph | MeGly | MeLeu | Pro | MePhe | MeAla | MeVal | Asp-pip | | 1565.0 | AA50 | 1564.5 | [M-H]- | 0.81 |
| pd117 | gMeAbu | Leu | MeLeu | McAla | MePhe | Ala | MePhe | MeAbu | MeLeu | Thr | MeAla | MeLeu | Asp-pip | | 1539.0 | AA50 | 1538.5 | [M-H)- | 0.83 |
| pd118 | gMeAbu | MeLeu | MeIle | Phe | Pro | MeLeu | Ser(tBu) | MePhe | Thr | MeAbu | Pro | MeLeu | Asp-pip | | 1635.0 | AA50 | 1634.6 | [M-H]- | 0.88 |
| pd119 | MeAla | MeLeu | Ser(tBu) | MePhe | MeAbu | MePhe | Thr | MeIle | Leu | MeLeu | Pro | MeVal | Asp-pip | | 1637.1 | AA50 | 1636.6 | [M-H]- | 0.89 |
| pd120 | MeAla | Leu | MePhe | bMeAla | MeLeu | bMeAla | Leu | MeIle | Thr | MeGly | MeLeu | MePhe | Asp-pip | | 1539.0 | AA50 | 1538.5 | [M-H]- | 0.83 |
| pd121 | MeAla | Ile | MePhe | MeLeu | Thr | MeGly | MePhe | MeLeu | MeAla | MeLeu | Ser(tBu) | MeAbu | Asp-pip | | 1583.0 | AA50 | 1582.5 | [M-H]- | 0.86 |
| pd122 | MeGly | MePhe | Abu | MeLeu | MeThr | MeLeu | MeAla | MePhe | Val | Pro | Abu | MeVal | Asp-pip | | 1508.9 | AA50 | 1508.4 | [M-H]- | 0.81 |
| pd123 | D-MeVal | MePhe | Pro | MeVal | MeLeu | Thr | nBuGly | MeLeu | Ser(tBu) | MePhe | MeAla | MeVal | Asp-pip | | 1637.1 | AA50 | 1636.6 | [M-H]- | 0.91 |
| pd124 | gMeAbu | MeLeu | MeIle | Pro | MeVal | MePhe | MeAla | MeLeu | Thr | MeAla | Hph | MeLeu | Asp-pip | | 1593.0 | AA50 | 1592.5 | [M-H]- | 0.83 |
| pd125 | MeAla | Leu | MeLeu | MeAla | Pro | Hph | MeLeu | MeIle | MeThr | MeAbu | MeLeu | Pro | Asp-pip | | 1543.0 | AA50 | 1542.5 | [M-H]- | 0.81 |
| pd126 | MeLeu | MePhe | MeAbu | MeGly | Thr | MeLeu | Leu | MePhe | MeVal | Pro | MeAla | MeVal | Asp-pip | | 1551.0 | AA50 | 1550.5 | [M-H]- | 0.84 |
| pd127 | MeAla | Leu | MeLeu | Ile | MePhe | Ala | MeLeu | MeIle | Val | MeGly | MeLeu | Leu | D-3-Abu | | 1420.0 | AA50 | 1419.5 | [M-H]- | 0.90 |
| pd128 | D-A a | MeAla | MePhe | Val | MeLeu | Thr | MeGly | Abu | MeLeu | Ser(tBu) | MePhe | MeVal | D-3-Abu | | 1429.9 | AA50 | 1429.4 | [M-H]- | 0.79 |
| pd129 | MeGly | Ile | MeLeu | Abu | MeLeu | Thr | MeGly | Leu | MePhe | MeLeu | Ser(tBu) | MeVal | D-3-Abu | | 1438.0 | AA50 | 1437.5 | [M-H]- | 0.85 |
| pd130 | D-MeAla | Val | MePhe | Ile | MeLeu | Thr | Pro | MeAla | MeLeu | Ser(tBu) | MePhe | MeVal | D-3-Abu | | 1498.0 | AA50 | 1497.4 | [M-H]- | 0.86 |
| pd131 | D-Val | MeLeu | Leu | MeVal | MeLeu | MeGly | Thr | MePhe | Ser(tBu) | MeAbu | MeAla | MeVal | D-3-Abu | | 1438.0 | AA50 | 1437.4 | [M-H]- | 0.86 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd132 | gMeAbu | Ile | MeAbu | Ala | MePhe | MePhe | Val | MeLeu | MeThr | MeAla | Ile | MeVal | D-3-Abu | | 1441.9 | AA50 | 1441.4 | [M-H]- | 0.81 |
| pd133 | bMeAla | MeLeu | Ile | Pro | MePhe | MeAla | MeLeu | Hph | Ile | MeAla | Thr | MeLeu | D-3-Abu | | 1467.9 | AA50 | 1467.4 | [M-H]- | 0.83 |
| pd134 | MeAla | MeAla | MeLeu | Ile | MePhe | Val | MeLeu | MeIle | Thr | MeGly | MeLeu | Leu | D-3-Abu | | 1422.0 | AA50 | 1421.5 | [M-H]- | 0.84 |
| pd135 | MeLeu | Ile | MeLeu | Abu | MeLeu | MeThr | Leu | Pro | MePhe | MeAbu | Leu | MeVal | D-3-Abu | | 1476.0 | AA50 | 1475.5 | [M-H]- | 0.88 |
| pd136 | D-MeAla | MeVal | Pro | Ile | MePhe | Thr | MeLeu | MeAla | MeLeu | Ser(tBu) | MePhe | MeVal | D-3-Abu | | 1512.0 | AA50 | 1511.5 | [M-H]- | 0.88 |
| pd137 | gMeAbu | MeAla | Ile | MeVal | MeAla | MePhe | Val | MeLeu | MeThr | MeAla | Hph | MeVal | D-3-Abu | | 1441.9 | AA50 | 1441.4 | [M-H]- | 0.78 |
| pd138 | bMeAla | Hph | MeLeu | Pro | MePhe | MeAla | MeLeu | Ile | MeVal | bMeAla | Thr | MeLeu | D-3-Abu | | 1467.9 | AA50 | 1467.5 | [M-H]- | 0.81 |
| pd139 | MeLeu | Ile | MeLeu | MeAbu | MeLeu | MeThr | Pro | Leu | MePhe | MeAbu | Ser(tBu) | MeVal | D-3-Abu | | 1520.0 | AA50 | 1519.5 | [M-H]- | 0.88 |
| pd140 | MeGly | MeVal | MeLeu | MeAla | MeLeu | Thr | MeGly | MeAbu | MePhe | MeLeu | Leu | MeVal | D-3-Abu | | 1393.9 | AA50 | 1393.4 | [M-H]- | 0.79 |
| pd141 | bMeAla | Leu | MeLeu | Ile | MePhe | Hph | MeIle | bAla | MeLeu | Pro | Gly | MeLeu | D-3-MeAbu | | 1466.0 | AA50 | 1465.5 | [M-H]- | 0.87 |
| pd142 | bMeAla | Leu | MeLeu | Ile | MePhe | Hph | MeIle | Ser(tBu) | MeLeu | Pro | MeLeu | MeGly | D-3-MeAbu | | 1552.0 | AA50 | 1551.6 | [M-H]- | 0.92 |
| pd143 | gMeAbu | MeLeu | Ile | MeGly | MePhe | MePhe | Ser(tBu) | MeLeu | MeAla | MeVal | MeAbu | Leu | D-3-MeAbu | | 1512.0 | AA50 | 1511.5 | [M-H]- | 0.89 |
| pd144 | MeLeu | bMeAla | MeLeu | Ser(tBu) | MePhe | bMeAla | MePhe | MeIle | Leu | MeGly | MeLeu | Leu | D-3-MeAbu | | 1540.0 | AA50 | 1539.5 | [M-H]- | 0.89 |
| pd145 | gMeAbu | MeLeu | MePhe | Gly | Ile | MePhe | Ser(tBu) | MeLeu | Thr | MeAla | Ile | Leu | D-3-MeAbu | | 1500.0 | AA50 | 1499.5 | [M-H]- | 0.86 |
| pd146 | MeGly | MePhe | Thr | MeLeu | Val | MeLeu | Pro | MePhe | Abu | MeAla | Ser(tBu) | Val | D-3-MeAbu | | 1455.9 | AA50 | 1455.4 | [M-H]- | 0.80 |
| pd147 | D-Val | MeLeu | Ala | MeVal | MeLeu | Gly | MeThr | MePhe | MeAbu | Ser(tBu) | MeVal | MeAla | D-3-MeAbu | | 1409.9 | AA50 | 1409.4 | [M-H]- | 0.81 |
| pd148 | Ala | MeAla | MeLeu | Ile | MeIle | Val | MeLeu | MePhe | Thr | MeGly | MeAla | MeLeu | D-3-MeAbu | | 1393.9 | AA50 | 1393.4 | [M-H]- | 0.82 |
| pd149 | MeGly | MePhe | Thr | MeLeu | Val | MeLeu | Pro | MePhe | Abu | MeAla | MeAbu | Val | D-3-MeAbu | | 1411.9 | AA50 | 1411.4 | [M-H]- | 0.76 |
| pd150 | D-MeAla | MeAla | MePhe | Val | MeLeu | MeThr | MeGly | Ala | MeLeu | Ser(tBu) | MePhe | MeAbu | D-3-MeAbu | | 1443.9 | AA50 | 1443.4 | [M-H]- | 0.79 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd151 | D-Ala | MeIle | Ile | MePhe | MeAla | MeLeu | MeGly | Ala | MeLeu | Ser(tBu) | MePhe | Val | MeAsp-pip | | 1553.0 | AA50 | 1552.5 | [M-H]- | 0.86 |
| pd152 | MeAla | Leu | MeLeu | MePhe | Ile | Ala | MePhe | MeIle | Gly | MeLeu | Leu | MeAla | MeAsp-pip | | 1537.0 | AA50 | 1536.5 | [M-H]- | 0.86 |
| pd153 | D-Ala | MeIle | Ile | MePhe | MeAla | MeLeu | MeGly | Ala | MeLeu | MeAla | Val | MePhe | MeAsp-pip | | 1495.0 | AA50 | 1494.4 | [M-H]- | 0.81 |
| pd154 | bMeAla | MeLeu | Ile | MePhe | nBuGly | MePhe | Ser(tBu) | MeLeu | bAla | MeAla | MeIle | Leu | MeAsp-pip | | 1623.1 | AA50 | 1622.6 | [M-H]- | 0.90 |
| pd155 | MeAla | Leu | MeLeu | Ser(tBu) | MePhe | Ala | MePhe | MeIle | Gly | MeLeu | MeVal | MeAla | MeAsp-pip | | 1567.0 | AA50 | 1566.5 | [M-H]- | 0.90 |
| pd156 | MeGly | MeLeu | Ile | MeLeu | Val | MePhe | Leu | MePhe | MeAla | MeVal | MeAla | Gly | MeAsp-pip | | 1509.0 | AA50 | 1508.5 | [M-H]- | 0.86 |
| pd157 | D-Val | MeLeu | Val | MeVal | MeLeu | MeVal | MeGly | MeLeu | MeAla | MePhe | Leu | MeAla | MeAsp-pip | | 1517.0 | AA50 | 1516.5 | [M-H]- | 0.87 |
| pd158 | D-Val | MePhe | MeVal | Thr | MeLeu | Leu | MeGly | MeLeu | Ser(tBu) | Ala | MePhe | MeAla | MeAsp-pip | | 1569.0 | AA50 | 1568.5 | [M-H]- | 0.84 |
| pd159 | MeLeu | Ser(tBu) | MeVal | Ile | Pro | nBuGly | MeLeu | Thr | MeLeu | Pro | Hph | Ile | MeAsp-pip | | 1629.1 | AA50 | 1628.6 | [M-H]- | 0.89 |
| pd160 | MeGly | Ile | MeLeu | Hph | MeLeu | MeAla | MeVal | Leu | MePhe | Thr | MeLeu | Leu | MeAsp-pip | | 1609.1 | AA50 | 1608.5 | [M-H]- | 0.92 |
| pd161 | D-MeVal | MeLeu | Hph | MeVal | MeLeu | Thr | MeAla | MeLeu | Ser(tBu) | MePhe | MeVal | Leu | MeAsp-pip | | 1681.1 | AA50 | 1680.7 | [M-H]- | 0.94 |
| pd162 | D-Val | MePhe | MeVal | Thr | MeLeu | Abu | MeGly | MeLeu | Ser(tBu) | MeAla | MePhe | MeGly | MeAsp-pip | | 1541.0 | AA50 | 1540.5 | [M-H]- | 0.83 |
| pd163 | MeAla | Leu | MeLeu | MePhe | Abu | MeGly | Leu | MeIle | MeThr | McPhe | Ile | MeLeu | MeAsp-pip | | 1595.0 | AA50 | 1594.5 | [M-H]- | 0.84 |
| pd164 | MeLeu | MeVal | Pro | Ile | MePhe | MeGly | MeLeu | Thr | Pro | Ala | MeLeu | Leu | MeAsp-pip | | 1515.0 | AA50 | 1514.5 | [M-H]- | 0.81 |
| pd165 | MeGly | Ile | MeLeu | Hph | MeLeu | MeAla | MeVal | Leu | MeLeu | Thr | MeLeu | MePhe | MeAsp-pip | | 1623.1 | AA50 | 1622.6 | [M-H]- | 0.94 |
| pd166 | bMeAla | Leu | MeLeu | Ile | MePhe | MeLeu | MePhe | Ser(tBu) | MeLeu | MeThr | MeAla | MeLeu | MeAsp-pip | | 1681.1 | AA50 | 1680.7 | [M-H]- | 0.94 |

(continued)

| ID | 1 | 2 | 3 | 4 | 5 | 6 | 7 | 8 | 9 | 10 | 11 | 12 | 13 | 14 | exact mass | LCMS cond. | obs. mass (m/z) | ion form | Rt (min) |
|---|---|---|---|---|---|---|---|---|---|---|---|---|---|---|---|---|---|---|---|
| pd167 | MeAla | MeLeu | Thr | MePhe | Abu | MeIle | MeLeu | MePhe | Leu | MeGly | MeIle | MeLeu | MeAsp-pip | | 1609.1 | AA50 | 1608.6 | [M-H]- | 0.91 |
| pd168 | MeGly | MePhe | Ile | MeLeu | MeThr | nBuGly | MePhe | Leu | MeVal | MeAla | Ser(tBu) | MeAbu | MeAsp-pip | | 1611.0 | AA50 | 1610.5 | [M-H]- | 0.87 |

[Table 5]

| Analysis conditions | AA50 |
|---|---|
| Device | Acquity UPLC/SQD2 |
| Column (I.D. x length) (mm) | Ascentis Express C18 (2.1x50) |
| Mobile phase | A) 10mM AA,water<br>B) methanol |
| Gradient (A/B) | 50/50 (initial) → 0/100 (0.7 min) → 0/100 (0.7 min) |
| Flow rate (mL/min) | 0.9 |
| Column temperature (°C) | 35 |
| Wavelength | 210-400nm<br>PDA total |

Example 3. Metabolic stability test of cyclic peptide compounds

The metabolic stability of the cyclic peptide compounds consisting of 13 or 14 amino acid residues synthesized in Example 2 was evaluated using a liver microsomal stability assay.

Under the presence and absence of NADPH, a microsome solution prepared to a concentration of 0.5 mg protein/mL using 100 mM phosphate buffer (pH 7.4) with mouse liver microsomes (male, Corning Incorporated, USA) and the compound at a concentration of 1 μM were subjected to a metabolic reaction for 22 minutes, 44 minutes, or 66 minutes. Protein removal processing was performed over time using acetonitrile, and the remaining amount of unchanged compound was analyzed using LCMS to calculate the intrinsic hepatic clearance (CLh, int) (Table 6).

[Table 6]

| ID | Mouse Hepatic Microsome CLh,int (μL/min/mg Protein) | |
|---|---|---|
| | NADPH(+) | NADPH(-) |
| pd001 | 34.2 | 29.6 |
| pd002 | 34.2 | 30.1 |
| pd003 | 17.9 | 19.0 |
| pd004 | 36.2 | 27.4 |
| pd005 | 1.4 | 1.1 |
| pd006 | 51.4 | 30.0 |
| pd007 | 40.8 | 34.9 |
| pd008 | 13.4 | 11.8 |
| pd009 | 38.9 | 42.4 |
| pd010 | 59.7 | 27.8 |
| pd011 | 27.3 | 22.9 |
| pd012 | 41.3 | 20.1 |
| pd013 | 12.1 | 6.1 |
| pd014 | 31.1 | 33.0 |
| pd015 | 32.5 | 26.2 |
| pd016 | 49.9 | 32.9 |
| pd017 | 39.4 | 40.1 |
| pd018 | 42.3 | 34.1 |
| pd019 | 34.7 | 24.9 |
| pd020 | 33.3 | 24.9 |

(continued)

| ID | Mouse Hepatic Microsome CLh,int (μL/min/mg Protein) | |
|---|---|---|
| | NADPH(+) | NADPH(-) |
| pd021 | 37.1 | 36.3 |
| pd022 | 44.4 | 36.4 |
| pd023 | 43.3 | 26.2 |
| pd024 | 16.6 | 20.0 |
| pd025 | 14.8 | 15.8 |
| pd026 | 63.5 | 29.9 |
| pd027 | 42.4 | 31.1 |
| pd028 | 37.2 | 22.1 |
| pd029 | 38.2 | 36.6 |
| pd030 | 33.7 | 28.5 |
| pd031 | 40.3 | 32.3 |
| pd032 | 28.9 | 28.7 |
| pd033 | 47.8 | 34.1 |
| pd034 | 46.8 | 36.0 |
| pd035 | 28.3 | 27.5 |
| pd036 | 35.4 | 25.7 |
| pd037 | 18.3 | 17.7 |
| pd038 | 12.0 | 18.1 |
| pd039 | 21.4 | 15.6 |
| pd040 | 24.8 | 22.3 |
| pd041 | 25.1 | 23.5 |
| pd042 | 32.3 | 18.1 |
| pd043 | 40.7 | 34.8 |
| pd044 | 54.3 | 33.1 |
| pd045 | 43.9 | 26.4 |
| pd046 | 6.2 | 5.4 |
| pd047 | 41.4 | 30.0 |
| pd048 | 30.2 | 20.7 |
| pd049 | 30.6 | 29.1 |
| pd050 | 13.2 | 15.9 |
| pd051 | 24.6 | 28.8 |
| pd052 | 28.0 | 32.0 |
| pd053 | 45.7 | 24.1 |
| pd054 | 32.5 | 25.1 |
| pd055 | 27.1 | 15.0 |
| pd056 | 55.9 | 30.2 |
| pd057 | 50.4 | 44.8 |
| pd058 | 16.3 | 14.8 |

(continued)

| ID | Mouse Hepatic Microsome CLh,int (μL/min/mg Protein) | |
|---|---|---|
| | NADPH(+) | NADPH(-) |
| pd059 | 12.7 | 18.6 |
| pd060 | 32.4 | 38.6 |
| pd061 | 39.0 | 39.7 |
| pd062 | 39.0 | 24.1 |
| pd063 | 47.5 | 33.7 |
| pd064 | 41.4 | 43.3 |
| pd065 | 41.7 | 40.2 |
| pd066 | 38.9 | 36.4 |
| pd067 | 17.0 | 10.8 |
| pd068 | 15.7 | 12.5 |
| pd069 | 16.5 | 20.5 |
| pd070 | 15.0 | 14.6 |
| pd071 | 13.3 | 6.2 |
| pd072 | 22.4 | 16.6 |
| pd073 | 24.3 | 2.9 |
| pd074 | 26.9 | 26.9 |
| pd075 | 35.6 | 42.8 |
| pd076 | 10.9 | 18.2 |
| pd077 | 40.0 | 40.3 |
| pd078 | 28.3 | 22.9 |
| pd079 | 26.1 | 38.4 |
| pd080 | 29.3 | 27.8 |
| pd081 | 18.4 | 5.7 |
| pd082 | 32.6 | 38.5 |
| pd083 | 11.4 | 7.3 |
| pd084 | 39.0 | 37.8 |
| pd085 | 26.9 | 28.9 |
| pd086 | 20.1 | 20.0 |
| pd087 | 31.8 | 29.0 |
| pd088 | 20.9 | 24.2 |
| pd089 | 29.0 | 29.8 |
| pd090 | 45.0 | 23.9 |
| pd091 | 39.4 | 36.1 |
| pd092 | 22.0 | 26.3 |
| pd093 | 26.8 | 20.4 |
| pd094 | 22.5 | 16.7 |
| pd095 | 25.6 | 29.2 |
| pd096 | 18.8 | 18.2 |

(continued)

| ID | Mouse Hepatic Microsome CLh,int (μL/min/mg Protein) | |
|---|---|---|
| | NADPH(+) | NADPH(-) |
| pd097 | 20.4 | 18.8 |
| pd098 | 44.5 | 37.8 |
| pd099 | 30.3 | 27.8 |
| pd100 | 39.6 | 28.4 |
| pd101 | 41.1 | 31.5 |
| pd102 | 32.9 | 25.1 |
| pd103 | 25.5 | 21.3 |
| pd104 | 36.0 | 36.2 |
| pd105 | 28.9 | 22.7 |
| pd106 | 32.7 | 37.4 |
| pd107 | 36.3 | 31.5 |
| pd108 | 39.9 | 35.5 |
| pd109 | 40.6 | 30.3 |
| pd110 | 38.5 | 30.5 |
| pd111 | 18.7 | 28.4 |
| pd112 | 30.5 | 21.2 |
| pd113 | 24.4 | 16.4 |
| pd114 | 35.6 | 29.2 |
| pd115 | 24.9 | 12.3 |
| pd116 | 19.2 | 14.9 |
| pd117 | 25.9 | 14.4 |
| pd118 | 31.2 | 19.8 |
| pd119 | 41.2 | 34.2 |
| pd120 | 25.8 | 18.4 |
| pd121 | 21.9 | 18.2 |
| pd122 | 11.1 | 9.6 |
| pd123 | 31.0 | 36.0 |
| pd124 | 16.9 | 16.6 |
| pd125 | 16.7 | 18.1 |
| pd126 | 23.3 | 18.2 |
| pd127 | 41.6 | 28.0 |
| pd128 | 18.0 | 11.3 |
| pd129 | 76.5 | 19.7 |
| pd130 | 22.7 | 6.8 |
| pd131 | 17.8 | 13.5 |
| pd132 | 16.2 | 7.7 |
| pd133 | 25.6 | 23.6 |
| pd134 | 38.5 | 11.4 |

(continued)

| ID | Mouse Hepatic Microsome CLh,int (μL/min/mg Protein) | |
|---|---|---|
| | NADPH(+) | NADPH(-) |
| pd135 | 39.5 | 28.4 |
| pd136 | 27.2 | 28.8 |
| pd137 | 14.1 | 7.5 |
| pd138 | 39.3 | 11.1 |
| pd139 | 32.3 | 28.2 |
| pd140 | 10.5 | 10.3 |
| pd141 | 31.8 | 21.1 |
| pd142 | 31.7 | 33.9 |
| pd143 | 39.3 | 21.0 |
| pd144 | 41.0 | 34.0 |
| pd145 | 35.5 | 35.7 |
| pd146 | 20.3 | 14.1 |
| pd147 | 13.1 | 14.8 |
| pd148 | 25.2 | 16.8 |
| pd149 | 10.8 | 1.5 |
| pd150 | 15.2 | 4.2 |
| pd151 | 29.6 | 22.4 |
| pd152 | 40.2 | 32.5 |
| pd153 | 15.1 | 26.0 |
| pd154 | 41.7 | 34.3 |
| pd155 | 48.8 | 37.2 |
| pd156 | 34.1 | 23.7 |
| pd157 | 31.7 | 24.9 |
| pd158 | 19.3 | 13.7 |
| pd159 | 49.5 | 42.0 |
| pd160 | 21.5 | 22.3 |
| pd161 | 35.3 | 37.0 |
| pd162 | 12.5 | 16.3 |
| pd163 | 28.2 | 36.5 |
| pd164 | 14.6 | 10.0 |
| pd165 | 40.9 | 27.2 |
| pd166 | 32.4 | 34.0 |
| pd167 | 35.9 | 33.0 |
| pd168 | 21.9 | 21.8 |

It was shown that all tested cyclic peptide compounds consisting of 13 or 14 amino acid residues have high metabolic stability.

Example 4. Membrane permeability test of cyclic peptide compounds

The membrane permeability of the cyclic peptide compounds consisting of 13 or 14 amino acid residues synthesized in Example 2 was evaluated according to the method for measuring membrane permeability using Caco-2 cells disclosed in WO 2018/225864 shown below.

Using a mixed medium of FaSSIF containing 1% DMSO and DMEM as the apical medium, and DMEM as the basal medium, Caco-2 cells and peptide compounds are preincubated for 20 to 24 hours under conditions of 5% $CO_2$, 37°C, and 80 rpm. Afterward, the culture medium on the Apical side and the Basal side are aspirated and washed, the peptide compound was added to a FaSSIF/HBSS buffer solution containing 1% DMSO (1% DMSO) (pH 6.0) at the Apical side, a HBSS buffer solution containing 4% BSA (pH 7.4) was added to the Basal side, and measurement of membrane permeability is started. Each well is shaken under 5% $CO_2$ at 37°C and 80 rpm, and at 180 minutes after the start of shaking, a sample on the Basal side is collected. The amount of peptide compounds in the sample was measured using LCMS, and the membrane permeability coefficient was calculated from the permeation amount (Table 7). When calculating the membrane permeability coefficient ($P_{app}$) from the permeation amount, the following expression can be used.

[Expression 2]

$$P_{app} = \frac{dQ}{dt} \times \frac{1}{C_1(0) \times S}$$

dQ/dt represents the permeation rate of drug (the amount of drug appearing on the Basal side per unit time), C1(0) represents the concentration of drug added on the Apical side, and S represents the total surface area of cells.

[Table 7]

| ID | Caco2 $P_{app}$ ($10^{-6}$ cm/sec) |
|---|---|
| pd001 | 0.94 |
| pd002 | 0.39 |
| pd003 | 0.27 |
| pd004 | 0.82 |
| pd005 | 0.05 |
| pd006 | <0.11 |
| pd007 | 0.11 |
| pd008 | 0.04 |
| pd009 | 0.13 |
| pd010 | 0.72 |
| pd011 | 0.11 |
| pd012 | 4.30 |
| pd013 | 0.04 |
| pd014 | <0.11 |
| pd015 | 0.04 |
| pd016 | 0.47 |
| pd017 | 0.09 |
| pd018 | 0.11 |
| pd019 | <0.11 |
| pd020 | <0.11 |
| pd021 | 0.15 |
| pd022 | 0.15 |

(continued)

| ID | Caco2 $P_{app}$ ($10^{-6}$ cm/sec) |
|---|---|
| pd023 | 0.03 |
| pd024 | 0.30 |
| pd025 | 0.05 |
| pd026 | 0.58 |
| pd027 | 0.02 |
| pd028 | 0.03 |
| pd029 | 0.02 |
| pd030 | 0.04 |
| pd031 | 0.04 |
| pd032 | 0.72 |
| pd033 | 0.03 |
| pd034 | 0.27 |
| pd035 | <0.11 |
| pd036 | 0.28 |
| pd037 | 0.26 |
| pd038 | 0.04 |
| pd039 | 0.03 |
| pd040 | 0.17 |
| pd041 | 0.13 |
| pd042 | 0.59 |
| pd043 | 0.16 |
| pd044 | 0.83 |
| pd045 | 0.32 |
| pd046 | 0.06 |
| pd047 | 0.16 |
| pd048 | 0.28 |
| pd049 | 0.32 |
| pd050 | 0.54 |
| pd051 | 0.21 |
| pd052 | 1.01 |
| pd053 | 1.64 |
| pd054 | 0.49 |
| pd055 | 0.12 |
| pd056 | 0.27 |
| pd057 | 1.16 |
| pd058 | 0.72 |
| pd059 | 0.52 |
| pd060 | 0.63 |
| pd061 | 0.51 |

(continued)

| ID | Caco2 $P_{app}$ ($10^{-6}$ cm/sec) |
|---|---|
| pd062 | <0.11 |
| pd063 | 0.03 |
| pd064 | 0.04 |
| pd065 | 0.54 |
| pd066 | 0.33 |
| pd067 | <0.12 |
| pd068 | 0.27 |
| pd069 | <0.08 |
| pd070 | 0.03 |
| pd071 | 0.22 |
| pd072 | <0.13 |
| pd073 | <0.12 |
| pd074 | 0.22 |
| pd075 | 0.17 |
| pd076 | <0.10 |
| pd077 | 0.32 |
| pd078 | 2.75 |
| pd079 | 0.12 |
| pd080 | 0.25 |
| pd081 | 0.02 |
| pd082 | 0.03 |
| pd083 | 0.04 |
| pd084 | 0.21 |
| pd085 | 0.17 |
| pd086 | 0.06 |
| pd087 | <0.07 |
| pd088 | 0.11 |
| pd089 | <0.11 |
| pd090 | <0.12 |
| pd091 | 0.11 |
| pd092 | 0.09 |
| pd093 | <0.14 |
| pd094 | <0.13 |
| pd095 | <0.10 |
| pd096 | 0.51 |
| pd097 | 0.54 |
| pd098 | 0.57 |
| pd099 | 0.41 |
| pd100 | <0.12 |

(continued)

| ID | Caco2 $P_{app}$ ($10^{-6}$ cm/sec) |
|---|---|
| pd101 | 0.16 |
| pd102 | 0.09 |
| pd103 | 0.08 |
| pd104 | 0.20 |
| pd105 | 0.11 |
| pd106 | 0.24 |
| pd107 | 0.81 |
| pd108 | 0.03 |
| pd109 | 0.83 |
| pd110 | 0.43 |
| pd111 | 0.04 |
| pd112 | 0.35 |
| pd113 | 0.20 |
| pd114 | 0.16 |
| pd115 | 0.09 |
| pd116 | 0.19 |
| pd117 | 0.17 |
| pd118 | 0.46 |
| pd119 | 0.50 |
| pd120 | 0.69 |
| pd121 | 1.02 |
| pd122 | 0.06 |
| pd123 | 0.56 |
| pd124 | 0.13 |
| pd125 | 0.13 |
| pd126 | 0.88 |
| pd127 | 0.36 |
| pd128 | 0.05 |
| pd129 | 0.71 |
| pd130 | 3.88 |
| pd131 | 2.18 |
| pd132 | 0.36 |
| pd133 | 0.53 |
| pd134 | 0.75 |
| pd135 | 1.95 |
| pd136 | 0.53 |
| pd137 | 0.55 |
| pd138 | 0.23 |
| pd139 | 1.24 |

(continued)

| ID | Caco2 $P_{app}$ ($10^{-6}$ cm/sec) |
|---|---|
| pd140 | 0.31 |
| pd141 | 0.24 |
| pd142 | 0.71 |
| pd143 | 4.73 |
| pd144 | 1.28 |
| pd145 | 0.05 |
| pd146 | 0.20 |
| pd147 | 0.26 |
| pd148 | 0.12 |
| pd149 | 0.20 |
| pd150 | 0.29 |
| pd151 | 0.39 |
| pd152 | 1.02 |
| pd153 | 0.20 |
| pd154 | 0.82 |
| pd155 | 0.87 |
| pd156 | 0.39 |
| pd157 | 1.42 |
| pd158 | 0.44 |
| pd159 | 0.64 |
| pd160 | 0.06 |
| pd 161 | 0.09 |
| pd162 | 0.51 |
| pd163 | 0.04 |
| pd164 | 0.06 |
| pd165 | 0.10 |
| pd166 | 0.13 |
| pd167 | 0.23 |
| pd168 | 1.11 |

It was demonstrated that the tested cyclic peptide compounds consisting of 13 or 14 amino acid residues include a variety of compounds ranging from those with high membrane permeability to those with low permeability.

Example 5. Analysis of various properties and membrane permeability of cyclic peptide compounds

As demonstrated in Example 3, since all the tested cyclic peptide compounds consisting of 13 or 14 amino acid residues were found to have high metabolic stability, we focused on the membrane permeability shown in Example 4 as an indicator of drug-likeness and analyzed the relationship with various properties of the cyclic peptide compounds.
The values of membrane permeability and various properties of each cyclic peptide used in the analysis are shown in Table 8.

[Table 8]

| ID | Caco2 $P_{app}$ ($10^{-6}$ cm/sec) | totalAA | n-alkyl number | cLogP | totalAB | cLogP/ totalAB |
|---|---|---|---|---|---|---|
| pd001 | 0.94 | 14 | 10 | 16.1 | 15 | 1.07 |
| pd002 | 0.39 | 14 | 10 | 18.2 | 15 | 1.22 |
| pd003 | 0.27 | 14 | 10 | 15.9 | 15 | 1.06 |
| pd004 | 0.82 | 14 | 10 | 17.3 | 15 | 1.16 |
| pd005 | 0.05 | 14 | 10 | 13.4 | 15 | 0.89 |
| pd006 | <0.11 | 14 | 7 | 16.2 | 14 | 1.16 |
| pd007 | 0.11 | 14 | 7 | 17.1 | 14 | 1.22 |
| pd008 | 0.04 | 14 | 7 | 14.0 | 14 | 1.00 |
| pd009 | 0.13 | 14 | 7 | 15.9 | 14 | 1.14 |
| pd010 | 0.72 | 14 | 7 | 14.4 | 14 | 1.03 |
| pd011 | 0.11 | 14 | 7 | 13.9 | 14 | 0.99 |
| pd012 | 4.30 | 14 | 8 | 15.4 | 14 | 1.10 |
| pd013 | 0.04 | 14 | 7 | 13.4 | 15 | 0.89 |
| pd014 | <0.11 | 14 | 7 | 17.2 | 15 | 1.15 |
| pd015 | 0.04 | 14 | 7 | 18.0 | 15 | 1.20 |
| pd016 | 0.47 | 14 | 8 | 16.5 | 15 | 1.10 |
| pd017 | 0.09 | 14 | 8 | 15.2 | 15 | 1.01 |
| pd018 | 0.11 | 14 | 8 | 18.4 | 15 | 1.23 |
| pd019 | <0.11 | 14 | 7 | 14.3 | 15 | 0.95 |
| pd020 | <0.11 | 14 | 7 | 15.8 | 15 | 1.05 |
| pd021 | 0.15 | 14 | 7 | 16.4 | 15 | 1.09 |
| pd022 | 0.15 | 14 | 7 | 14.9 | 15 | 0.99 |
| pd023 | 0.03 | 14 | 7 | 14.6 | 15 | 0.98 |
| pd024 | 0.30 | 14 | 7 | 14.1 | 15 | 0.94 |
| pd025 | 0.05 | 14 | 7 | 13.8 | 15 | 0.92 |
| pd026 | 0.58 | 14 | 7 | 15.2 | 15 | 1.02 |
| pd027 | 0.02 | 14 | 7 | 17.7 | 15 | 1.18 |
| pd028 | 0.03 | 14 | 7 | 15.4 | 15 | 1.03 |
| pd029 | 0.02 | 14 | 7 | 16.7 | 15 | 1.11 |
| pd030 | 0.04 | 14 | 7 | 14.9 | 15 | 0.99 |
| pd031 | 0.04 | 14 | 7 | 16.7 | 15 | 1.11 |
| pd032 | 0.72 | 14 | 8 | 17.8 | 15 | 1.19 |
| pd033 | 0.03 | 14 | 8 | 19.0 | 15 | 1.26 |
| pd034 | 0.27 | 14 | 8 | 15.9 | 15 | 1.06 |
| pd035 | <0.11 | 14 | 8 | 19.4 | 15 | 1.29 |
| pd036 | 0.28 | 14 | 8 | 17.4 | 15 | 1.16 |
| pd037 | 0.26 | 14 | 8 | 15.0 | 15 | 1.00 |
| pd038 | 0.04 | 14 | 8 | 13.5 | 15 | 0.90 |
| pd039 | 0.03 | 14 | 8 | 15.0 | 15 | 1.00 |

(continued)

| ID | Caco2 $P_{app}$ ($10^{-6}$ cm/sec) | totalAA | n-alkyl number | cLogP | totalAB | cLogP/ totalAB |
|---|---|---|---|---|---|---|
| pd040 | 0.17 | 14 | 9 | 17.0 | 15 | 1.13 |
| pd041 | 0.13 | 14 | 9 | 15.6 | 15 | 1.04 |
| pd042 | 0.59 | 14 | 9 | 15.4 | 15 | 1.03 |
| pd043 | 0.16 | 14 | 9 | 18.5 | 15 | 1.23 |
| pd044 | 0.83 | 14 | 9 | 16.7 | 15 | 1.11 |
| pd045 | 0.32 | 14 | 9 | 16.7 | 15 | 1.11 |
| pd046 | 0.06 | 14 | 9 | 13.7 | 15 | 0.91 |
| pd047 | 0.16 | 14 | 10 | 17.7 | 15 | 1.18 |
| pd048 | 0.28 | 14 | 10 | 16.2 | 15 | 1.08 |
| pd049 | 0.32 | 14 | 8 | 16.1 | 14 | 1.15 |
| pd050 | 0.54 | 14 | 8 | 14.3 | 14 | 1.02 |
| pd051 | 0.21 | 14 | 8 | 15.1 | 14 | 1.08 |
| pd052 | 1.01 | 14 | 8 | 16.5 | 14 | 1.18 |
| pd053 | 1.64 | 14 | 8 | 17.0 | 14 | 1.22 |
| pd054 | 0.49 | 14 | 9 | 17.0 | 14 | 1.21 |
| pd055 | 0.12 | 14 | 9 | 13.5 | 14 | 0.97 |
| pd056 | 0.27 | 14 | 9 | 16.2 | 14 | 1.16 |
| pd057 | 1.16 | 14 | 9 | 18.1 | 14 | 1.30 |
| pd058 | 0.72 | 14 | 10 | 16.2 | 14 | 1.15 |
| pd059 | 0.52 | 14 | 10 | 14.6 | 14 | 1.04 |
| pd060 | 0.63 | 14 | 10 | 16.8 | 14 | 1.20 |
| pd061 | 0.51 | 14 | 10 | 18.8 | 14 | 1.34 |
| pd062 | <0.11 | 14 | 7 | 13.6 | 14 | 0.97 |
| pd063 | 0.03 | 14 | 7 | 15.5 | 14 | 1.11 |
| pd064 | 0.04 | 14 | 8 | 17.5 | 14 | 1.25 |
| pd065 | 0.54 | 14 | 9 | 17.6 | 14 | 1.26 |
| pd066 | 0.33 | 14 | 10 | 18.7 | 14 | 1.34 |
| pd067 | <0.12 | 14 | 8 | 14.7 | 14 | 1.05 |
| pd068 | 0.27 | 14 | 9 | 15.2 | 14 | 1.09 |
| pd069 | <0.08 | 14 | 9 | 15.3 | 14 | 1.09 |
| pd070 | 0.03 | 14 | 9 | 14.1 | 14 | 1.01 |
| pd071 | 0.22 | 14 | 10 | 15.0 | 14 | 1.07 |
| pd072 | <0.13 | 14 | 10 | 15.0 | 14 | 1.07 |
| pd073 | <0.12 | 14 | 10 | 15.3 | 14 | 1.09 |
| pd074 | 0.22 | 14 | 8 | 17.2 | 15 | 1.15 |
| pd075 | 0.17 | 14 | 8 | 17.8 | 15 | 1.19 |
| pd076 | <0.10 | 14 | 9 | 15.5 | 15 | 1.03 |
| pd077 | 0.32 | 14 | 9 | 18.0 | 15 | 1.20 |
| pd078 | 2.75 | 14 | 9 | 17.3 | 15 | 1.15 |

(continued)

| ID | Caco2 $P_{app}$ ($10^{-6}$ cm/sec) | totalAA | n-alkyl number | cLogP | totalAB | cLogP/ totalAB |
|---|---|---|---|---|---|---|
| pd079 | 0.12 | 14 | 9 | 18.8 | 15 | 1.26 |
| pd080 | 0.25 | 14 | 9 | 16.9 | 15 | 1.12 |
| pd081 | 0.02 | 14 | 10 | 15.2 | 15 | 1.01 |
| pd082 | 0.03 | 14 | 10 | 19.6 | 15 | 1.31 |
| pd083 | 0.04 | 14 | 10 | 16.0 | 15 | 1.07 |
| pd084 | 0.21 | 14 | 10 | 19.5 | 15 | 1.30 |
| pd085 | 0.17 | 14 | 10 | 17.5 | 15 | 1.17 |
| pd086 | 0.06 | 14 | 8 | 16.0 | 15 | 1.06 |
| pd087 | <0.07 | 14 | 8 | 17.0 | 15 | 1.13 |
| pd088 | 0.11 | 14 | 8 | 15.6 | 15 | 1.04 |
| pd089 | <0.11 | 14 | 9 | 19.6 | 15 | 1.31 |
| pd090 | <0.12 | 14 | 9 | 14.3 | 15 | 0.95 |
| pd091 | 0.11 | 14 | 9 | 17.9 | 15 | 1.19 |
| pd092 | 0.09 | 14 | 9 | 19.7 | 15 | 1.31 |
| pd093 | <0.14 | 14 | 10 | 20.3 | 15 | 1.35 |
| pd094 | <0.13 | 14 | 10 | 14.0 | 15 | 0.93 |
| pd095 | <0.10 | 14 | 10 | 19.4 | 15 | 1.30 |
| pd096 | 0.51 | 14 | 10 | 16.8 | 15 | 1.12 |
| pd097 | 0.54 | 13 | 7 | 15.7 | 14 | 1.12 |
| pd098 | 0.57 | 13 | 8 | 16.0 | 14 | 1.15 |
| pd099 | 0.41 | 13 | 8 | 16.9 | 14 | 1.21 |
| pd100 | <0.12 | 13 | 7 | 18.4 | 14 | 1.32 |
| pd101 | 0.16 | 13 | 7 | 15.5 | 14 | 1.10 |
| pd102 | 0.09 | 13 | 7 | 14.8 | 14 | 1.05 |
| pd103 | 0.08 | 13 | 7 | 18.1 | 14 | 1.29 |
| pd104 | 0.20 | 13 | 7 | 16.2 | 14 | 1.16 |
| pd105 | 0.11 | 13 | 7 | 13.9 | 14 | 0.99 |
| pd106 | 0.24 | 13 | 7 | 15.2 | 14 | 1.08 |
| pd107 | 0.81 | 13 | 8 | 16.8 | 14 | 1.20 |
| pd108 | 0.03 | 13 | 8 | 18.4 | 14 | 1.31 |
| pd109 | 0.83 | 13 | 8 | 14.3 | 14 | 1.02 |
| pd110 | 0.43 | 13 | 8 | 14.9 | 14 | 1.07 |
| pd111 | 0.04 | 13 | 8 | 18.8 | 14 | 1.34 |
| pd112 | 0.35 | 13 | 8 | 14.5 | 14 | 1.03 |
| pd113 | 0.20 | 13 | 8 | 13.1 | 14 | 0.93 |
| pd114 | 0.16 | 13 | 8 | 16.9 | 14 | 1.21 |
| pd115 | 0.09 | 13 | 8 | 14.0 | 14 | 1.00 |
| pd116 | 0.19 | 13 | 9 | 16.0 | 14 | 1.14 |
| pd117 | 0.17 | 13 | 9 | 14.1 | 14 | 1.00 |

(continued)

| ID | Caco2 $P_{app}$ ($10^{-6}$ cm/sec) | totalAA | n-alkyl number | cLogP | totalAB | cLogP/ totalAB |
|---|---|---|---|---|---|---|
| pd118 | 0.46 | 13 | 9 | 14.4 | 14 | 1.03 |
| pd119 | 0.50 | 13 | 9 | 17.0 | 14 | 1.21 |
| pd120 | 0.69 | 13 | 9 | 13.7 | 14 | 0.98 |
| pd121 | 1.02 | 13 | 9 | 15.7 | 14 | 1.12 |
| pd122 | 0.06 | 13 | 9 | 14.2 | 14 | 1.01 |
| pd123 | 0.56 | 13 | 10 | 17.2 | 14 | 1.23 |
| pd124 | 0.13 | 13 | 10 | 15.3 | 14 | 1.09 |
| pd125 | 0.13 | 13 | 10 | 16.3 | 14 | 1.16 |
| pd126 | 0.88 | 13 | 10 | 15.7 | 14 | 1.12 |
| pd127 | 0.36 | 13 | 7 | 17.0 | 13 | 1.31 |
| pd128 | 0.05 | 13 | 7 | 13.0 | 13 | 1.00 |
| pd129 | 0.71 | 13 | 7 | 14.4 | 13 | 1.11 |
| pd130 | 3.88 | 13 | 8 | 14.8 | 13 | 1.14 |
| pd131 | 2.18 | 13 | 8 | 14.6 | 13 | 1.12 |
| pd132 | 0.36 | 13 | 8 | 13.4 | 13 | 1.03 |
| pd133 | 0.53 | 13 | 8 | 14.4 | 13 | 1.11 |
| pd134 | 0.75 | 13 | 8 | 15.6 | 13 | 1.20 |
| pd135 | 1.95 | 13 | 8 | 17.0 | 13 | 1.31 |
| pd136 | 0.53 | 13 | 9 | 15.5 | 13 | 1.19 |
| pd137 | 0.55 | 13 | 9 | 13.3 | 13 | 1.03 |
| pd138 | 0.23 | 13 | 9 | 13.7 | 13 | 1.06 |
| pd139 | 1.24 | 13 | 9 | 17.2 | 13 | 1.32 |
| pd140 | 0.31 | 13 | 10 | 14.9 | 13 | 1.15 |
| pd141 | 0.24 | 13 | 8 | 15.1 | 13 | 1.16 |
| pd142 | 0.71 | 13 | 9 | 17.6 | 13 | 1.35 |
| pd143 | 4.73 | 13 | 10 | 15.9 | 13 | 1.22 |
| pd144 | 1.28 | 13 | 10 | 16.7 | 13 | 1.29 |
| pd145 | 0.05 | 13 | 7 | 13.3 | 13 | 1.02 |
| pd146 | 0.20 | 13 | 8 | 13.2 | 13 | 1.01 |
| pd147 | 0.26 | 13 | 9 | 13.8 | 13 | 1.06 |
| pd148 | 0.12 | 13 | 9 | 14.8 | 13 | 1.14 |
| pd149 | 0.20 | 13 | 9 | 13.5 | 13 | 1.04 |
| pd150 | 0.29 | 13 | 10 | 13.9 | 13 | 1.07 |
| pd151 | 0.39 | 13 | 8 | 16.2 | 14 | 1.16 |
| pd152 | 1.02 | 13 | 8 | 17.2 | 14 | 1.23 |
| pd153 | 0.20 | 13 | 9 | 15.9 | 14 | 1.14 |
| pd154 | 0.82 | 13 | 9 | 17.0 | 14 | 1.22 |
| pd155 | 0.87 | 13 | 9 | 16.8 | 14 | 1.20 |
| pd156 | 0.39 | 13 | 9 | 16.3 | 14 | 1.16 |

(continued)

| ID | Caco2 $P_{app}$ ($10^{-6}$ cm/sec) | totalAA | n-alkyl number | cLogP | totalAB | cLogP/ totalAB |
|---|---|---|---|---|---|---|
| pd157 | 1.42 | 13 | 10 | 18.0 | 14 | 1.28 |
| pd158 | 0.44 | 13 | 8 | 15.0 | 14 | 1.07 |
| pd159 | 0.64 | 13 | 8 | 17.4 | 14 | 1.24 |
| pd160 | 0.06 | 13 | 8 | 18.0 | 14 | 1.29 |
| pd161 | 0.09 | 13 | 9 | 19.0 | 14 | 1.36 |
| pd162 | 0.51 | 13 | 9 | 14.2 | 14 | 1.02 |
| pd163 | 0.04 | 13 | 9 | 17.8 | 14 | 1.27 |
| pd164 | 0.06 | 13 | 9 | 14.9 | 14 | 1.07 |
| pd165 | 0.10 | 13 | 9 | 18.7 | 14 | 1.33 |
| pd166 | 0.13 | 13 | 10 | 18.4 | 14 | 1.32 |
| pd167 | 0.23 | 13 | 10 | 18.4 | 14 | 1.31 |
| pd168 | 1.11 | 13 | 10 | 17.1 | 14 | 1.22 |

In Table 8, total AA (total amino acid) represents the number of amino acid residues composing each cyclic peptide, and N-alkyl number represents the number of amides that are N-alkylated in the backbone amides.

cLogP, a partition coefficient calculated by a computer, was calculated using Daylight Version 4.95 from Daylight Chemical Information Systems, Inc. The total AB (total amide bond) refers to the number of amide bonds of the backbone and side chains contained in each compound. For example, in the cyclic peptides shown in this Example, it corresponds to one of the patterns listed in Table 9.

[Table 9]

| Pattern | total AA | C-terminal amino acid | total AB |
|---|---|---|---|
| 1 | 13 | D-3-Abu or D-3-MeAbu | 13 |
| 2 | 13 | Asp-pip or MeAsp-pip | 14 |
| 3 | 14 | D-3-Abu or D-3-MeAbu | 14 |
| 4 | 14 | Asp-pip or MeAsp-pip | 15 |

cLogP/total AB represents the value obtained by dividing cLogP by total AB, and it was used in this Example as an indicator of the lipophilicity of peptide compounds.

Example 5-1. Analysis of N-Alkyl Number and Membrane Permeability

The relationship between the N-alkyl number and membrane permeability of cyclic peptide compounds was analyzed (Table 10).

[Table 10]

| N-alkyl number | Caco2 $P_{app}$ ($10^{-6}$ cm/sec) | | | | | | total compounds |
|---|---|---|---|---|---|---|---|
| | $1.0 \leqq P_{app}$ | | $0.4 \leqq P_{app} < 1.0$ | | $P_{app} < 0.4$ | | |
| 7 | 0 cpds | 0% | 4 cpds | 11% | 32 cpds | 89% | 36 cpds |
| 8 | 7 cpds | 15% | 12 cpds | 25% | 29 cpds | 60% | 48 cpds |
| 9 | 4 cpds | 8% | 13 cpds | 27% | 31 cpds | 65% | 48 cpds |
| 10 | 4 cpds | 11% | 9 cpds | 25% | 23 cpds | 64% | 36 cpds |

In the case of compounds with the N-alkyl number of 7, 4 out of the 36 compounds tested showed $P_{app} \geq 0.4 \times$

$10^{-6}$ cm/s, but no compound showed $P_{app} \geq 1.0 \times 10^{-6}$ cm/s. In the case of compounds with the N-alkyl number of 8, 19 out of the 48 compounds tested showed $P_{app} \geq 0.4 \times 10^{-6}$ cm/s, and furthermore, 7 out of those compounds showed $P_{app} \geq 1.0 \times 10^{-6}$ cm/s. In the case of compounds with the N-alkyl number of 9, 17 out of the 48 compounds tested showed $P_{app} \geq 0.4 \times 10^{-6}$ cm/s, and furthermore, 4 out of those compounds showed $P_{app} \geq 1.0 \times 10^{-6}$ cm/s. In the case of compounds with the N-alkyl number of 10, 13 out of the 36 compounds tested showed $P_{app} \geq 0.4 \times 10^{-6}$ cm/s, and furthermore, 4 out of those compounds showed $P_{app} \geq 1.0 \times 10^{-6}$ cm/s.

From the above, it was demonstrated that compounds with the N-alkyl number of 7 can achieve $P_{app} \geq 0.4 \times 10^{-6}$ cm/s, and compounds with the N-alkyl number of 8 or more can achieve $P_{app} \geq 1.0 \times 10^{-6}$ cm/s. In addition, it was shown that the proportion of compounds that achieve $P_{app} \geq 0.4 \times 10^{-6}$ cm/s is higher for compounds with the N-alkyl number of 8 or more than for those with the N-alkyl number of 7.

Example 5-2. Analysis of lipophilicity and membrane permeability

The relationship between the lipophilicity and membrane permeability of cyclic peptide compounds was analyzed (Table 11). In this Example, cLogP/total AB was used as an indicator of lipophilicity.

[Table 11]

| cLogP/totalAB | Caco2 $P_{app}$ ($10^{-6}$ cm/sec) | | | | | | total compounds |
|---|---|---|---|---|---|---|---|
| | $1.0 \leqq P_{app}$ | | $0.4 \leqq P_{app} < 1.0$ | | $P_{app} < 0.4$ | | |
| cLogP/totalAB<1.0 | 0 cpds | 0% | 1 cpds | 5% | 19 cpds | 95% | 20 cpds |
| 1.0≦clogP/totalAB<1.1 | 1 cpds | 2% | 13 cpds | 25% | 38 cpds | 73% | 52 cpds |
| 1.1≦LogP/totalAB | 14 cpds | 15% | 24 cpds | 25% | 58 cpds | 60% | 96 cpds |

In the case of compounds with cLogP/totalAB of cLogP/totalAB < 1.0, 1 out of the 20 compounds tested showed $P_{app} \geq 0.4 \times 10^{-6}$ cm/s, but no compound showed $P_{app} \geq 1.0 \times 10^{-6}$ cm/s. In the case of compounds with cLogP/totalAB of 1.0 ≤ cLogP/totalAB < 1.1, 14 out of the 52 compounds tested showed $P_{app} \geq 0.4 \times 10^{-6}$ cm/s, and furthermore, 1 out of those compounds showed $P_{app} \geq 1.0 \times 10^{-6}$ cm/s. In the case of compounds with cLogP/totalAB of 1.1 ≤ cLogP/totalAB, 39 out of the 96 compounds tested showed $P_{app} \geq 0.4 \times 10^{-6}$ cm/s, and furthermore, 14 out of those compounds showed $P_{app} \geq 1.0 \times 10^{-6}$ cm/s.

From the above, it was demonstrated that compounds with cLogP/totalAB < 1.0 have difficulty achieving $P_{app} \geq 0.4 \times 10^{-6}$ cm/s, compounds with 1.0 ≤ cLogP/totalAB < 1.1 can achieve $P_{app} \geq 1.0 \times 10^{-6}$ cm/s, and compounds with 1.1 ≤ cLogP/totalAB can achieve $P_{app} \geq 1.0 \times 10^{-6}$ cm/s. It was also demonstrated that the proportion of compounds achieving $P_{app} \geq 0.4 \times 10^{-6}$ cm/s is higher for compounds with 1.1 ≤ cLogP/totalAB than for compounds with cLogP/totalAB < 1.1.

Example 5-3. Analysis of MeLeu and membrane permeability

For each cyclic peptide compound, the relationship between the number of MeLeu and membrane permeability was analyzed (Table 12). It was found from Example 5-2 that it is difficult for compounds with cLogP/totalAB < 1.0 to achieve $P_{app} \geq 0.4 \times 10^{-6}$ cm/s, thus the analysis was conducted only on compounds with cLogP/totalAB ≥ 1.0.

[Table 12]

| Number of MeLeu | Caco-2 $P_{app}$ ($10^{-6}$ cm/sec) | | | | | | total compounds |
|---|---|---|---|---|---|---|---|
| | $1.0 \leq P_{app}$ | | $0.4 \leq P_{app} < 1.0$ | | $P_{app} < 0.4$ | | |
| 1 | 1 cpds | 13% | 4 cpds | 50% | 3 cpds | 38% | 8 cpds |
| 2 | 5 cpds | 7% | 15 cpds | 20% | 55 cpds | 73% | 75 cpds |
| 3 | 9 cpds | 14% | 18 cpds | 29% | 36 cpds | 57% | 63 cpds |
| 4 | 0 cpds | 0% | 0 cpds | 0% | 4 cpds | 100% | 4 cpds |

In the case of compounds with one MeLeu, 5 out of the 8 compounds tested showed $P_{app} \geq 0.4 \times 10^{-6}$ cm/s, in the case of compounds with two MeLeu, 20 out of the 75 compounds tested showed $P_{app} \geq 0.4 \times 10^{-6}$ cm/s, and in the case of compounds with three MeLeu, 27 out of the 63 compounds tested showed $P_{app} \geq 0.4 \times 10^{-6}$ cm/s. On the other hand, in the case of compounds with four MeLeu, none of the four compounds tested showed $P_{app} \geq 1.0 \times 10^{-6}$ cm/s.

Example 6. Mouse PK test of cyclic peptide compounds

Examples 3 to 5 revealed that the N-alkyl number is 8 or more and the cLogP/totalAB is 1.1 or more are the conditions for a cyclic peptide compound consisting of 13 or 14 amino acid residues to exhibit particularly good drug-likeness. We evaluated the plasma concentration changes after intravenous or oral administration of four types of peptides that satisfy these conditions to mice.

To male mice (C57BL/6J, 10 weeks old, purchased from Charles River Laboratories Japan, Inc.: 3 mice per group), the compound was administered intravenously at a dose of 1 mg/kg or orally at a dose of 50 mg/kg, and blood was collected from the jugular vein over time up to 24 hours after administration. The collected blood was dispensed into tubes that were pre-treated with heparin as an anticoagulant. Plasma was separated from the blood by centrifugation, and after deproteinization with acetonitrile, the plasma concentration of the compound was measured with an LC/MS/MS device (XEVO TQ-XS, manufactured by Waters Corporation). Based on the obtained plasma concentration-time profile, the following pharmacokinetic parameters were calculated by non-compartmental analysis using the analysis software Phoenix WinNonlin 6.1 (manufactured by Pharsight Corporation, USA) (Tables 13 and 14).

$AUC_{inf}$: area under the plasma concentration-time curve (ng-h/mL)
CL: systemic clearance (mL/min/kg)
Vss: steady-state distribution volume (L/kg)
t1/2: elimination half-life (hours)
Cmax: maximum plasma concentration (ng/mL)
Tmax: time to reach maximum plasma concentration (hours)
F: bioavailability (%)

[Table 13]

| Pharmacokinetic parameters at intravenous administration | | | | | |
|---|---|---|---|---|---|
| ID | totalAA | AUCinf (ng·h/mL) | CL (mL/min/kg) | Vss (L/kg) | t1/2 (h) |
| pd057 | 14 | 1200 | 14 | 11 | 12 |
| pd065 | 14 | 8700 | 1.9 | 0.57 | 5.4 |
| pd135 | 13 | 10000 | 1.7 | 0.81 | 6.4 |
| pd144 | 13 | 3500 | 4.9 | 1.6 | 5.3 |

[Table 14]

| Pharmacokinetic parameters at oral administration | | | | | | |
|---|---|---|---|---|---|---|
| ID | totalAA | AUCinf (ng·h/mL) | t1/2 (h) | Cmax (ng/mL) | Tmax (h) | F (%) |
| pd057 | 14 | 20000 | 6.6 | 2200 | 2.7 | 33 |
| pd065 | 14 | 110000 | 5.8 | 11000 | 2.7 | 26 |
| pd135 | 13 | 79000 | 7.6 | 7000 | 1.7 | 16 |
| pd144 | 13 | 71000 | 9.7 | 7000 | 1.3 | 40 |

All tested compounds had low systemic clearance (1.7-14 mL/min/kg) after intravenous administration, suggesting high metabolic stability. The bioavailability of the compounds was up to 40%, and the four peptides tested had oral absorption suitable for development as oral administration agents as expected.

[Table 15]

| Compound No. | Structural formula | Compound No. | Structural formula |
| --- | --- | --- | --- |
| pd001 | 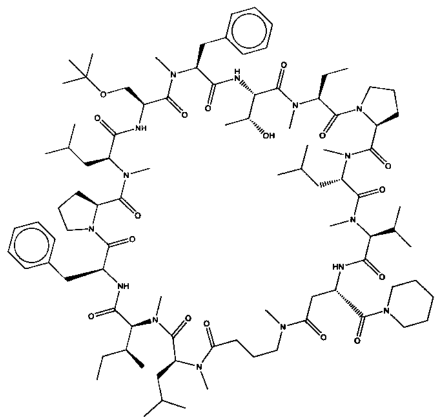 | pd003 | 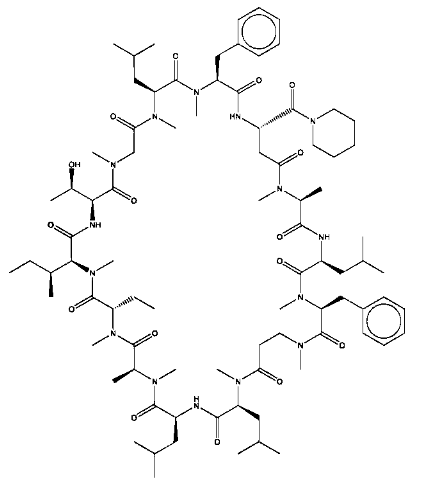 |
| pd002 | 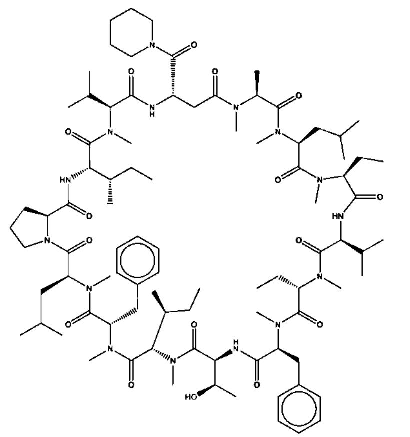 | pd004 | 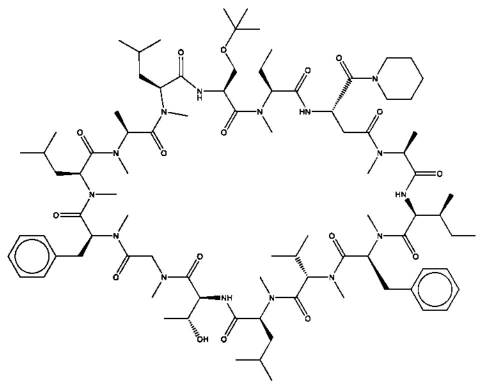 |

(continued)

| Compound No. | Structural formula | Compound No. | Structural formula |
|---|---|---|---|
| pd005 | 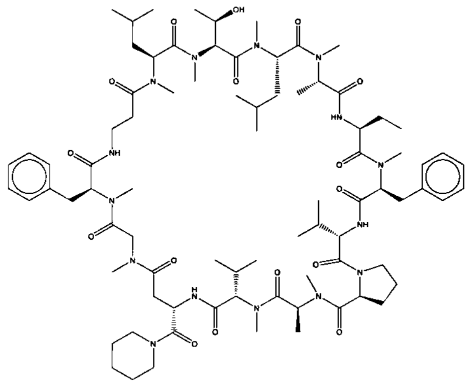 | pd007 | 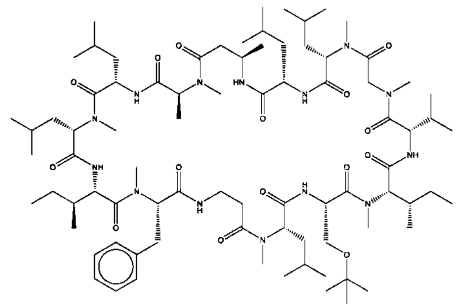 |
| pd006 | 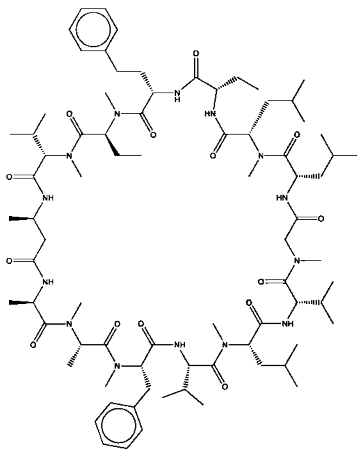 | pd008 | 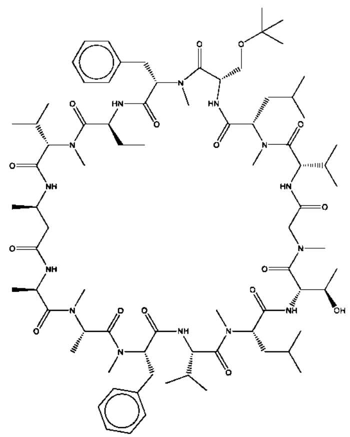 |

(continued)

| Compound No. | Structural formula | Compound No. | Structural formula |
|---|---|---|---|
| pd009 | 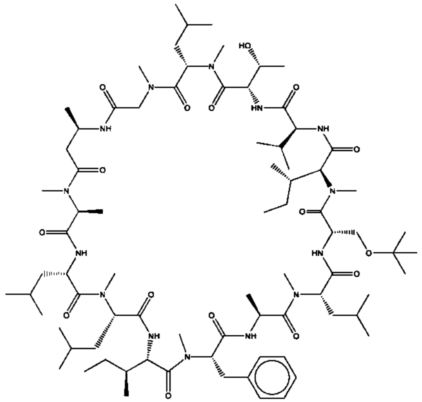 | pd011 | 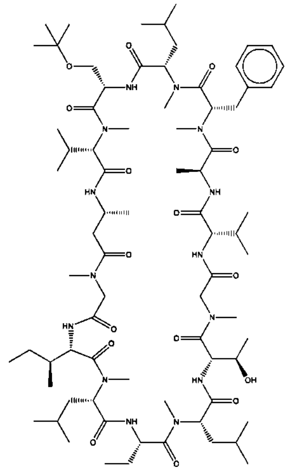 |
| pd010 | 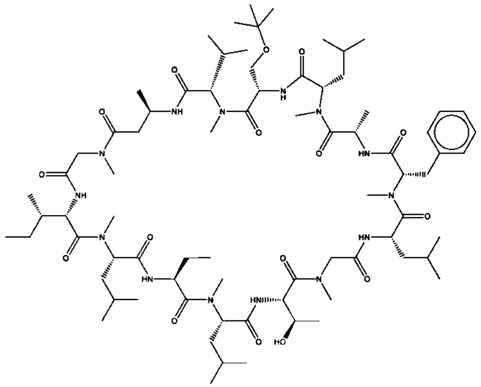 | pd012 | 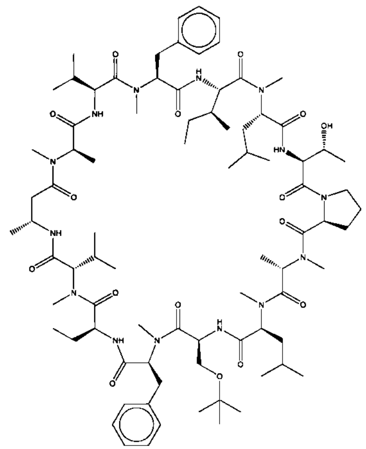 |

(continued)

| Compound No. | Structural formula | Compound No. | Structural formula |
|---|---|---|---|
| pd013 | 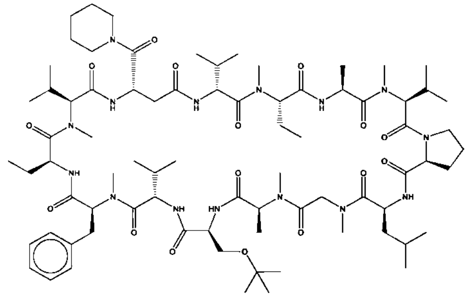 | pd015 | 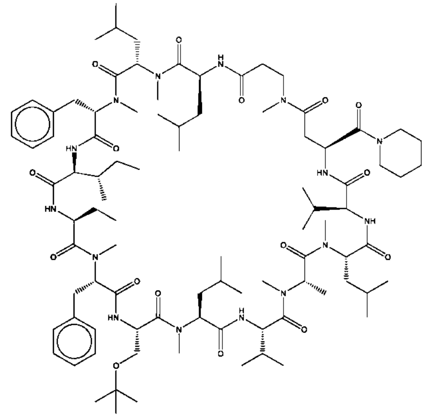 |
| pd014 | 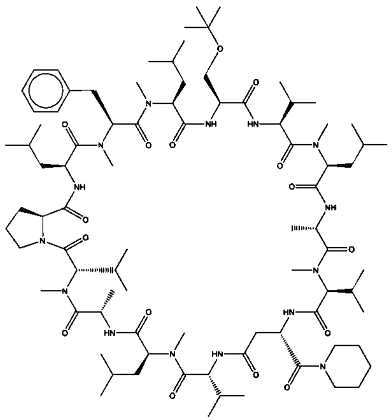 | pd016 | 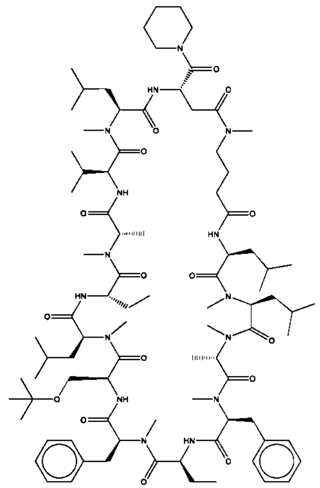 |

(continued)

| Compound No. | Structural formula | Compound No. | Structural formula |
|---|---|---|---|
| pd017 | 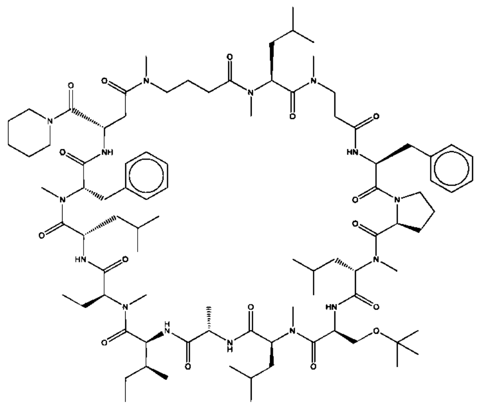 | pd019 | 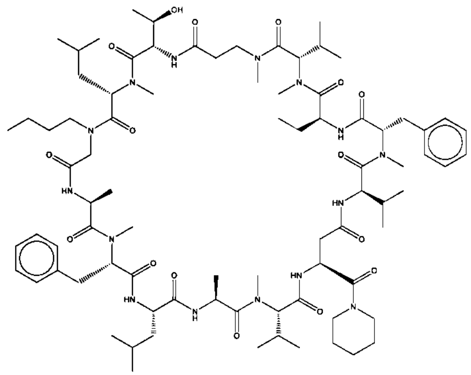 |
| pd018 | 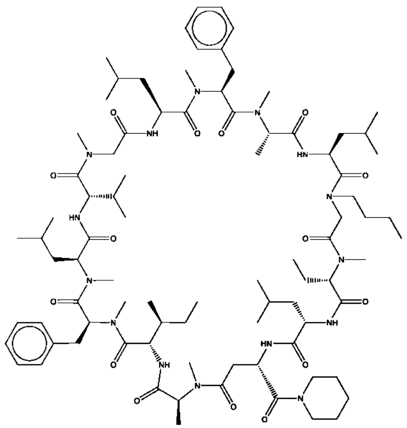 | pd020 | 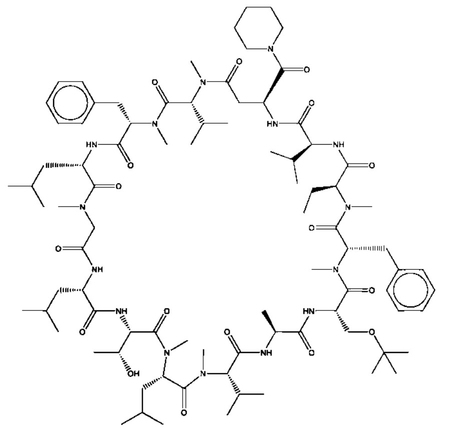 |

(continued)

| Compound No. | Structural formula | Compound No. | Structural formula |
|---|---|---|---|
| pd021 | 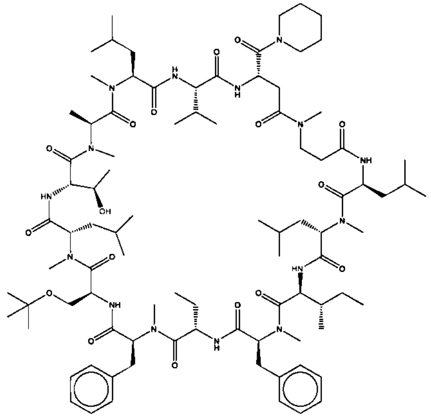 | pd023 | 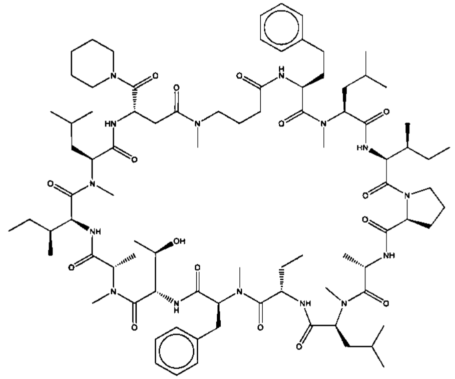 |
| pd022 | 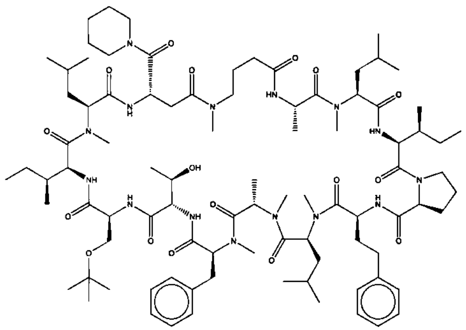 | pd024 | 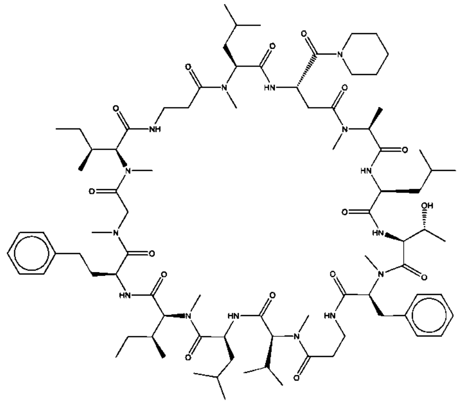 |

(continued)

| Compound No. | Structural formula | Compound No. | Structural formula |
|---|---|---|---|
| pd025 | 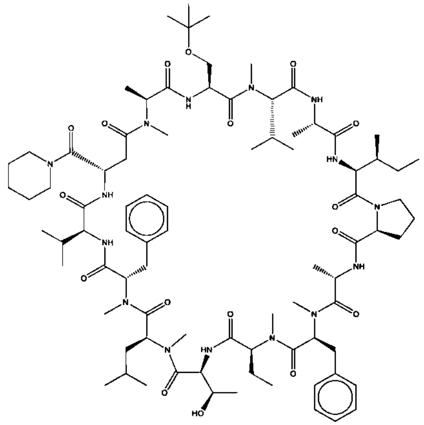 | pd027 | 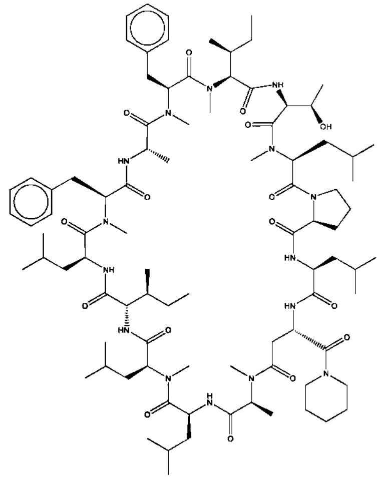 |
| pd026 | 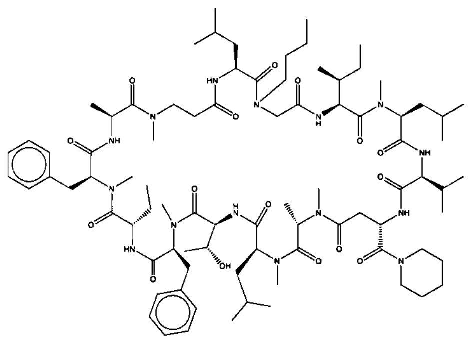 | pd028 | 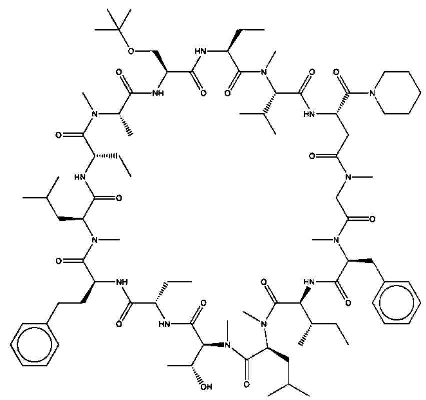 |

(continued)

| Compound No. | Structural formula | Compound No. | Structural formula |
|---|---|---|---|
| pd029 | 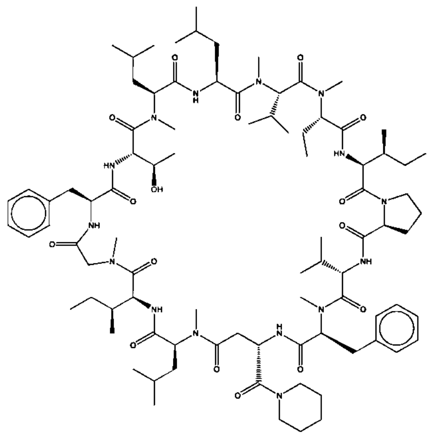 | pd031 | 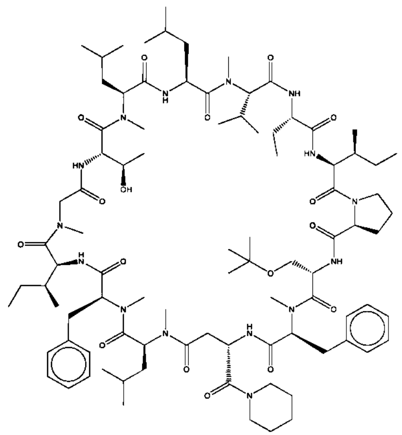 |
| pd030 | 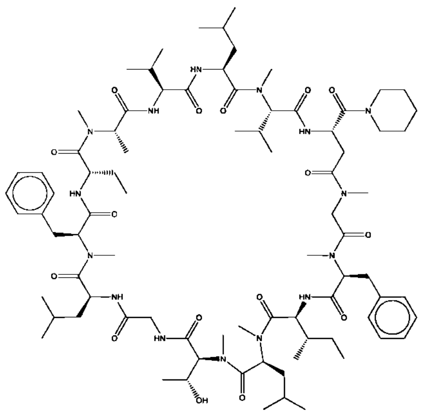 | pd032 | 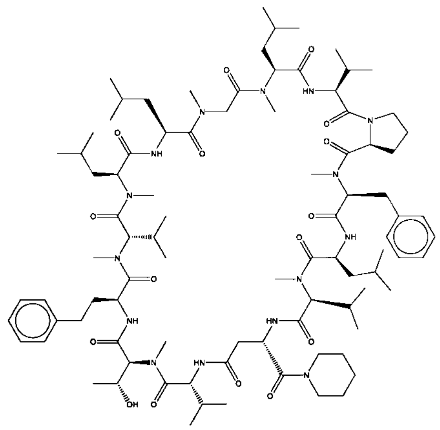 |

(continued)

| Compound No. | Structural formula | Compound No. | Structural formula |
| --- | --- | --- | --- |
| pd033 | 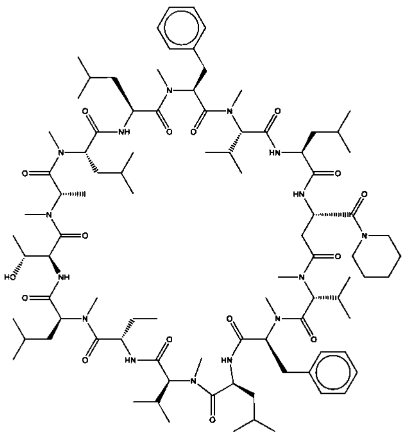 | pd035 | 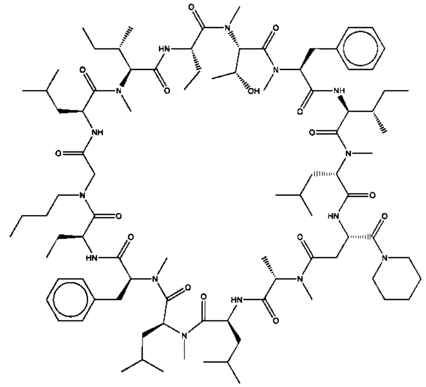 |
| pd034 | 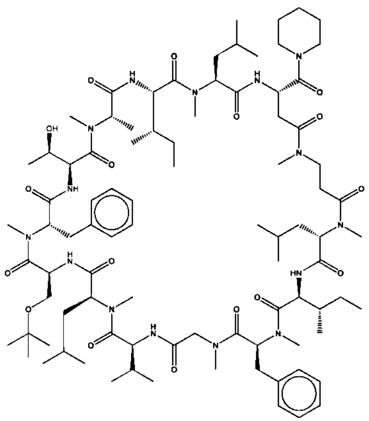 | pd036 | 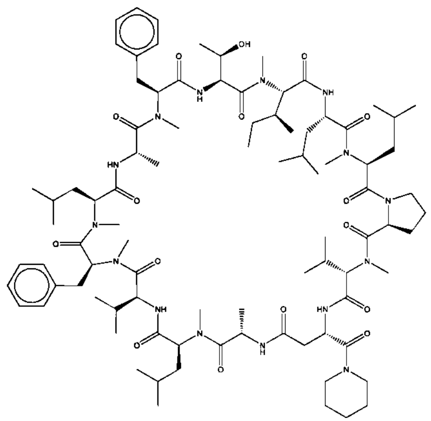 |

(continued)

| Compound No. | Structural formula | Compound No. | Structural formula |
|---|---|---|---|
| pd037 | 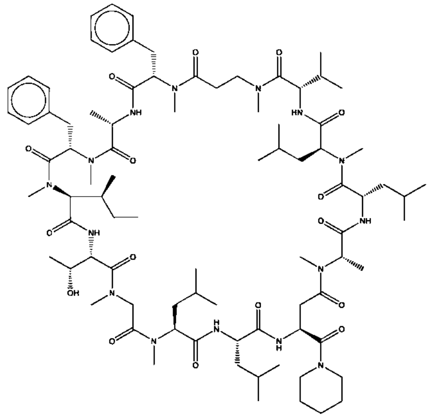 | pd039 | 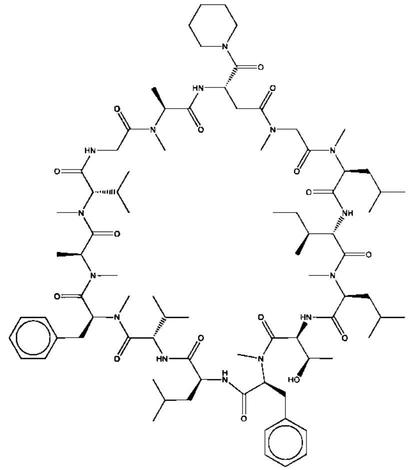 |
| pd038 | 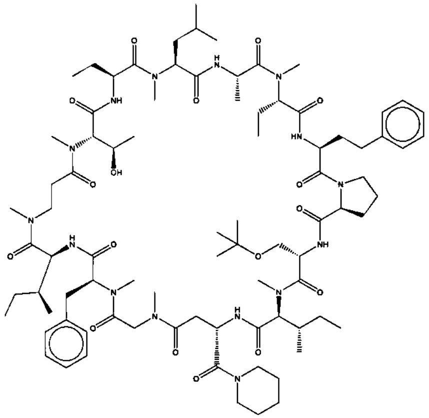 | pd040 | 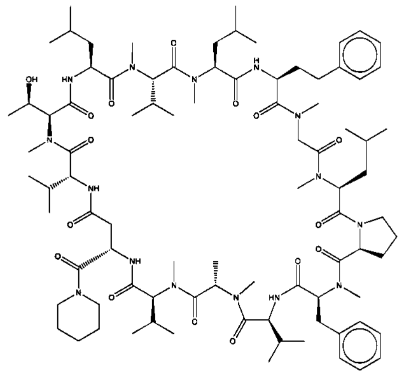 |

(continued)

| Compound No. | Structural formula | Compound No. | Structural formula |
| --- | --- | --- | --- |
| pd041 | 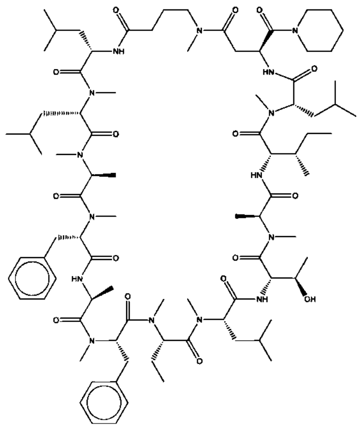 | pd043 | 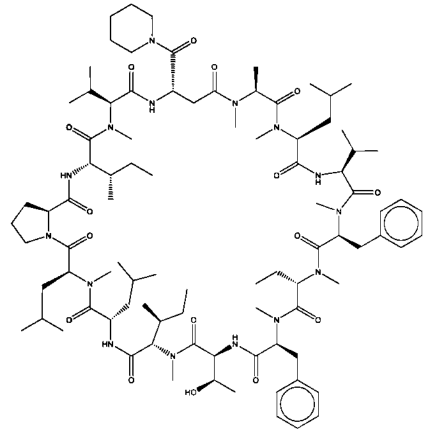 |
| pd042 | 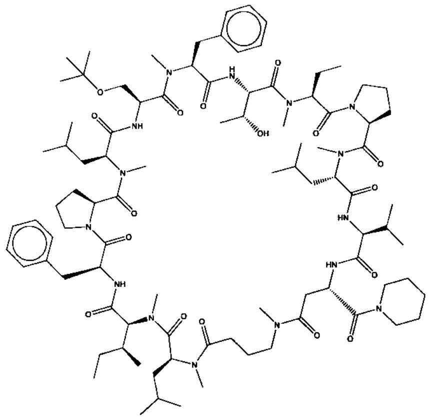 | pd044 | 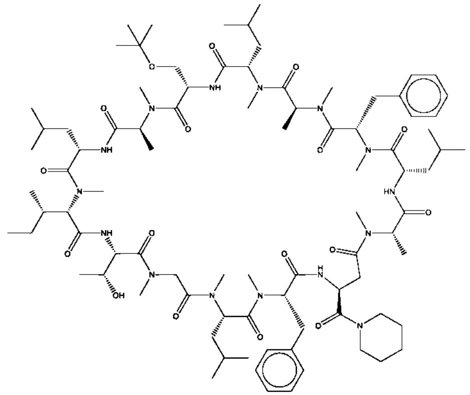 |

(continued)

| Compound No. | Structural formula | Compound No. | Structural formula |
|---|---|---|---|
| pd045 | 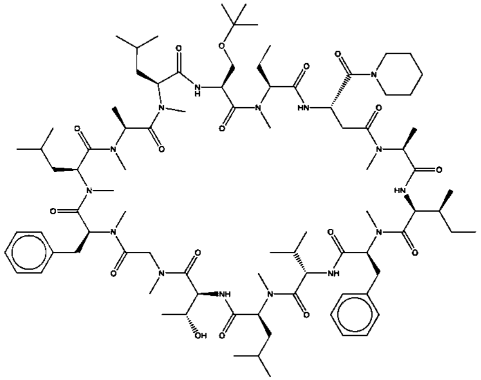 | pd047 | 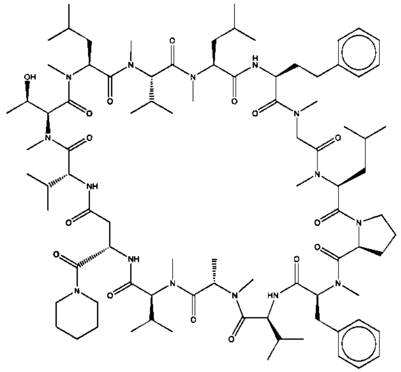 |
| pd046 | 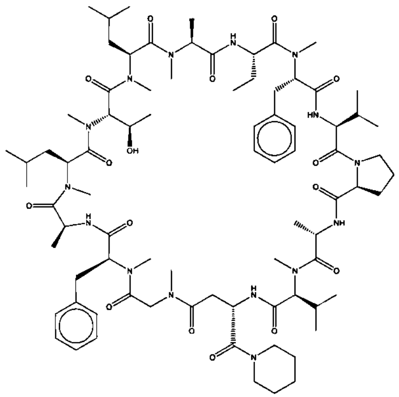 | pd048 | 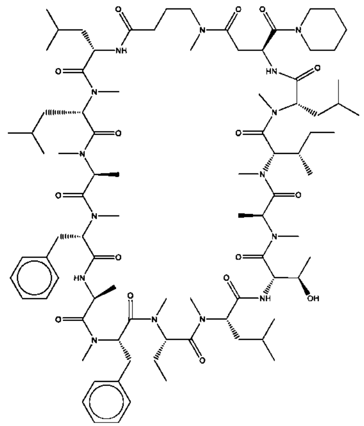 |

(continued)

| Compound No. | Structural formula | Compound No. | Structural formula |
|---|---|---|---|
| pd049 | 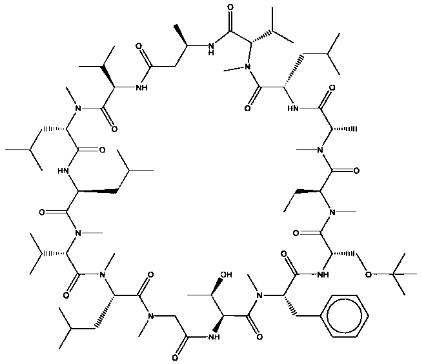 | pd051 | 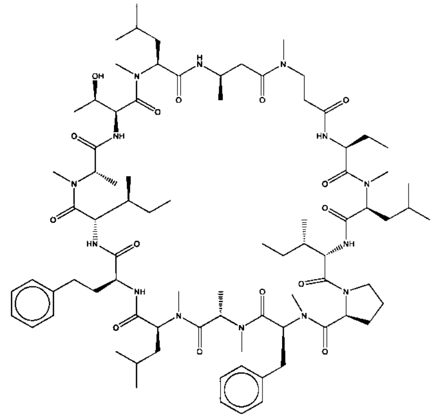 |
| pd050 | 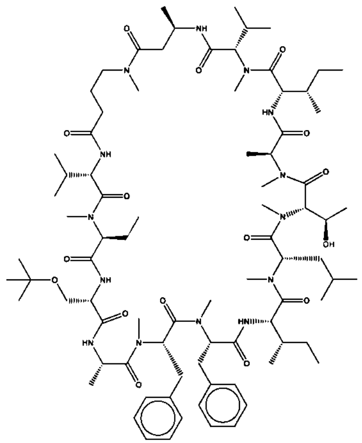 | pd052 | 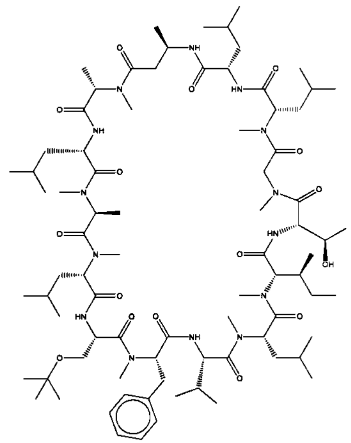 |

(continued)

| Compound No. | Structural formula | Compound No. | Structural formula |
| --- | --- | --- | --- |
| pd053 | 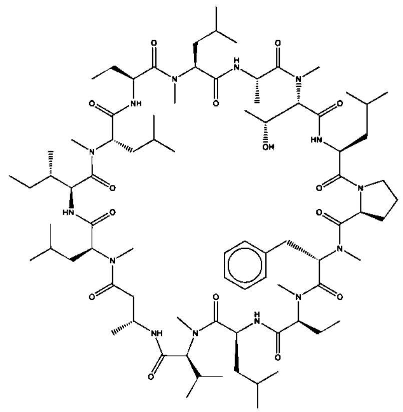 | pd055 | 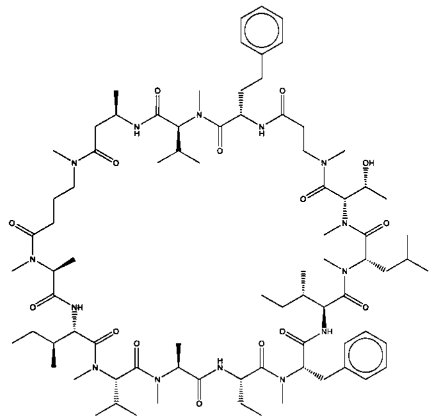 |
| pd054 | 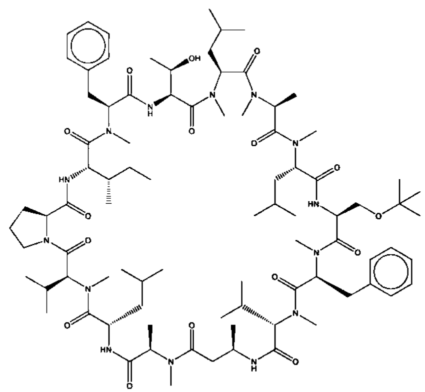 | pd056 | 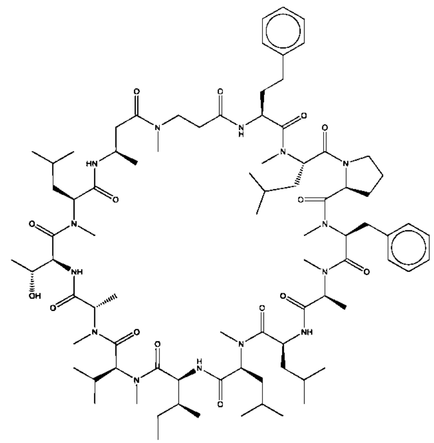 |

(continued)

| Compound No. | Structural formula | Compound No. | Structural formula |
|---|---|---|---|
| pd057 | 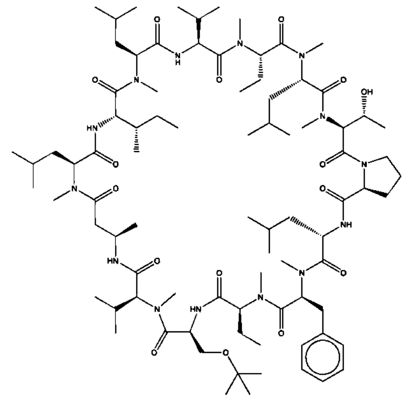 | pd059 | 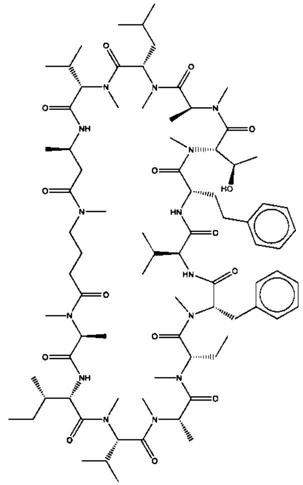 |
| pd058 | 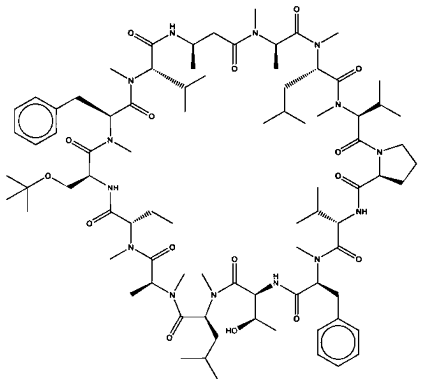 | pd060 | 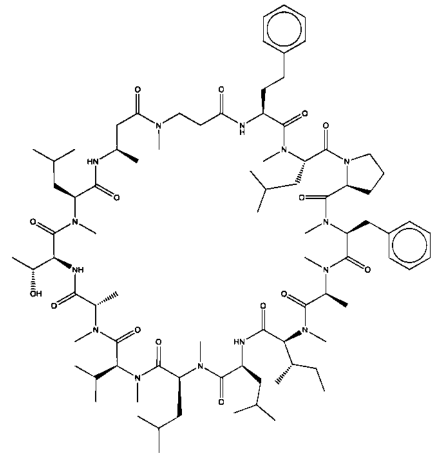 |

(continued)

| Compound No. | Structural formula | Compound No. | Structural formula |
| --- | --- | --- | --- |
| pd061 | 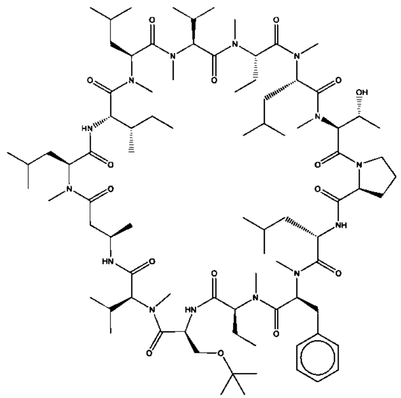 | pd063 | 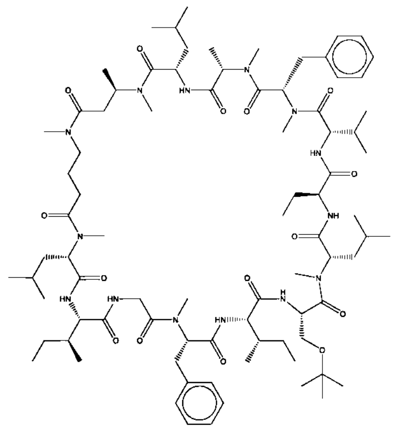 |
| pd062 | 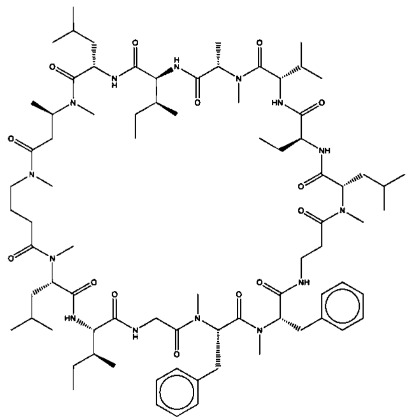 | pd064 | 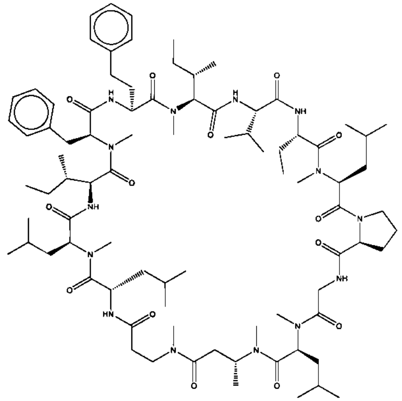 |

(continued)

| Compound No. | Structural formula | Compound No. | Structural formula |
|---|---|---|---|
| pd065 | 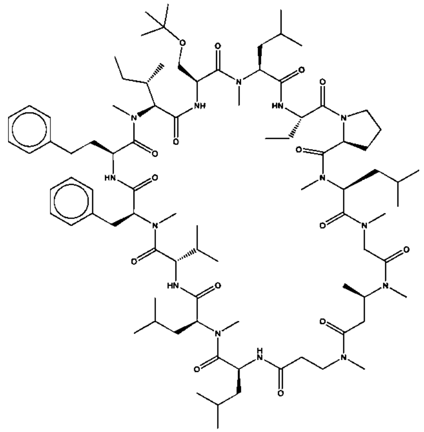 | pd067 | 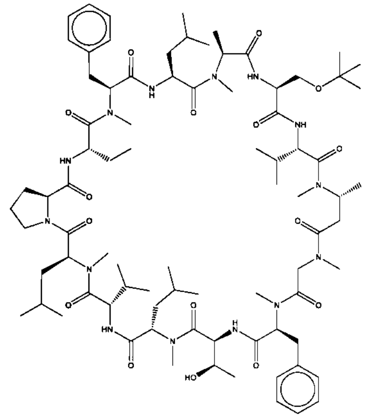 |
| pd066 | 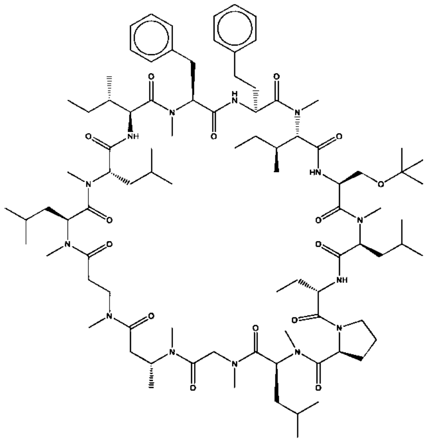 | pd068 | 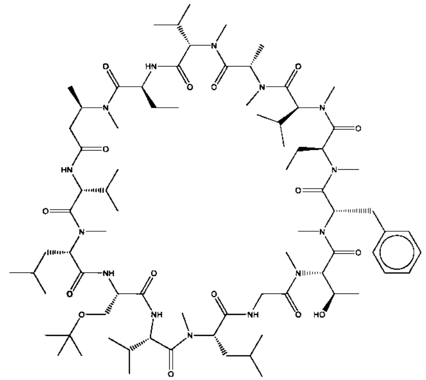 |

(continued)

| Compound No. | Structural formula | Compound No. | Structural formula |
|---|---|---|---|
| pd069 | 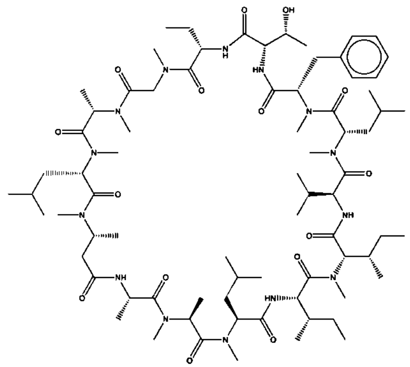 | pd071 | 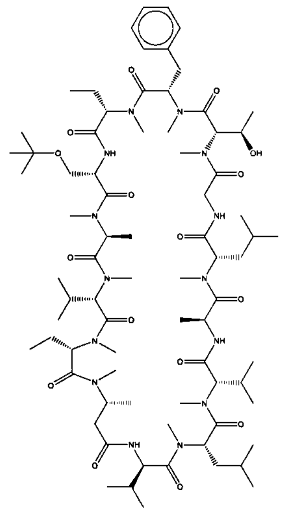 |
| pd070 | 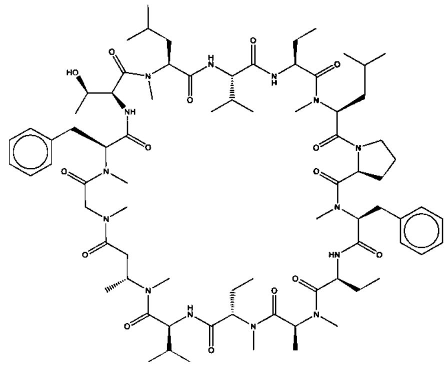 | pd072 | 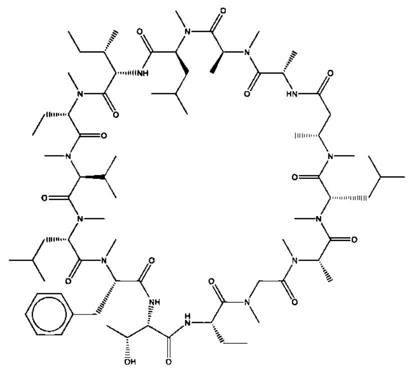 |

(continued)

| Compound No. | Structural formula | Compound No. | Structural formula |
|---|---|---|---|
| pd073 | 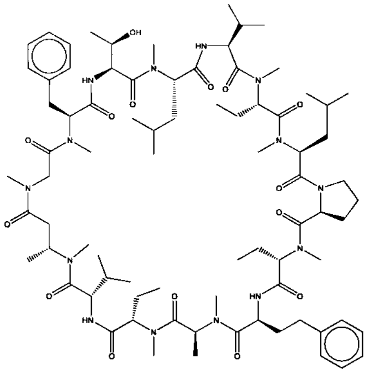 | pd075 | 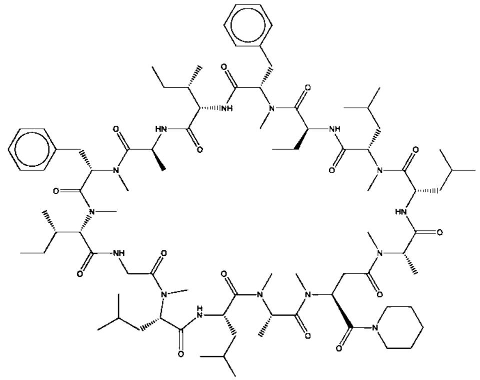 |
| pd074 | 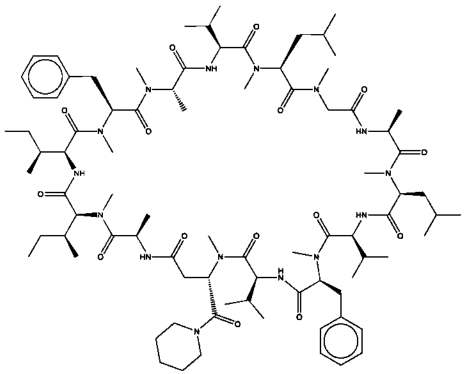 | pd076 | 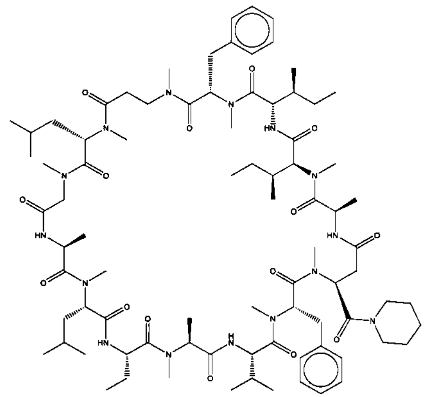 |

(continued)

| Compound No. | Structural formula | Compound No. | Structural formula |
|---|---|---|---|
| pd077 | 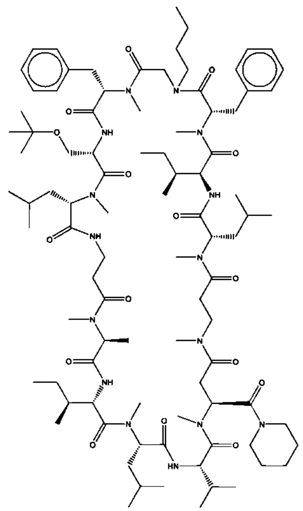 | pd079 | 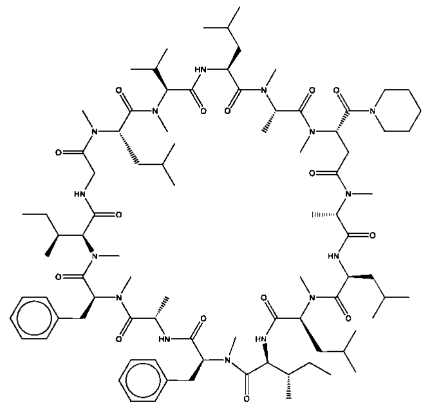 |
| pd078 | 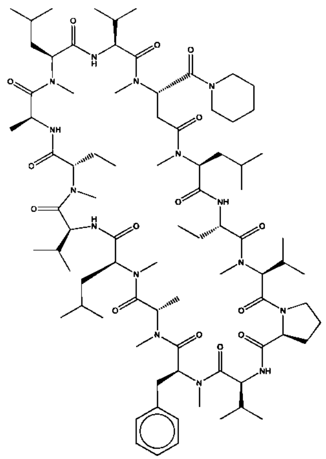 | pd080 | 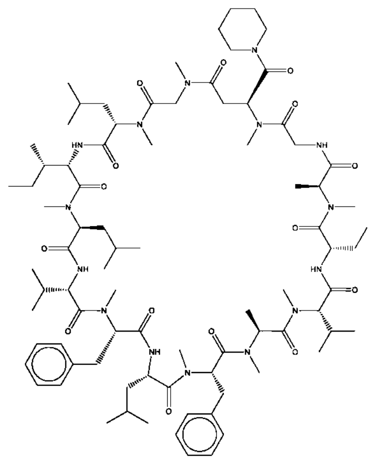 |

(continued)

| Compound No. | Structural formula | Compound No. | Structural formula |
|---|---|---|---|
| pd081 | 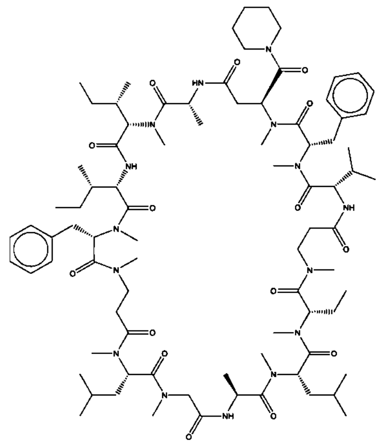 | pd083 | 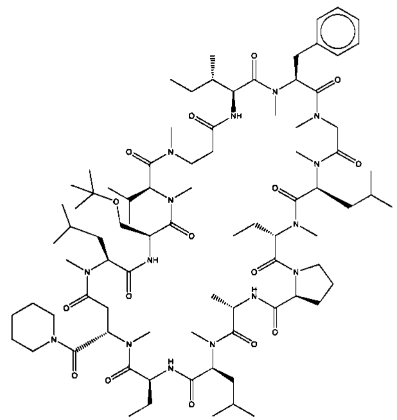 |
| pd082 | 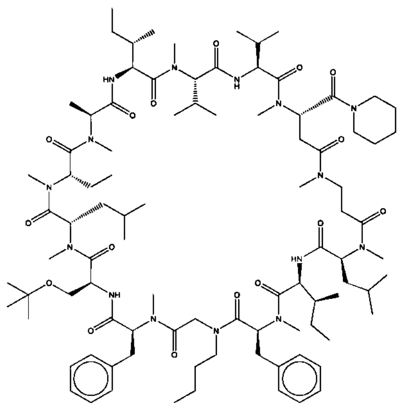 | pd084 | 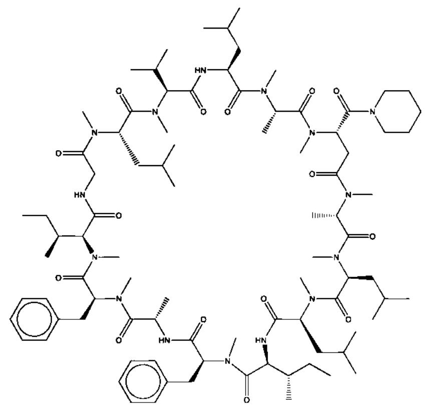 |

(continued)

| Compound No. | Structural formula | Compound No. | Structural formula |
|---|---|---|---|
| pd085 | 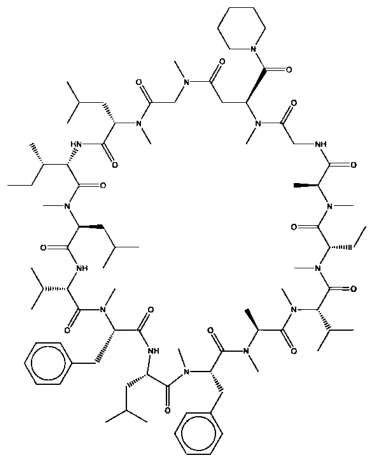 | pd087 | 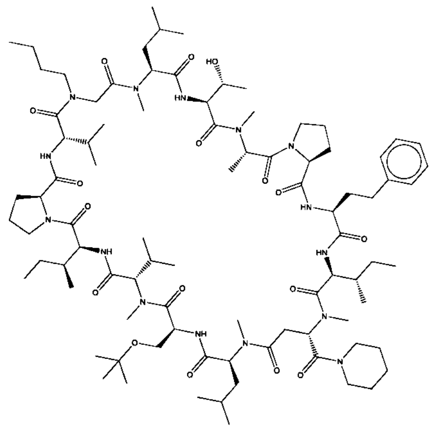 |
| pd086 | 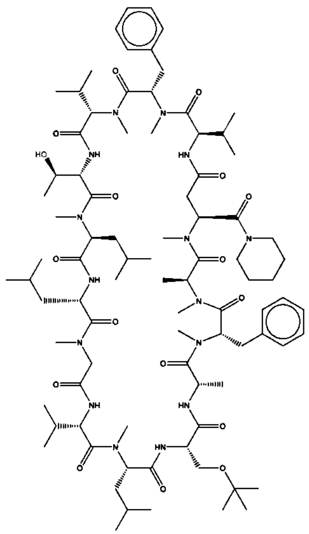 | pd088 | 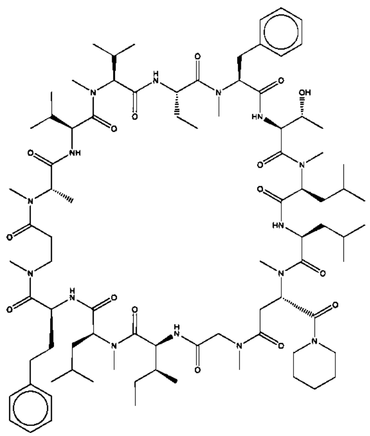 |

(continued)

| Compound No. | Structural formula | Compound No. | Structural formula |
|---|---|---|---|
| pd089 | 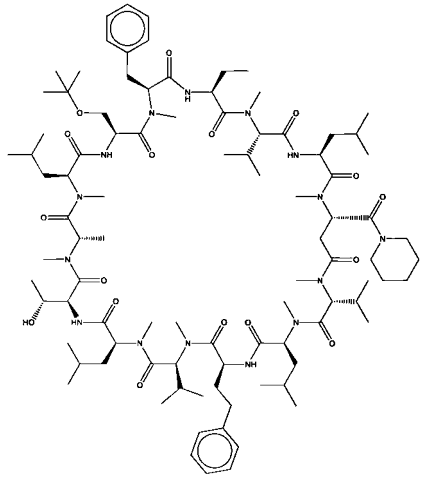 | pd091 | 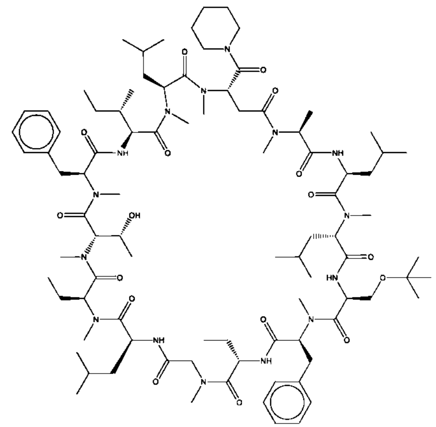 |
| pd090 | 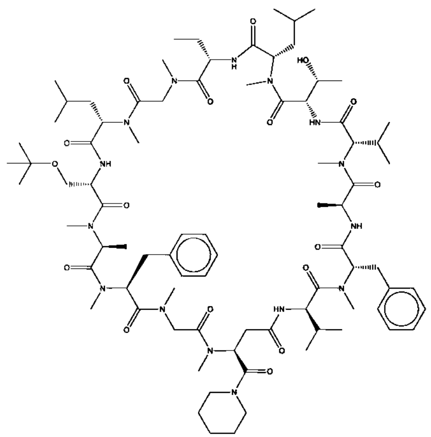 | pd092 | 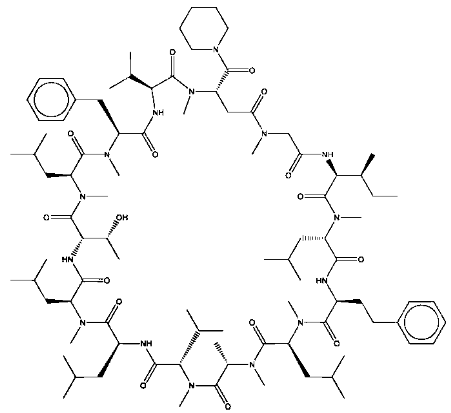 |

(continued)

| Compound No. | Structural formula | Compound No. | Structural formula |
|---|---|---|---|
| pd093 | 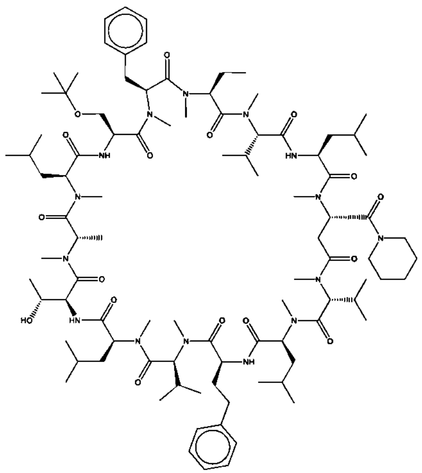 | pd095 | 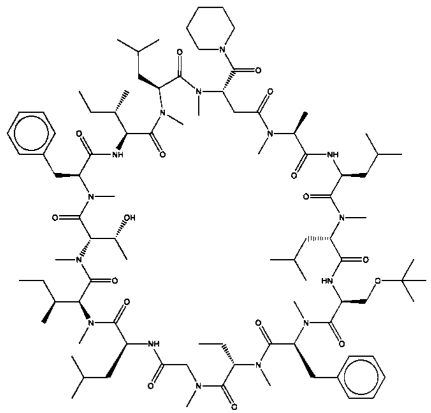 |
| pd094 | 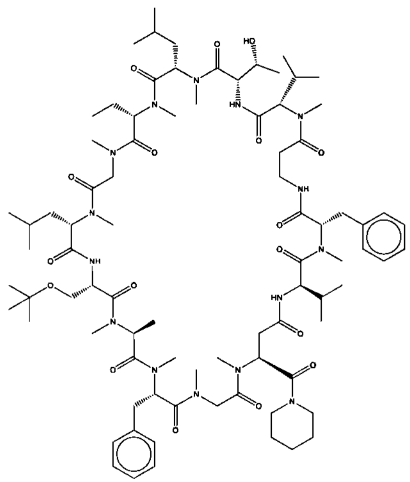 | pd096 | 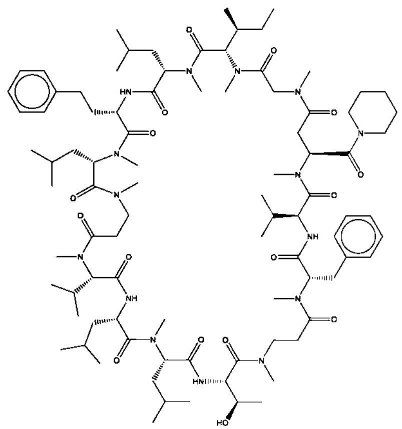 |

(continued)

| Compound No. | Structural formula | Compound No. | Structural formula |
|---|---|---|---|
| pd097 | 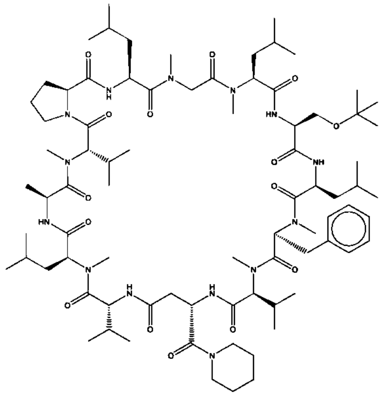 | pd099 | 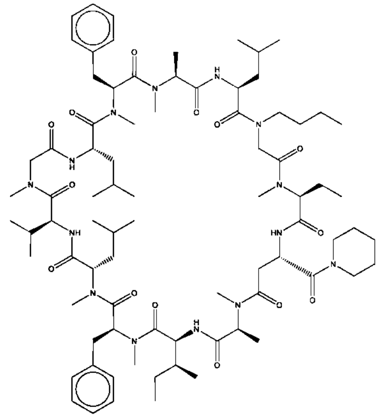 |
| pd098 | 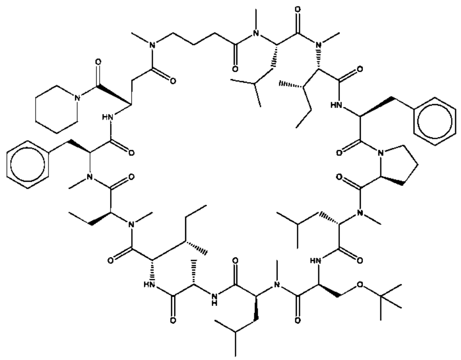 | pd100 | 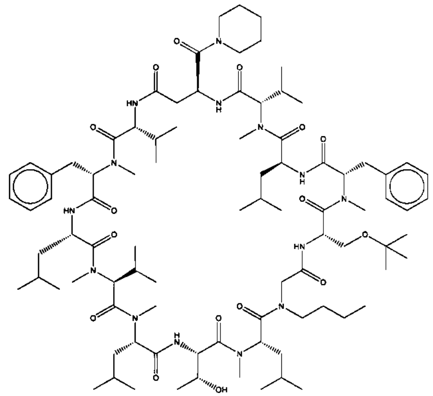 |

(continued)

| Compound No. | Structural formula | Compound No. | Structural formula |
|---|---|---|---|
| pd101 | 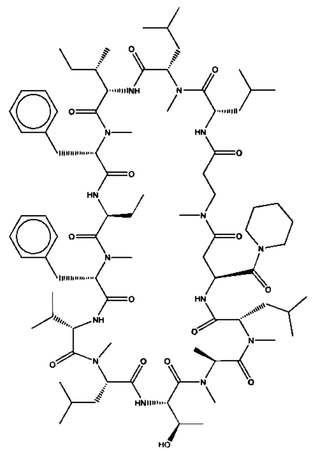 | pd103 | 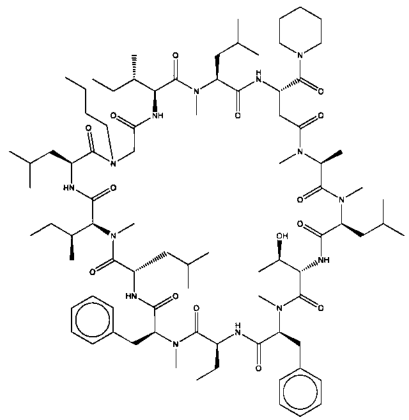 |
| pd102 | 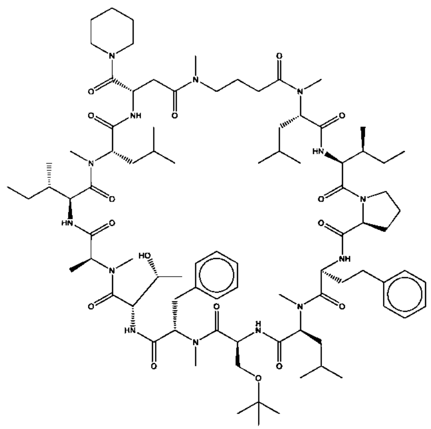 | pd104 | 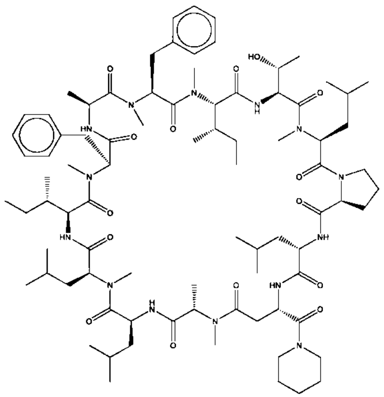 |

(continued)

| Compound No. | Structural formula | Compound No. | Structural formula |
|---|---|---|---|
| pd105 | 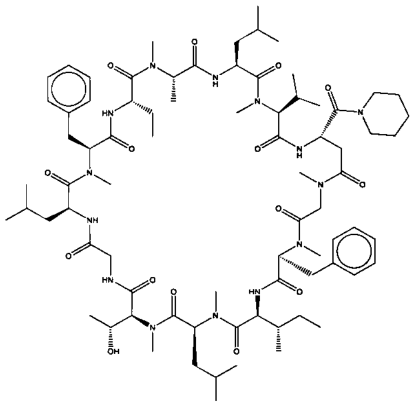 | pd107 | 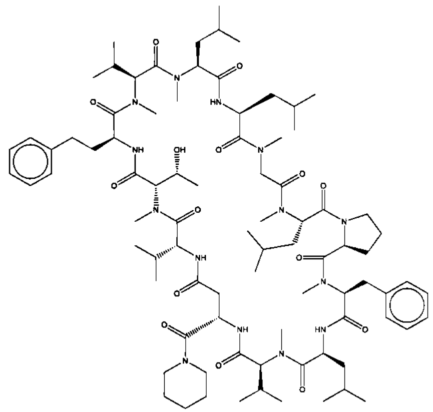 |
| pd106 | 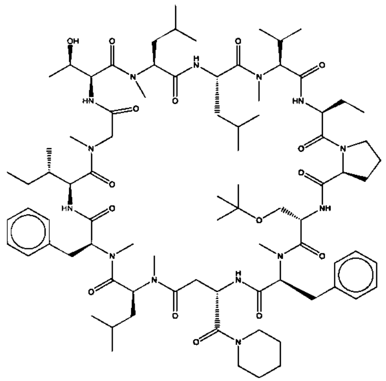 | pd108 | 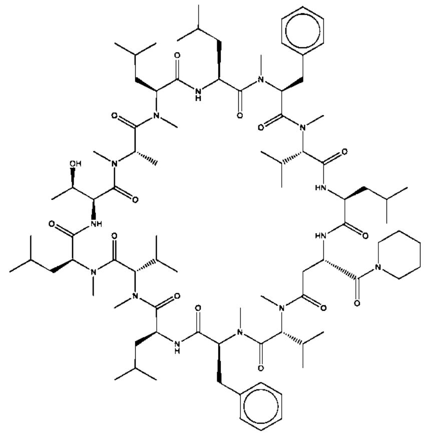 |

(continued)

| Compound No. | Structural formula | Compound No. | Structural formula |
|---|---|---|---|
| pd109 | 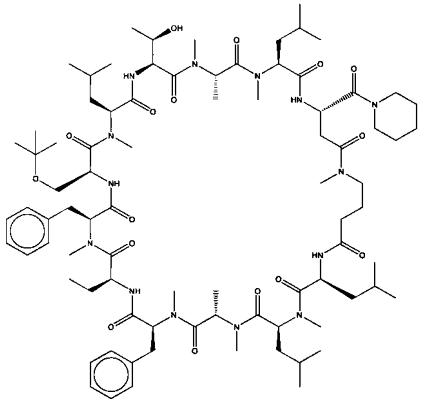 | pd111 | 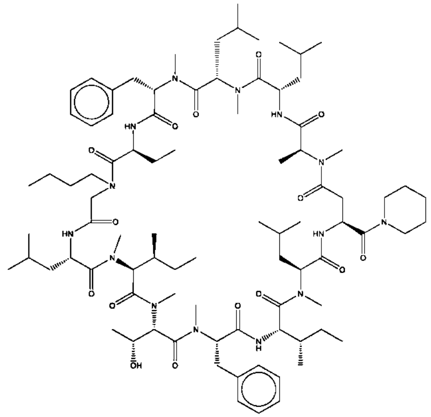 |
| pd110 | 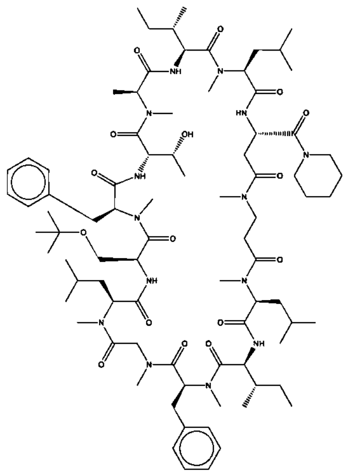 | pd112 | 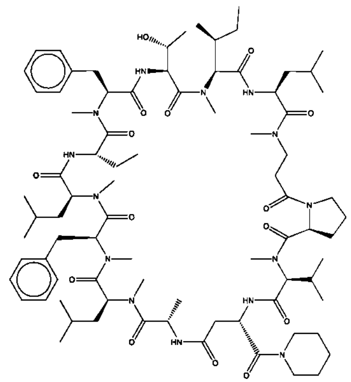 |

(continued)

| Compound No. | Structural formula | Compound No. | Structural formula |
| --- | --- | --- | --- |
| pd113 | 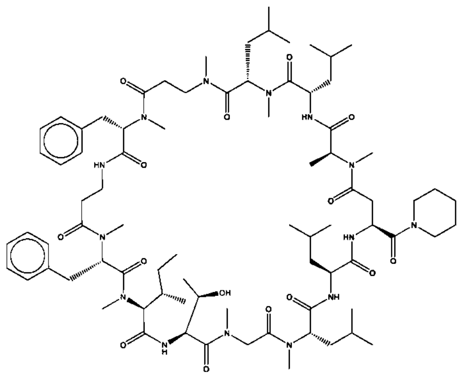 | pd115 | 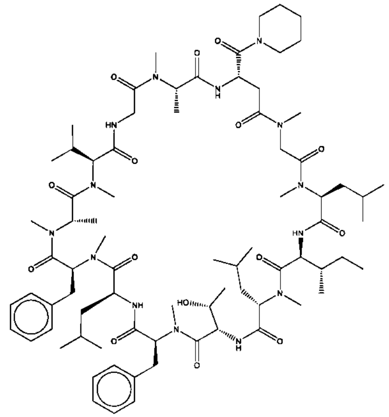 |
| pd114 | 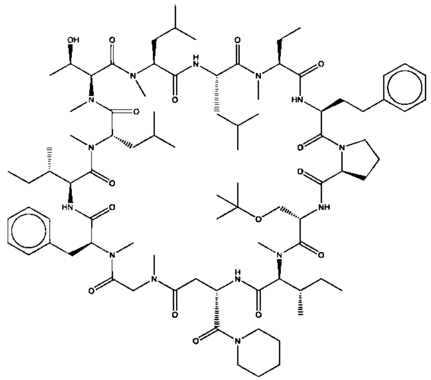 | pd116 | 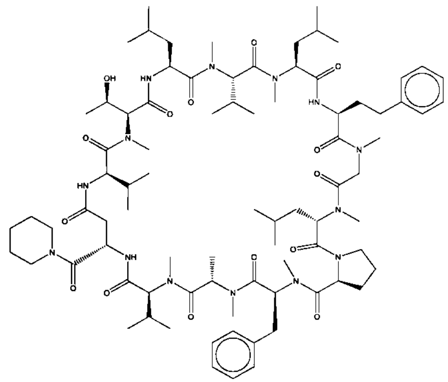 |

(continued)

| Compound No. | Structural formula | Compound No. | Structural formula |
|---|---|---|---|
| pd117 | 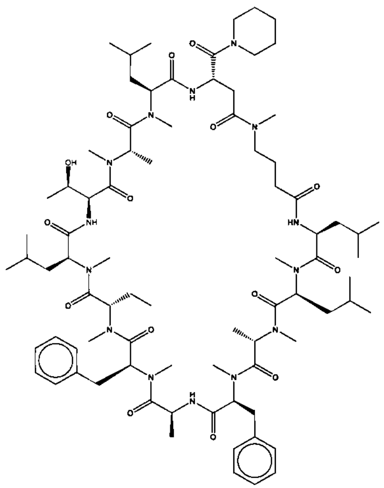 | pd119 | 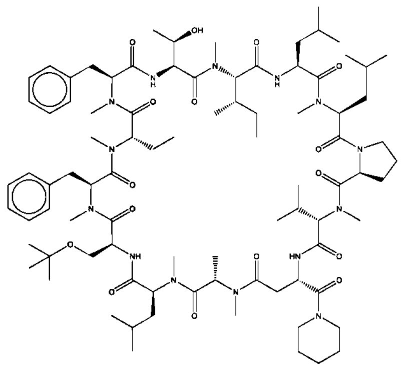 |
| pd118 | 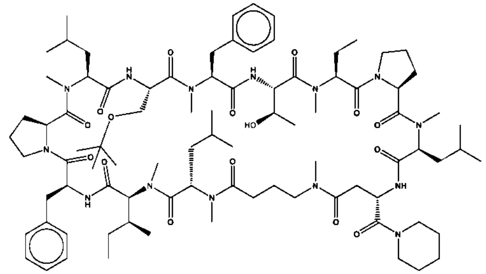 | pd120 | 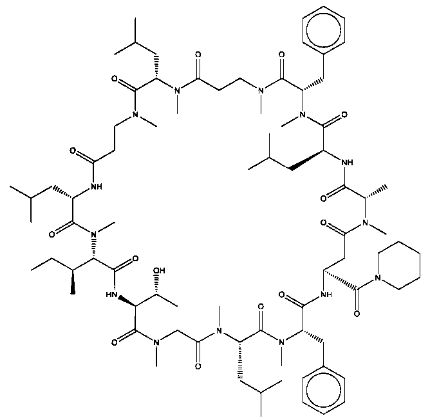 |

(continued)

| Compound No. | Structural formula | Compound No. | Structural formula |
|---|---|---|---|
| pd121 | 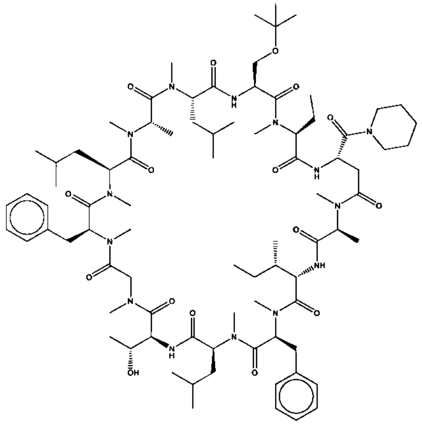 | pd123 | 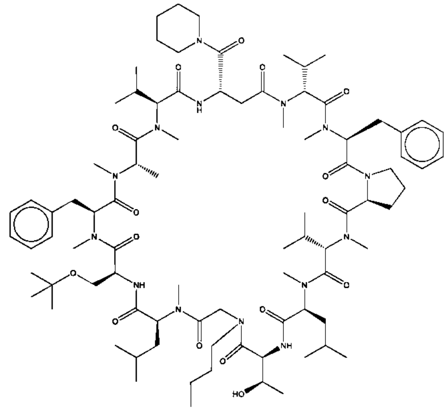 |
| pd122 | 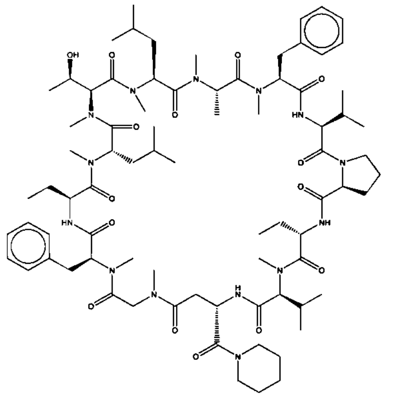 | pd124 | 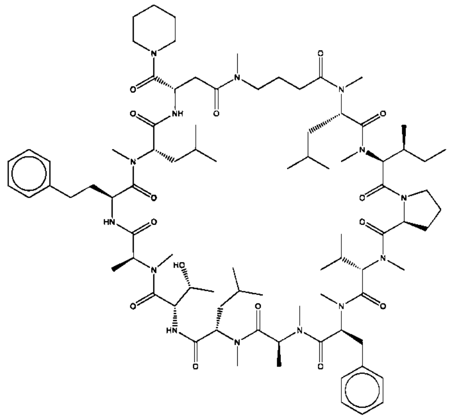 |

(continued)

| Compound No. | Structural formula | Compound No. | Structural formula |
|---|---|---|---|
| pd125 | 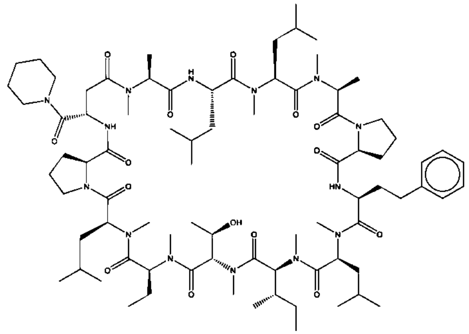 | pd127 | 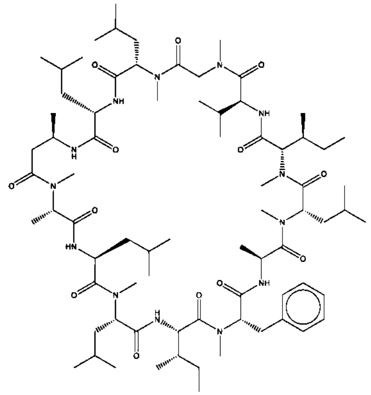 |
| pd126 | 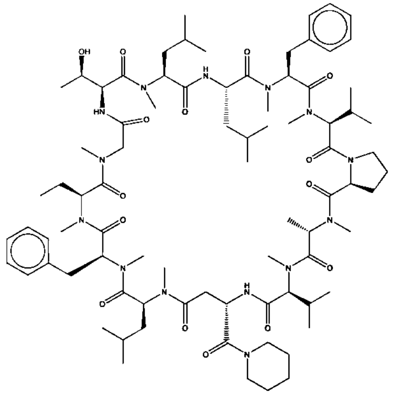 | pd128 | 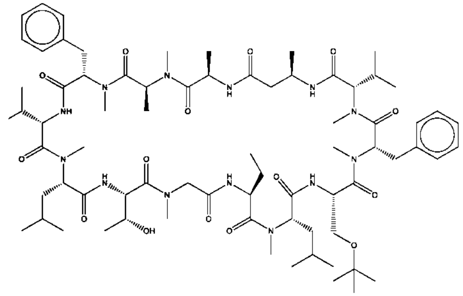 |

(continued)

| Compound No. | Structural formula | Compound No. | Structural formula |
|---|---|---|---|
| pd129 | 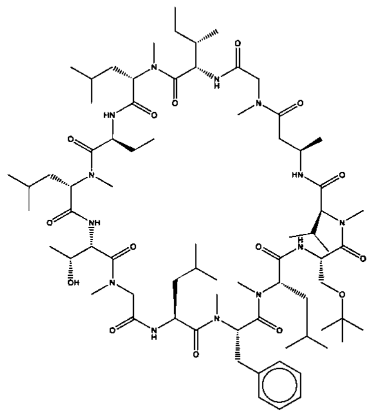 | pd131 | 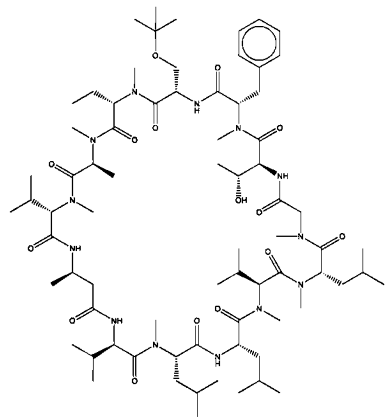 |
| pd130 | 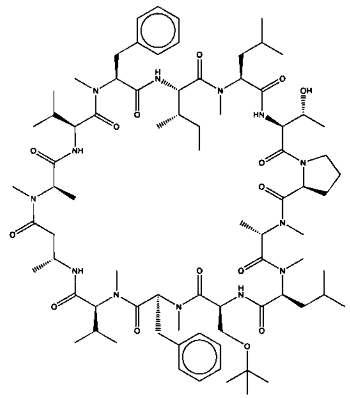 | pd132 | 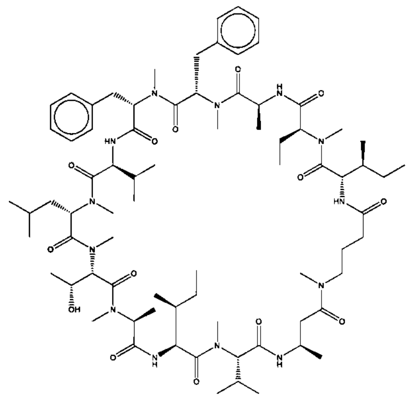 |

(continued)

| Compound No. | Structural formula | Compound No. | Structural formula |
|---|---|---|---|
| pd133 | 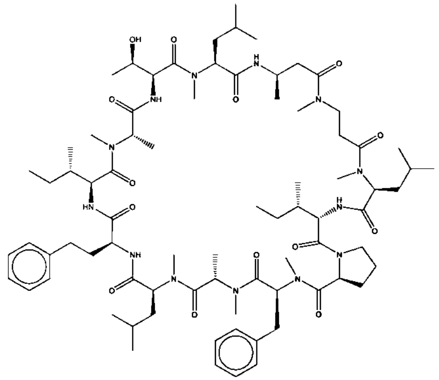 | pd135 | 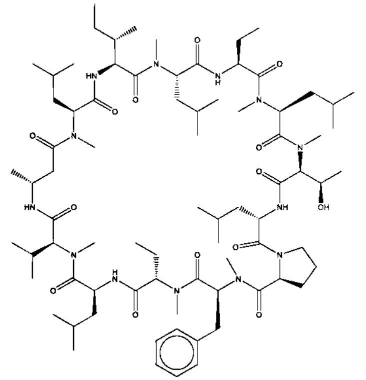 |
| pd134 | 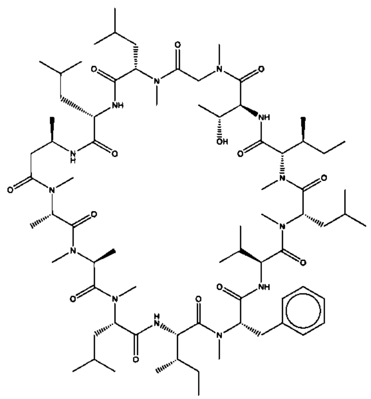 | pd136 | 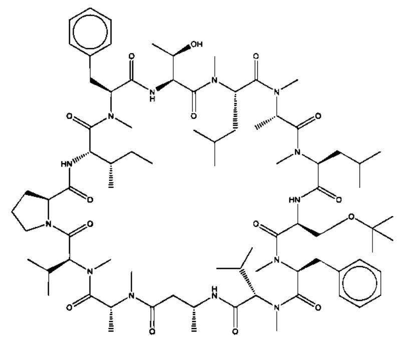 |

(continued)

| Compound No. | Structural formula | Compound No. | Structural formula |
|---|---|---|---|
| pd137 | 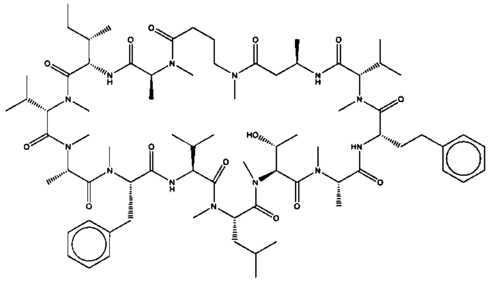 | pd139 | 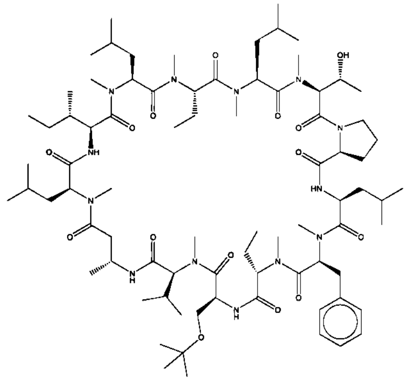 |
| pd138 | 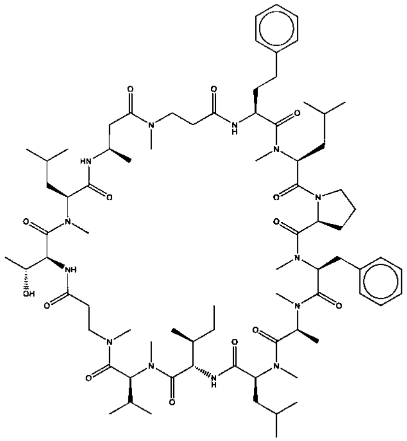 | pd140 | 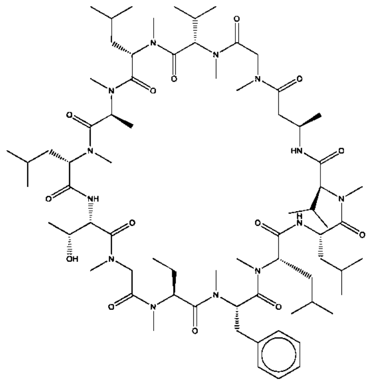 |

(continued)

| Compound No. | Structural formula | Compound No. | Structural formula |
|---|---|---|---|
| pd141 | 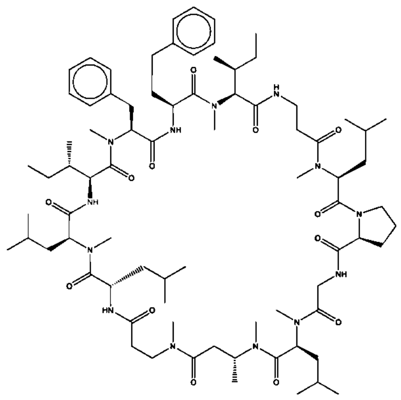 | pd143 | 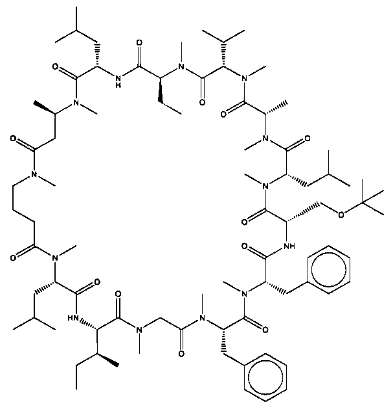 |
| pd142 | 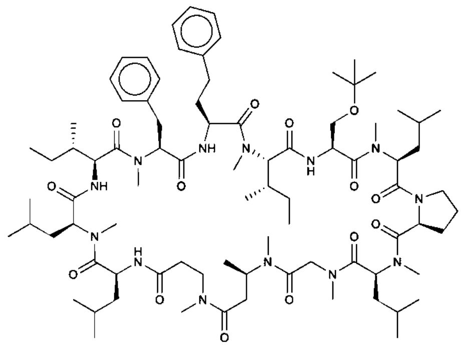 | pd144 | 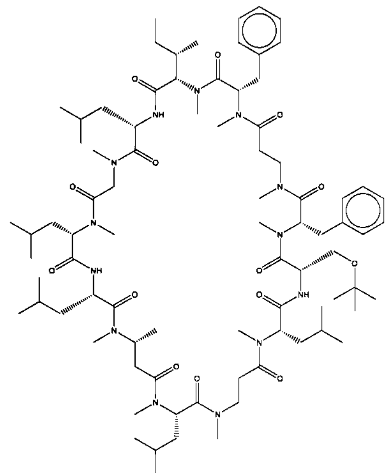 |

(continued)

| Compound No. | Structural formula | Compound No. | Structural formula |
|---|---|---|---|
| pd145 | 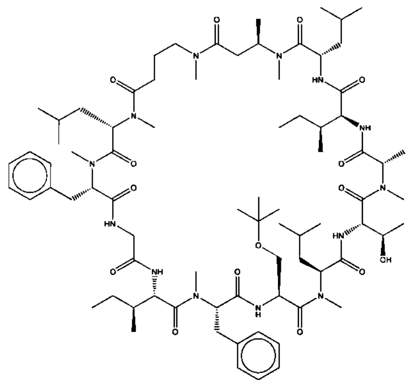 | pd147 | 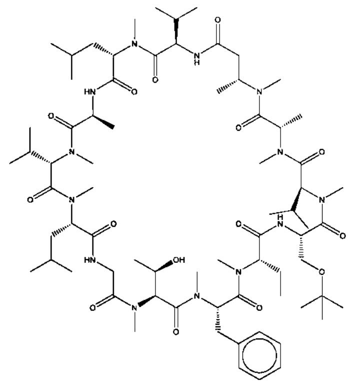 |
| pd146 | 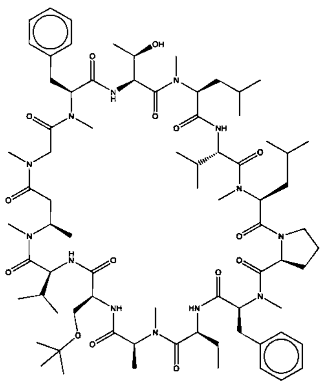 | pd148 | 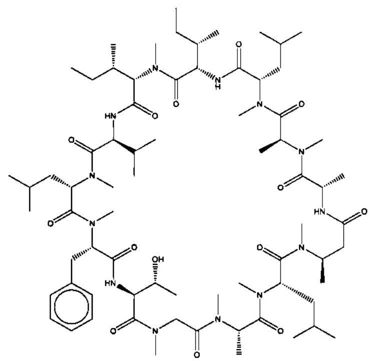 |

(continued)

| Compound No. | Structural formula | Compound No. | Structural formula |
|---|---|---|---|
| pd149 | 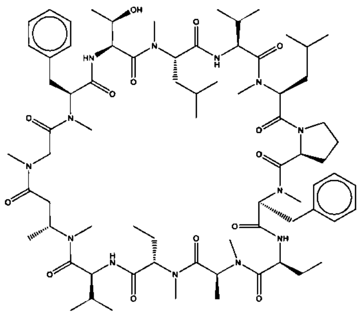 | pd151 | 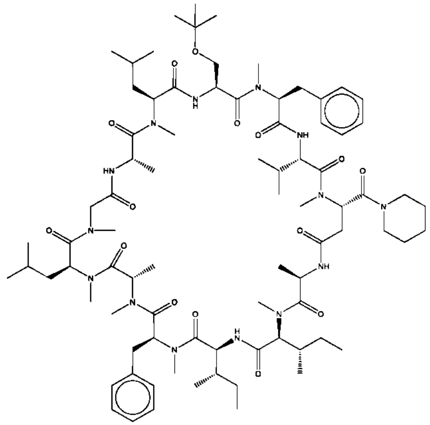 |
| pd150 | 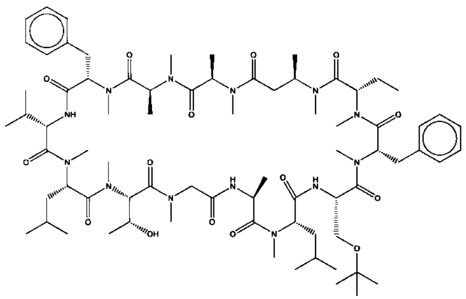 | pd152 | 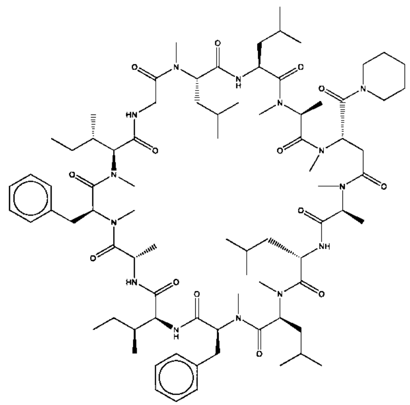 |

(continued)

| Compound No. | Structural formula | Compound No. | Structural formula |
|---|---|---|---|
| pd153 | 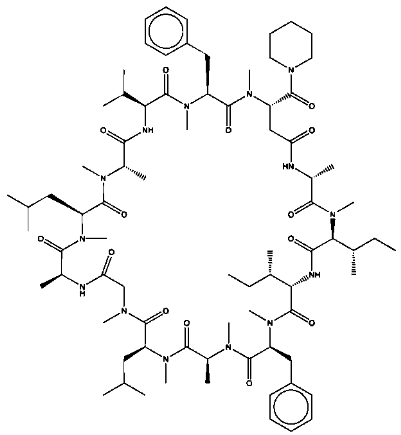 | pd155 | 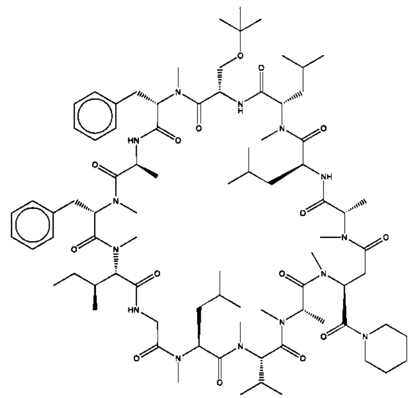 |
| pd154 | 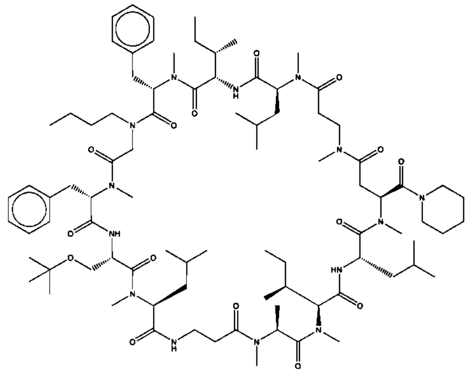 | pd156 | 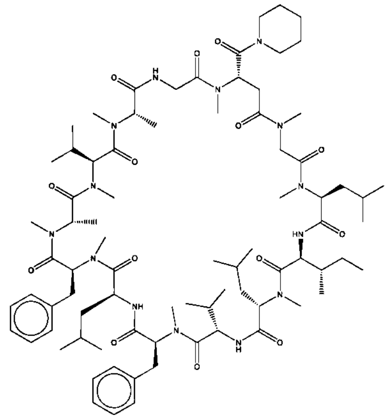 |

(continued)

| Compound No. | Structural formula | Compound No. | Structural formula |
|---|---|---|---|
| pd157 | 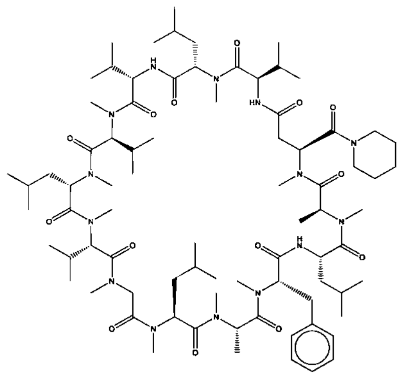 | pd159 | 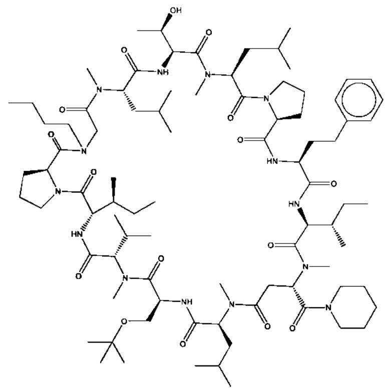 |
| pd158 | 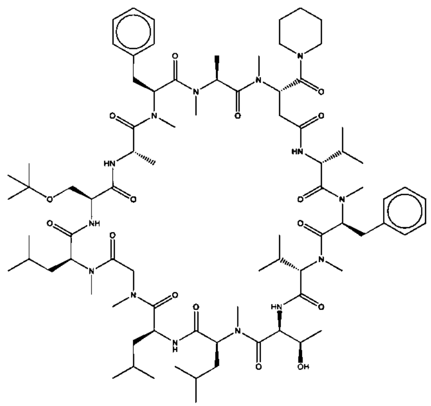 | pd160 | 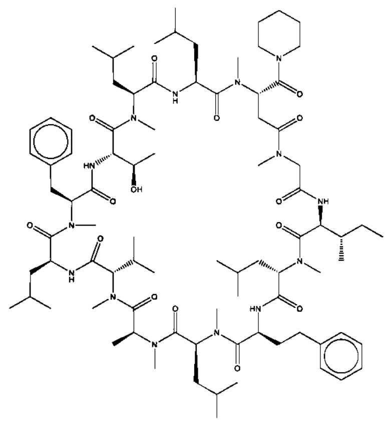 |

(continued)

| Compound No. | Structural formula | Compound No. | Structural formula |
|---|---|---|---|
| pd161 | 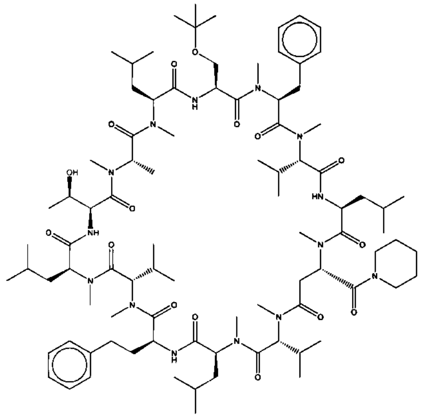 | pd163 | 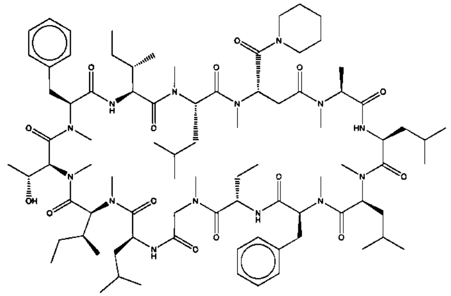 |
| pd162 | 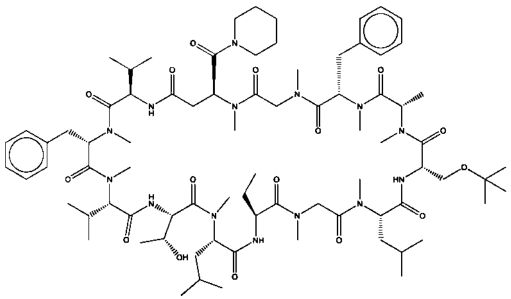 | pd164 | 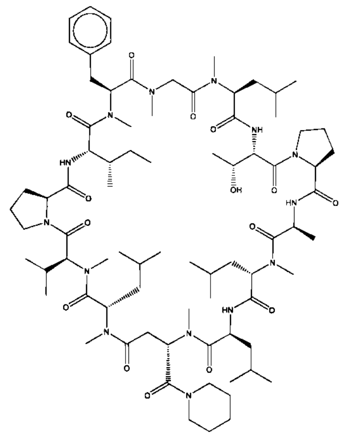 |

(continued)

| Compound No. | Structural formula | Compound No. | Structural formula |
|---|---|---|---|
| pd165 | 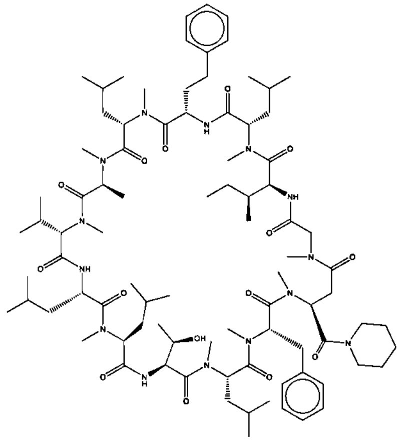 | pd167 | 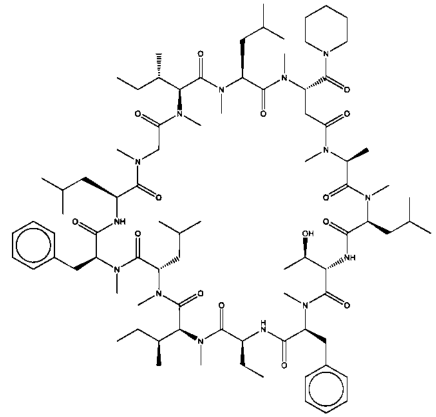 |
| pd166 | 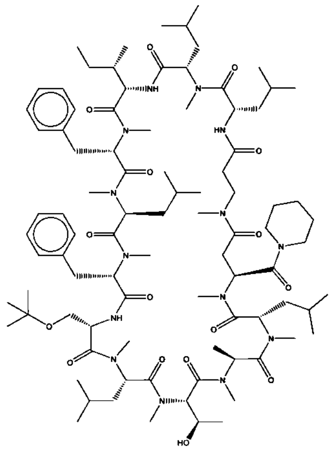 | pd168 | 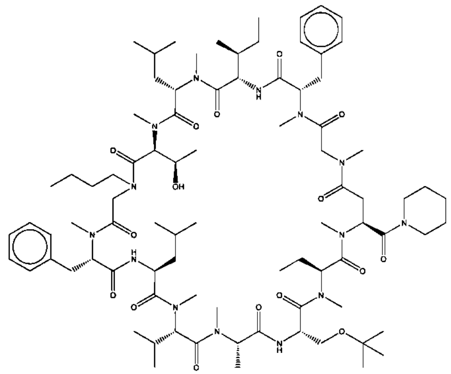 |

**Claims**

1. A cyclic peptide compound optionally linked to a nucleic acid, the compound comprising a cyclic portion, wherein

(1) the cyclic portion is composed of 13 or 14 amino acid residues,
(2) among the amino acid residues composing the cyclic portion, the number of N-substituted amino acid residues is 7 or more, the number of amino acid residues having the same side chain is 3 or less, and the number of natural amino acid residues is 4 or less, and
(3) ClogP/total amide bond (ClogP/total AB) is 1.0 to 1.8.

2. The cyclic peptide compound optionally linked to a nucleic acid according to claim 1, wherein the number of N-substituted amino acids composing the cyclic portion is 7 to 13.

3. The cyclic peptide compound optionally linked to a nucleic acid according to claim 1 or 2, wherein the N-substituted amino acids are N-alkyl amino acids.

4. The cyclic peptide compound optionally linked to a nucleic acid according to any one of claims 1 to 3, wherein the cyclic peptide compound has 3 or less hydrogen bond donors.

5. The cyclic peptide compound optionally linked to a nucleic acid according to any one of claims 1 to 4, wherein a side chain of the amino acid composing the cyclic portion is composed of at least one selected from the group consisting of an alkyl group, a cycloalkyl group, an aralkyl group, and an amide group, wherein an arbitrary of the alkyl group and the nitrogen atom of the amino group of the amino acid may be taken together to form a ring.

6. The cyclic peptide compound optionally linked to a nucleic acid according to claim 5, wherein the alkyl group is a $C_1$-$C_6$ alkyl group optionally substituted with at least one group selected from the group consisting of a halogen atom, a hydroxy group, a $C_3$-$C_8$ cycloalkyl group, and a $C_1$-$C_6$ alkoxy group.

7. The cyclic peptide compound optionally linked to a nucleic acid according to claim 5 or 6, wherein the cycloalkyl group is a $C_3$-$C_{10}$ cycloalkyl group optionally substituted with at least one group selected from the group consisting of a halogen atom, a hydroxy group, a $C_1$-$C_6$ alkyl group, a $C_1$-$C_6$ haloalkyl group, a $C_1$-$C_6$ alkoxy group, and a $C_1$-$C_6$ haloalkoxy group.

8. The cyclic peptide compound optionally linked to a nucleic acid according to any one of claims 5 to 7, wherein the aralkyl group is a $C_7$-$C_{14}$ aralkyl group optionally substituted with at least one group selected from the group consisting of a halogen atom, a $C_1$-$C_6$ alkyl group, a $C_1$-$C_6$ haloalkyl group, a $C_1$-$C_6$ alkoxy group, and a $C_1$-$C_6$ haloalkoxy group.

9. The cyclic peptide compound optionally linked to a nucleic acid according to any one of claims 5 to 8, wherein the amide group is represented by formula: $-CONR^1R^2$, wherein $R^1$ and $R^2$ are each independently a hydrogen atom or a $C_1$-$C_6$ alkyl group, or $R^1$ and $R^2$, together with a nitrogen atom to which they are attached, form a 4- to 8-membered saturated heterocyclic ring, and the 4- to 8-membered saturated heterocyclic ring is optionally substituted with at least one group independently selected from the group consisting of a halogen atom, an oxo group, a $C_1$-$C_6$ alkyl group, and a 4- to 7-membered heterocyclyl group.

10. The cyclic peptide compound optionally linked to a nucleic acid according to any one of claims 1 to 9, wherein among amino acids composing the cyclic portion, the number of natural amino acids is 1 to 4.

11. The cyclic peptide compound optionally linked to a nucleic acid according to any one of claims 1 to 10, wherein amino acids composing the cyclic portion include a natural amino acid selected from the group consisting of Gly, Ala, Val, Leu, Ile, Thr, Phe, and Pro.

12. The cyclic peptide compound optionally linked to a nucleic acid according to any one of claims 1 to 11, wherein among amino acids composing the cyclic portion, the number of non-natural amino acids is 9 or more.

13. The cyclic peptide compound optionally linked to a nucleic acid according to any one of claims 1 to 12, wherein amino acids composing the cyclic portion include a non-natural amino acid selected from the group consisting of Abu, Asp-pip, bAla, bMeAla, D-3-Abu, D-3-MeAbu, D-Ala, D-MeAla, D-MeVal, D-Val, gMeAbu, Hph, MeAbu, MeAla, MeAsp-pip, MeGly, MeIle, MeLeu, MePhe, MeThr, MeVal, nBuGly, and Ser(tBu).

**14.** The cyclic peptide compound optionally linked to a nucleic acid according to any one of claims 1 to 13, wherein a value of Caco-2 $P_{app}$ (cm/sec) of the cyclic peptide compound is $0.4 \times 10^{-6}$ or more.

**15.** A library comprising the cyclic peptide compound optionally linked to a nucleic acid according to any one of claims 1 to 14.

**16.** A method for screening for a cyclic peptide compound that is capable of specifically binding to a target molecule, the method comprising the steps of:

(i) bringing the library according to claim 15 into contact with the target molecule; and
(ii) selecting a cyclic peptide compound that is capable of specifically binding to the target molecule.

**Fig.1**

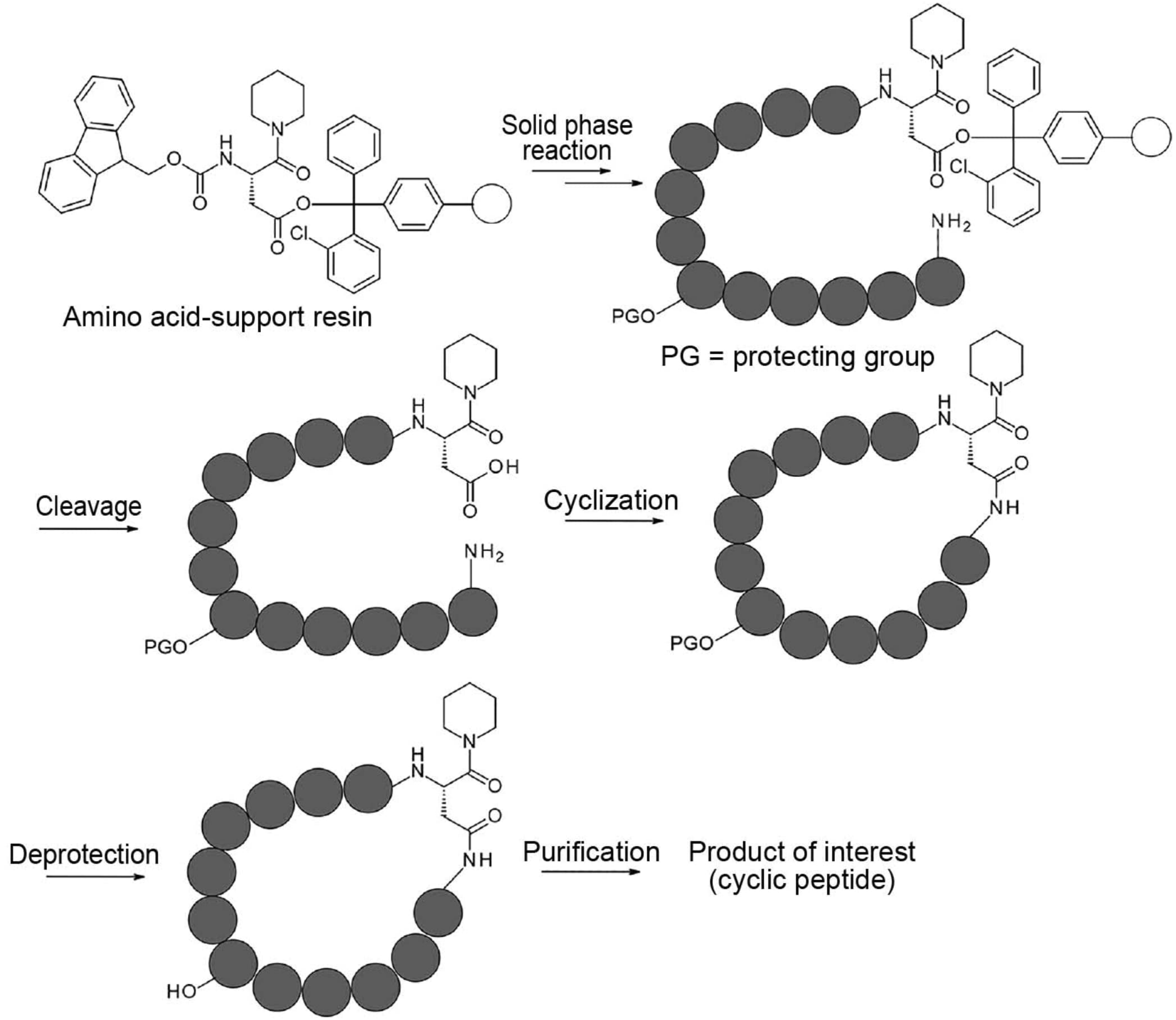
Amino acid-support resin
Solid phase reaction
PG = protecting group
Cleavage
Cyclization
Deprotection
Purification
Product of interest (cyclic peptide)

## INTERNATIONAL SEARCH REPORT

International application No.
**PCT/JP2024/039298**

**A. CLASSIFICATION OF SUBJECT MATTER**

***C07K 7/54***(2006.01)i; ***C40B 40/10***(2006.01)i
FI: C07K7/54; C40B40/10

According to International Patent Classification (IPC) or to both national classification and IPC

**B. FIELDS SEARCHED**

Minimum documentation searched (classification system followed by classification symbols)

C07K1/00-19/00; C40B10/00;40/02;40/06-40/10;50/00

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2013/100132 A1 (CHUGAI SEIYAKU KABUSHIKI KAISHA) 04 July 2013 (2013-07-04)<br>tables 11-1, 11-3-1 | 1-16 |
| A | WO 2018/225864 A1 (CHUGAI SEIYAKU KABUSHIKI KAISHA) 13 December 2018 (2018-12-13)<br>tables 17-26 | 1-16 |
| A | WO 2020/122182 A1 (CHUGAI SEIYAKU KABUSHIKI KAISHA) 18 June 2020 (2020-06-18)<br>tables 4-15, 27-38 | 1-16 |
| A | TANADA, M., et al., Development of Orally Bioavailable Peptides Targeting an Intracellular Protein: From a Hit to a Clinical KRAS Inhibitor, J. Am. Chem. Soc., 18 July 2023, vol. 145, pp. 16610-16620<br>particularly, Abstract, tables 1-4, 6 | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 January 2025** | **21 January 2025** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
PCT/JP2024/039298

C. DOCUMENTS CONSIDERED TO BE RELEVANT

| Category* | Citation of document, with indication, where appropriate, of the relevant passages | Relevant to claim No. |
|---|---|---|
| A | OHTA, A., et al., Validation of a New Methodology to Create Oral Drugs beyond the Rule of 5 for Intracellular Tough Targets, J. Am. Chem. Soc., 24 October 2023, vol. 145, pp. 24035-24051<br>particularly, Results, fig. 2 | 1-16 |

Form PCT/ISA/210 (second sheet) (July 2022)

**INTERNATIONAL SEARCH REPORT**
**Information on patent family members**

International application No.
**PCT/JP2024/039298**

| Patent document cited in search report | Publication date (day/month/year) | Patent family member(s) | Publication date (day/month/year) |
|---|---|---|---|
| WO 2013/100132 A1 | 04 July 2013 | EP 2813512 A1<br>tables 11-1, 11-3-1 | |
| WO 2018/225864 A1 | 13 December 2018 | EP 3636807 A1<br>tables 17-26 | |
| WO 2020/122182 A1 | 18 June 2020 | EP 3896056 A1<br>tables 4-15, 27-38 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2018225864 A
- WO 2020122182 A
- WO 2024214825 A
- WO 2016148044 A
- WO 2008117833 A
- WO 2012074129 A


### Non-patent literature cited in the description


- *Future Med. Chem*, 2009, vol. 1, 1289-1310
- *Acc. Chem. Res*, 2008, vol. 41, 1331-1342
- *Angew. Chem. Int. Ed.*, 2013, vol. 52, 254-269
- *Chem. Rev.*, 2019, vol. 119, 10360-10391
- *CLOGP Reference Manual Daylight Version 4.9*, 01 August 2011, https://www.daylight.com/dayhtml/-doc/clogp
- **LL VON MOLTKE et al.** Midazolam hydroxylation by human liver microsomes in vitro: inhibition by fluoxetine, norfluoxetine, and by azole antifungal agents. *J Clin Pharmacol*, 1996, vol. 36 (9), 783-791
- **M. KATO et al.** The intestinal first-pass metabolism of substances ofCYP3A4 and P-glycoprotein-quantitative analysis based on information from the literature. *Drug Metab. Pharmacokinet*, 2003, vol. 18 (6), 365-372
- **YANAGAWA, H et al.** *FEBS Lett*, 1997, vol. 414, 405-408
- **SZOSTAK J.W. et al.** *Proc. Natl. Acd. Sci. USA*, 1997, vol. 94, 12297-12302
- *Proc Natl Acad Sci USA.*, 1997, vol. 94, 12297-302
- *FEBS Lett.*, 1997, vol. 414, 405-8
- *Nucleic Acids Res.*, 2009, vol. 37 (16), 108
- *Proc Natl Acad Sci U S A.*, 1994, vol. 91, 9022-6
- *Nucleic AcidsRes.*, 2005, vol. 33, e10
- *Proc Natl Acad Sci U S A.*, 2004, vol. 101, 2806-10
- *Nat Biotechnol.*, 1998, vol. 16, 652-6
- Solid Phase Synthesis Handbook. Merck KGaA, 01 May 2002
- *Nat Biotechnol.*, 2001, vol. 19, 751-5
- **SHIMIZU Y** ; **INOUE A** ; **TOMARI Y** ; **SUZUKI T** ; **YOKOGAWA T** ; **NISHIKAWA K** ; **UEDA T**. *Methods Mol Biol*, 2010, vol. 607, 11-21
- **SHIMIZU Y** ; **UEDA T**. *PURE technology*
- *J Am Chem Soc.*, 2005, vol. 127, 11727-35
- **KWON I et al.** Breaking the degeneracy of the genetic code. *J Am Chem Soc.*, 2003, vol. 125, 7512-3
- **KAWAKAMI T et al.** Ribosomal synthesis of polypeptoids and peptoid-peptide hybrids. *J Am Chem Soc.*, 2008, vol. 130, 16861-3
- **KAWAKAMI T et al.** Diverse backbone-cyclized peptides via codon reprogramming. *Nat Chem Biol.*, 2009, vol. 5, 888-90
- **DEDKOVA LM et al.** Construction of modified ribosomes for incorporation of D-amino acids into proteins. *Biochemistry*, 2006, vol. 45, 15541-51
- **DOI Y et al.** Elongation factor Tu mutants expand amino acid tolerance of protein biosynthesis system. *J Am Chem Soc.*, 2007, vol. 129, 14458-62
- **PARK HS et al.** Expanding the genetic code of Escherichia coli with phosphoserine. *Science*, 2011, vol. 333, 1151-4
- *Biochemistry*, 2003, vol. 42, 9598-608
- **ANDERSON JC** ; **SCHULTZ PG**. *Chem Biol.*, 2003, vol. 10, 1077-84
- *Proc.Natl.Acad.Sci. U S A.*, 2002, vol. 99, 9715-20
- **KIGA D** ; **SAKAMOTO K** ; **KODAMA K** ; **KIGAWA T** ; **MATSUDA T** ; **YABUKI T** ; **SHIROUZU M** ; **HARADA Y** ; **NAKAYAMA H** ; **TAKIO K**. *Science*, 2003, vol. 301, 964-7
- **CHIN JW** ; **CROPP TA** ; **ANDERSON JC** ; **MUKHERJI M** ; **ZHANG Z** ; **SCHULTZ PG** ; **CHIN, JW**. *Proc.Natl.Acad.Sci. U S A.*, 2006, vol. 103, 4356-61
- *J Am Chem Soc.*, 2008, vol. 130, 6131-6
- *Biochemistry*, 1984, vol. 23, 1468-73
- *J Am Chem Soc.*, 2002, vol. 124, 6834-5
- *Chem Commun (Camb).*, 2005 (34), 4321-3
- *J Am Chem Soc.*, 2004, vol. 126, 15984-9